(19) Europäisches Patentamt / European Patent Office / Office européen des brevets

(11) **EP 2 612 151 B1**

(12) **EUROPEAN PATENT SPECIFICATION**

(45) Date of publication and mention
of the grant of the patent:
**09.08.2017   Bulletin 2017/32**

(51) Int Cl.:
***G01N 33/574*** (2006.01)   ***A61K 39/395*** (2006.01)

(21) Application number: **11754597.0**

(22) Date of filing: **31.08.2011**

(86) International application number:
**PCT/US2011/050069**

(87) International publication number:
**WO 2012/031027 (08.03.2012 Gazette 2012/10)**

(54) **BIOMARKERS AND METHODS OF TREATMENT**

BIOMARKER UND BEHANDLUNGSVERFAHREN

BIOMARQUEURS ET PROCÉDÉS DE TRAITEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **30.06.2011   US 201161503489 P
01.06.2011   US 201161492338 P
18.05.2011   US 201161487527 P
07.12.2010   US 420703 P
07.10.2010   US 390995 P
05.10.2010   US 389922 P
31.08.2010   US 378911 P**

(43) Date of publication of application:
**10.07.2013   Bulletin 2013/28**

(73) Proprietor: **Genentech, Inc.
South San Francisco, CA 94080 (US)**

(72) Inventors:
• **PATEL, Premal
South San Francisco, California 94080 (US)**
• **PETERSON, Amy C.
San Francisco, California 94110 (US)**
• **YAUCH, Robert L.
South San Francisco, California 94080 (US)**
• **ZHA, Jiping
Taicang Economic Development Area
Jiangsu Province 215400 (CN)**

(74) Representative: **Woolley, Lindsey Claire et al
Mewburn Ellis LLP
City Tower
40 Basinghall Street
London EC2V 5DE (GB)**

(56) References cited:
**WO-A2-2010/045345**

• **GOETSCH L ET AL: "Selection criteria for
c-Met-targeted therapies: Emerging evidence for
biomarkers", BIOMARKERS IN MEDICINE 2010
FUTURE MEDICINE LTD. GBR LNKD-
DOI:10.2217/BMM.09.67, vol. 4, no. 1, February
2010 (2010-02), pages 149-170, XP009154090,
ISSN: 1752-0363**
• **SMOLEN GROMOSLAW A ET AL: "Amplification
of MET may identify a subset of cancers with
extreme sensitivity to the selective tyrosine
kinase inhibitor PHA-665752", PROCEEDINGS
OF THE NATIONAL ACADEMY OF SCIENCES OF
USA, NATIONAL ACADEMY OF SCIENCE,
WASHINGTON, DC; US, vol. 103, no. 7, 1 February
2006 (2006-02-01), pages 2316-2321,
XP002392188, ISSN: 0027-8424, DOI:
10.1073/PNAS.0508776103**
• **KATSUHIRO OKUDA ET AL: "Met gene copy
number predicts the prognosis for completely
resected non-small cell lung cancer", CANCER
SCIENCE, vol. 99, no. 11, 1 November 2008
(2008-11-01), pages 2280-2285, XP55012508,
ISSN: 1347-9032, DOI:
10.1111/j.1349-7006.2008.00916.x**

**(Cont. next page)**

- **KAMIYA: "Human c-Met ELISA For the quantitative determination of c-Met in human serum, EDTA-plasma or cell culture media", 20080201, no. KT-444, 1 February 2008 (2008-02-01), pages 1-6, XP007919726,**
- **SATTLER MARTIN ET AL: "The role of the c-Met pathway in lung cancer and the potential for targeted therapy.", THERAPEUTIC ADVANCES IN MEDICAL ONCOLOGY JUL 2011 LNKD-PUBMED:21904579, vol. 3, no. 4, July 2011 (2011-07), pages 171-184, XP55012527, ISSN: 1758-8359**

SEQUENCE LISTING

<110> GENENTECH, INC.

<120> BIOMARKERS AND METHODS OF TREATMENT

<130> P4492R1 WO

<140>
<141>

<150> 61/503,489
<151> 2011-06-30

<150> 61/492,338
<151> 2011-06-01

<150> 61/487,527
<151> 2011-05-18

<150> 61/420,703
<151> 2010-12-07

<150> 61/390,995
<151> 2010-10-07

<150> 61/389,922
<151> 2010-10-05

<150> 61/378,911
<151> 2010-08-31

<160> 13

<170> PatentIn version 3.5

<210> 1
<211> 10
<212> PRT
<213> Artificial Sequence

<220>
<221> source
<223> /note="Description of Artificial Sequence: Synthetic peptide"

<400> 1

```
Gly Tyr Thr Phe Thr Ser Tyr Trp Leu His
1               5               10
```

<210> 2
<211> 18
<212> PRT
<213> Artificial Sequence

<220>
<221> source
<223> of Artificial Sequence: Synthetic peptide"

<400> 2

```
Gly Met Ile Asp Pro Ser Asn Ser Asp Thr Arg Phe Asn Pro Asn Phe

    1               5               10              15


Lys Asp
```

<210> 3
<211> 12
<212> PRT
<213> Artificial Sequence

<220>
<221> source
<223> /note="Description of Artificial Sequence: Synthetic peptide"

<400> 3

```
Ala Thr Tyr Arg Ser Tyr Val Thr Pro Leu Asp Tyr
1               5                   10
```

<210> 4
<211> 17
<212> PRT
<213> Artificial Sequence

<220>
<221> source
<223> /note="Description of Artificial Sequence: Synthetic peptide"

<400> 4

```
Lys Ser Ser Gln Ser Leu Leu Tyr Thr Ser Ser Gln Lys Asn Tyr Leu
1               5                   10                  15
```

Ala

<210> 5
<211> 7
<212> PRT
<213> Artificial Sequence

<220>
<221> source
<223> /note="Description of Artificial Sequence: Synthetic peptide"

<400> 5

```
Trp Ala Ser Thr Arg Glu Ser
1               5
```

<210> 6
<211> 9
<212> PRT
<213> Artificial Sequence

<220>
<221> source
<223> /note="Description of Artificial Sequence: Synthetic peptide"

<400> 6

```
Gln Gln Tyr Tyr Ala Tyr Pro Trp Thr
1               5
```

<210> 7
<211> 119
<212> PRT
<213> Artificial Sequence

<220>
<221> source
<223> /note="Description of Artificial Sequence: Synthetic polypeptide"

<400> 7

```
Glu Val Gln Leu Val Glu Ser Gly Gly Gly Leu Val Gln Pro Gly Gly
1               5               10              15

Ser Leu Arg Leu Ser Cys Ala Ala Ser Gly Tyr Thr Phe Thr Ser Tyr
            20              25              30

Trp Leu His Trp Val Arg Gln Ala Pro Gly Lys Gly Leu Glu Trp Val
        35              40              45

Gly Met Ile Asp Pro Ser Asn Ser Asp Thr Arg Phe Asn Pro Asn Phe
    50              55              60

Lys Asp Arg Phe Thr Ile Ser Ala Asp Thr Ser Lys Asn Thr Ala Tyr
65              70              75              80

Leu Gln Met Asn Ser Leu Arg Ala Glu Asp Thr Ala Val Tyr Tyr Cys
                85              90              95

Ala Thr Tyr Arg Ser Tyr Val Thr Pro Leu Asp Tyr Trp Gly Gln Gly
            100             105             110

Thr Leu Val Thr Val Ser Ser
        115
```

<210> 8
<211> 114
<212> PRT
<213> Artificial Sequence

<220>
<221> source
<223> /note="Description of Artificial Sequence: Synthetic polypeptide"

<400> 8

```
Asp Ile Gln Met Thr Gln Ser Pro Ser Ser Leu Ser Ala Ser Val Gly
```

```
      1                    5                         10                          15

Asp Arg Val Thr Ile Thr Cys Lys Ser Ser Gln Ser Leu Leu Tyr Thr
                20                      25                30

Ser Ser Gln Lys Asn Tyr Leu Ala Trp Tyr Gln Gln Lys Pro Gly Lys
            35                      40                  45

Ala Pro Lys Leu Leu Ile Tyr Trp Ala Ser Thr Arg Glu Ser Gly Val
        50                      55                  60

Pro Ser Arg Phe Ser Gly Ser Gly Ser Gly Thr Asp Phe Thr Leu Thr
65                      70                  75                      80

Ile Ser Ser Leu Gln Pro Glu Asp Phe Ala Thr Tyr Tyr Cys Gln Gln
                85                      90                  95

Tyr Tyr Ala Tyr Pro Trp Thr Phe Gly Gln Gly Thr Lys Val Glu Ile
            100                     105                 110

Lys Arg
```

<210> 9
<211> 222
<212> PRT
<213> Artificial Sequence

<220>
<221> source
<223> /note="Description of Artificial Sequence: Synthetic polypeptide"

<400> 9

```
Cys Pro Pro Cys Pro Ala Pro Glu Leu Leu Gly Gly Pro Ser Val Phe
1               5               10              15

Leu Phe Pro Pro Lys Pro Lys Asp Thr Leu Met Ile Ser Arg Thr Pro
            20              25              30

Glu Val Thr Cys Val Val Val Asp Val Ser His Glu Asp Pro Glu Val
            35              40              45

Lys Phe Asn Trp Tyr Val Asp Gly Val Glu Val His Asn Ala Lys Thr
        50              55              60

Lys Pro Arg Glu Glu Gln Tyr Asn Ser Thr Tyr Arg Val Val Ser Val
65              70              75              80

Leu Thr Val Leu His Gln Asp Trp Leu Asn Gly Lys Glu Tyr Lys Cys
                85              90              95

Lys Val Ser Asn Lys Ala Leu Pro Ala Pro Ile Glu Lys Thr Ile Ser
            100             105             110

Lys Ala Lys Gly Gln Pro Arg Glu Pro Gln Val Tyr Thr Leu Pro Pro
        115             120             125

Ser Arg Glu Glu Met Thr Lys Asn Gln Val Ser Leu Ser Cys Ala Val
    130             135             140

Lys Gly Phe Tyr Pro Ser Asp Ile Ala Val Glu Trp Glu Ser Asn Gly
145             150             155             160

Gln Pro Glu Asn Asn Tyr Lys Thr Thr Pro Pro Val Leu Asp Ser Asp
            165             170             175

Gly Ser Phe Phe Leu Val Ser Lys Leu Thr Val Asp Lys Ser Arg Trp
            180             185             190

Gln Gln Gly Asn Val Phe Ser Cys Ser Val Met His Glu Ala Leu His
        195             200             205

Asn His Tyr Thr Gln Lys Ser Leu Ser Leu Ser Pro Gly Lys
    210             215             220
```

<210> 10
<211> 222
<212> PRT
<213> Artificial Sequence

<220>
<221> source
<223> /note="Description of Artificial Sequence: Synthetic polypeptide"

<400> 10

```
Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
        50                  55                  60

Lys Pro Arg Glu Glu Gln Tyr Asn Ser Thr Tyr Arg Val Val Ser Val
```

```
           65                      70                      75                      80


       Leu Thr Val Leu His Gln Asp Trp Leu Asn Gly Lys Glu Tyr Lys Cys
                       85                  90                  95

       Lys Val Ser Asn Lys Ala Leu Pro Ala Pro Ile Glu Lys Thr Ile Ser
                   100                 105                 110

       Lys Ala Lys Gly Gln Pro Arg Glu Pro Gln Val Tyr Thr Leu Pro Pro
                   115                 120                 125

       Ser Arg Glu Glu Met Thr Lys Asn Gln Val Ser Leu Trp Cys Leu Val
           130                 135                 140

       Lys Gly Phe Tyr Pro Ser Asp Ile Ala Val Glu Trp Glu Ser Asn Gly
       145                 150                 155                 160

       Gln Pro Glu Asn Asn Tyr Lys Thr Thr Pro Pro Val Leu Asp Ser Asp
                   165                 170                 175

       Gly Ser Phe Phe Leu Tyr Ser Lys Leu Thr Val Asp Lys Ser Arg Trp
                   180                 185                 190

       Gln Gln Gly Asn Val Phe Ser Cys Ser Val Met His Glu Ala Leu His
                   195                 200                 205

       Asn His Tyr Thr Gln Lys Ser Leu Ser Leu Ser Pro Gly Lys
                   210                 215                 220
```

<210> 11
<211> 449
<212> PRT
<213> Artificial Sequence

<220>
<221> source
<223> /note="Description of Artificial Sequence: Synthetic polypeptide"

<400> 11

```
Glu Val Gln Leu Val Glu Ser Gly Gly Gly Leu Val Gln Pro Gly Gly
1               5               10              15

Ser Leu Arg Leu Ser Cys Ala Ala Ser Gly Tyr Thr Phe Thr Ser Tyr
            20              25              30

Trp Leu His Trp Val Arg Gln Ala Pro Gly Lys Gly Leu Glu Trp Val
        35              40              45

Gly Met Ile Asp Pro Ser Asn Ser Asp Thr Arg Phe Asn Pro Asn Phe
```

```
              50                        55                        60

Lys Asp Arg Phe Thr Ile Ser Ala Asp Thr Ser Lys Asn Thr Ala Tyr
65                  70                  75                      80

Leu Gln Met Asn Ser Leu Arg Ala Glu Asp Thr Ala Val Tyr Tyr Cys
                85                  90                  95

Ala Thr Tyr Arg Ser Tyr Val Thr Pro Leu Asp Tyr Trp Gly Gln Gly
            100                 105                 110

Thr Leu Val Thr Val Ser Ser Ala Ser Thr Lys Gly Pro Ser Val Phe
            115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
            165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
            195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
            245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
            275                 280                 285

Ala Lys Thr Lys Pro Arg Glu Glu Gln Tyr Asn Ser Thr Tyr Arg Val
    290                 295                 300
```

```
Val Ser Val Leu Thr Val Leu His Gln Asp Trp Leu Asn Gly Lys Glu
305             310             315                 320

Tyr Lys Cys Lys Val Ser Asn Lys Ala Leu Pro Ala Pro Ile Glu Lys
            325             330                 335

Thr Ile Ser Lys Ala Lys Gly Gln Pro Arg Glu Pro Gln Val Tyr Thr
            340             345                 350

Leu Pro Pro Ser Arg Glu Glu Met Thr Lys Asn Gln Val Ser Leu Ser
            355             360                 365

Cys Ala Val Lys Gly Phe Tyr Pro Ser Asp Ile Ala Val Glu Trp Glu
    370             375                 380

Ser Asn Gly Gln Pro Glu Asn Asn Tyr Lys Thr Thr Pro Pro Val Leu
385             390                 395                 400

Asp Ser Asp Gly Ser Phe Phe Leu Val Ser Lys Leu Thr Val Asp Lys
                405             410                 415

Ser Arg Trp Gln Gln Gly Asn Val Phe Ser Cys Ser Val Met His Glu
            420             425                 430

Ala Leu His Asn His Tyr Thr Gln Lys Ser Leu Ser Leu Ser Pro Gly
            435             440                 445

Lys
```

<210> 12
<211> 220
<212> PRT
<213> Artificial Sequence

<220>
<221> source
<223> /note="Description of Artificial Sequence: Synthetic polypeptide"

<400> 12

```
Asp Ile Gln Met Thr Gln Ser Pro Ser Ser Leu Ser Ala Ser Val Gly
1               5               10                  15

Asp Arg Val Thr Ile Thr Cys Lys Ser Ser Gln Ser Leu Leu Tyr Thr
            20              25                  30

Ser Ser Gln Lys Asn Tyr Leu Ala Trp Tyr Gln Gln Lys Pro Gly Lys
            35              40                  45
```

```
          Ala Pro Lys Leu Leu Ile Tyr Trp Ala Ser Thr Arg Glu Ser Gly Val
              50              55              60

          Pro Ser Arg Phe Ser Gly Ser Gly Ser Gly Thr Asp Phe Thr Leu Thr
          65              70              75              80

          Ile Ser Ser Leu Gln Pro Glu Asp Phe Ala Thr Tyr Tyr Cys Gln Gln
                          85              90              95

          Tyr Tyr Ala Tyr Pro Trp Thr Phe Gly Gln Gly Thr Lys Val Glu Ile
                  100             105             110

          Lys Arg Thr Val Ala Ala Pro Ser Val Phe Ile Phe Pro Pro Ser Asp
                  115             120             125

          Glu Gln Leu Lys Ser Gly Thr Ala Ser Val Val Cys Leu Leu Asn Asn
              130             135             140

          Phe Tyr Pro Arg Glu Ala Lys Val Gln Trp Lys Val Asp Asn Ala Leu
          145             150             155             160

          Gln Ser Gly Asn Ser Gln Glu Ser Val Thr Glu Gln Asp Ser Lys Asp
                          165             170             175

          Ser Thr Tyr Ser Leu Ser Ser Thr Leu Thr Leu Ser Lys Ala Asp Tyr
                  180             185             190

          Glu Lys His Lys Val Tyr Ala Cys Glu Val Thr His Gln Gly Leu Ser
              195             200             205

          Ser Pro Val Thr Lys Ser Phe Asn Arg Gly Glu Cys
              210             215             220
```

<210> 13
<211> 227
<212> PRT
<213> Artificial Sequence

<220>
<221> source
<223> /note="Description of Artificial Sequence: Synthetic polypeptide"

<400> 13

```
          Asp Lys Thr His Thr Cys Pro Pro Cys Pro Ala Pro Glu Leu Leu Gly
          1               5               10              15

          Gly Pro Ser Val Phe Leu Phe Pro Pro Lys Pro Lys Asp Thr Leu Met
                          20              25              30
```

```
Ile Ser Arg Thr Pro Glu Val Thr Cys Val Val Val Asp Val Ser His
        35          40              45

Glu Asp Pro Glu Val Lys Phe Asn Trp Tyr Val Asp Gly Val Glu Val
        50          55              60

His Asn Ala Lys Thr Lys Pro Arg Glu Glu Gln Tyr Asn Ser Thr Tyr
65          70              75              80

Arg Val Val Ser Val Leu Thr Val Leu His Gln Asp Trp Leu Asn Gly
            85              90              95

Lys Glu Tyr Lys Cys Lys Val Ser Asn Lys Ala Leu Pro Ala Pro Ile
        100             105             110

Glu Lys Thr Ile Ser Lys Ala Lys Gly Gln Pro Arg Glu Pro Gln Val
        115             120             125

Tyr Thr Leu Pro Pro Ser Arg Glu Glu Met Thr Lys Asn Gln Val Ser
    130             135             140

Leu Trp Cys Leu Val Lys Gly Phe Tyr Pro Ser Asp Ile Ala Val Glu
145             150             155             160

Trp Glu Ser Asn Gly Gln Pro Glu Asn Asn Tyr Lys Thr Thr Pro Pro
            165             170             175

Val Leu Asp Ser Asp Gly Ser Phe Phe Leu Tyr Ser Lys Leu Thr Val
        180             185             190

Asp Lys Ser Arg Trp Gln Gln Gly Asn Val Phe Ser Cys Ser Val Met
        195             200             205

His Glu Ala Leu His Asn His Tyr Thr Gln Lys Ser Leu Ser Leu Ser
210             215             220

Pro Gly Lys
225
```

**Description**

**FIELD OF THE INVENTION**

The present invention concerns cancer biomarkers. In particular, the invention concerns c-met as a biomarker for patient selection and prognosis in cancer, as well as methods of therapeutic treatment, use of diagnostic kits and methods of dertermining c-met biomarker expression.

**BACKGROUND**

Cancer remains to be one of the most deadly threats to human health. In the U.S., cancer affects nearly 1.3 million new patients each year, and is the second leading cause of death after heart disease, accounting for approximately 1 in 4 deaths. For example, breast cancer is the second most common form of cancer and the second leading cancer killer among American women. It is also predicted that cancer may surpass cardiovascular diseases as the number one cause of death within 5 years. Solid tumors are responsible for most of those deaths. Although there have been significant advances in the medical treatment of certain cancers, the overall 5-year survival rate for all cancers has improved only by about 10% in the past 20 years. Cancers, or malignant tumors, metastasize and grow rapidly in an uncontrolled manner, making timely detection and treatment extremely difficult.
Despite the significant advancement in the treatment of cancer, improved therapies are still being sought.
Goetsch et al. (2010) Biomarkers in Medicine 4, 149-170 describe c-Met expression or amplification as a prognostic marker, studies using cancer cell lines and suggestions for how to approach identifying c-Met stratification (predictive) biomarkers. Smolen et al. (2006) PNAS 103, 2316-2321 describe that MET amplification may identify a subset of epithelial cancers and thus may define a patient group that is appropriate for clinical trials of targeted therapy using MET inhibitors. Okuda et al. (2008) Cancer Science 99, 2280-2285 describe that a minor subset of patients with c-Met amplification may be good candidates for c-Met tyrosine kinase inhibitors. Kamiya (2008) "Human c-Met ELISA for the quantitative determination of c-Met in human serum, EDTA-plasma or cell culture media", no. KT-444, pages 1-6 describes a human c-Met ELISA kit.

**SUMMARY OF THE INVENTION**

This disclosure concerns uses af a c-met antagonist for effectively treating cancer patients and better methods for diagnosing disease for use in treating the disease optionally with c-met antagonist. In particular, the disclosure provides data from a randomized phase It clinical trial ofanti-c-met antibody MetMAb. (onartuzumab) in combination with erlotinib (TARCEVA®) in subjects with second- and third-line non-small cell lung cancer (NSCLC). C-met biomarker was used to identify a patient population in which MetMAb plus erlotinib treatment provided clinically meaningful benefit, evaluated by progression-free survival and overall survival, and a patient population in which MetMAb plus erlotinib treatment significantly increased the risk of cancer progression and death (compared to treatment with erlotinib alone). This worse outcome underscores the need to select patients who will benefit from treatment with c-met antagonist *(e.g.,* in combination with EGFR antagonist).
In the clinical trial, treatment with MetMAb and erlotinib provided a clinically meaningful benefit to patients with NSCLC that expressed high levels of c-met biomarker. The results showed that the efficacy, as evaluated by progression free survival (PFS) and overall survival (OS), was positive especially when compared to PFS and OS data for erlotinib treatment alone. The difference was statistically significant, and the addition of MetMAb to erlotinib nearly doubled the progression free and overall survival in patients with NSCLC that expressed high levels of c-met biomarker. The clinical trial data also showed that treatment with MetMAb in combination with erlotinib increased the risk of progression and death in patients with NSCLC that expressed low levels of c-met biomarker, relative to risk of progression and death in such patients treated with erlotinib alone. The results showed that the efficacy, as evaluated by PFS and OS, was worse in the MetMAb and erlotinib treated patients when compared with PFS and OS data for erlotinib treatment alone. The difference was statistically significant.
The clinical trial data also showed that high (also termed "elevated") c-met expression was strongly associated with a worse prognosis in erlotinib-treated NSCLC patients. Patients with NSCLC that expressed high c-met biomarker had increased risk of progression and approximately double the risk of death relative to patients with NSCLC that expressed low c-met biomarker. Thus, high c-met expression was a strongly significant prognostic factor for progression and survival in erlotinib-treated second- or third-line NSCLC patients.
In one aspect, the invention provides methods for identifying a cancer patient who is likely to respond to treatment with a c-met antagonist comprising the step of determining whether the patient's cancer has a high amount of c-met biomarker, wherein the c-met biomarker expression indicates that the patient is likely to respond to treatment with the c-met antagonist as defined in the claims. As used herein, "elevated" or "high" c-met refers to an amount of c-met associated with patient responsiveness to a treatment.

In another aspect, the invention provides methods for determining cancer patient prognosis, comprising the step of determining whether the patient's cancer has a high amount of c-met biomarker, wherein the c-met biomarker expression indicates that the patient is likely to have increased overall survival (OS) and/or progression-free survival (PFS) when the patient is treated with a c-met antagonist as defined in the claims.

In another aspect, the invention provides methods for determining c-met biomarker expression, comprising the step of determining whether a patient's cancer has a high amount of c-met biomarker, wherein c-met biomarker expression is protein expression and is determined in a sample from the patient using IHC, wherein high c-met biomarker expression is 50% or more of the tumor cells with moderate c-met staining intensity, combined moderate/high c-met staining intensity or high c-met staining intensity, wherein c-met expression is detected using a c-met antibody, wherein the c-met biomarker expression indicates that the patient is likely to have increased OS and/or PFS when the patient is treated with a c-met antagonist as defined in the claims.

In one aspect, the invention provides a method of determining patient prognosis, comprising determining amount of c-met biomarker in a patient cancer sample as defined in the claims. High c-met biomarker expression may means increased PFS and/or OS when the patient is treated with a combination of anti-c-met antibody and erlotinib and low c-met biomarker expression may means decreased PFS and/or OS when the patient is treated with a combination of anti-c-met antibody and erlotinib.

In one aspect, the disclosure provides a method of optimizing therapeutic efficacy comprising determining amount of c-met biomarker expression in a patient cancer sample.

In the methods of the invention involving high c-met biomarker expression, c-met biomarker protein expression is determined in a sample from the patient using immunohistochemistry (IHC) in which the IHC score is 2 or 3. In some embodiments, the IHC score is 2. In some embodiments, the IHC score is 3. High c-met biomarker expression is 50% or more of the tumor cells with moderate c-met staining intensity, combined moderate/high c-met staining intensity or high c-met staining intensity. C-met expression staining intensity is determined relative to c-met staining intensity of control cell pellets. Cell line A549 has moderate c-met staining intensity and, cell line H441 has strong c-met staining intensity. C-met biomarker expression may be nucleic acid expression and is determined in a sample from the patient using gene expression profiling, PCR (such as rtPCR), RNN-seq, microarray analysis, SAGE, MassARRAY technique, or FISH. In some embodiments, the patient whose cancer has high amounts of c-met biomarker has increased likelihood of greater PFS and/or OS relative to a patient whose cancer does not have high c-met biomarker. In some embodiments of any of the inventions disclosed herein, high c-met biomarker expression is met diagnostic positive (met diagnostic positive clinical status) as defined in accordance with Table A herein.

In another aspect, the invention provides methods for identifying a cancer patient who is less likely to be respond to treatment with a c-met antagonist comprising the step of determining whether the patient's cancer has a low amount of c-met biomarker, wherein the c-met biomarker expression indicates that the patient is less likely to respond to treatment with the c-met antagonist as defined herein. As used herein, a "low" amount of c-met refers to an amount of c-met associated with lack of response to a treatment, or, in some embodiments, an amount of c-met associated with worse response to a treatment (e.g. decreased clinical benefit compared to no treatment). C-met biomarker expression is protein expression and is determined in a sample from the patient using immunohistochemistry (IHC) in which the IHC score is 1 or 0. In some embodiments, the IHC score is 1. In some embodiments, the IHC score is 0. Low c-met biomarker expression is negative c-met staining, less than 50% of tumor cells with weak or combined weak and moderate c-met staining intensity, or 50% or more tumor cells with weak or combined weak and moderate c-met staining intensity but less than 50% tumor cells with moderate or combined moderate and strong c-met staining intensity. C-met expression staining intensity is determined relative to c-met staining intensity of control cell pellets. Cell line H1155 has negative c-met staining intensity and cell line HEK-293 has low c-met staining intensity. In some embodiments of any of the inventions disclosed herein, low c-met biomarker expression is met diagnostic negative (met diagnostic negative clinical status) as defined in accordance with Table A herein.

According to one embodiment, the invention concerns a c-met antagonist for use in methods for treating a patient with cancer comprising administering a therapeutically effective amount of a c-met antagonist to the patient if the patient has been found to have an elevated (high) amount of c-met (i.e. expression high c-met biomarker) as defined in the claims.

In one aspect, the invention provides said use in methods for treating a patient with cancer comprising administering a therapeutically effective amount of a c-met antagonist to the patient if the patient has been found to have a high amount of a c-met biomarker where the patient's cancer has been found to have a high amount of a c-met biomarker. In some embodiments, the c-met antagonist is an anti-c-met antibody. In some embodiments, the anti-c-met antibody is MetMAb (onartuzumab). C-met biomarker protein expression is determined in a sample from the patient using immunohistochemistry (IHC). In some embodiments, IHC score is 2 or 3. In some embodiments, the IHC score is 2. In some embodiments, the IHC score is 3. High c-met biomarker expression is 50% or more of the tumor cells with moderate c-met staining intensity, combined moderate/high c-met staining intensity or high c-met staining intensity. C-met expression staining intensity is determined relative to c-met staining intensity of control cell pellets. Cell line A549 has moderate c-met staining intensity and cell line H441 has strong c-met staining intensity. C-met biomarker expression may be nucleic acid expression and is determined in a sample from the patient using gene expression profiling, PCR (such as rtPCR), RNN-seq, microarray analysis, SAGE, MassARRAY technique, or FISH. In some embodiments, the patient has (has greater likelihood of) greater PFS and/or OS relative to a patient who does not have high c-met biomarker. In some embodiments of any of the inventions disclosed herein, high c-met biomarker expression is met diagnostic positive clinical status as defined in accordance with Table A herein.

In addition, the disclosure concerns methods for treating a patient with cancer comprising administering to the patient a therapeutically effective amount of a cancer medicament other than a c-met antagonist, if the patient has been found to have a low (i.e., low or substantially undetectable) amount of c-met biomarker.

In another aspect, the disclosure provides methods for treating a patient with cancer comprising administering a therapeutically effective amount of a cancer medicament other than a c-met antagonist to the patient who has been found to express low c-met biomarker (i.e., have a low amount of c-met biomarker) where the patient's cancer has been found to express low c-met biomarker. C-met biomarker protein expression is determined in a sample from the patient using immunohistochemistry (IHC). In some embodiments, the IHC score is 1 or 0. In some embodiments, the IHC score is 1. In some embodiments, the IHC score is 0. Low c-met biomarker expression is detected by the presence of negative c-met staining, less than 50% of tumor cells with weak or combined weak and moderate c-met staining intensity, or 50% or more tumor cells with weak or combined weak and moderate c-met staining intensity but less than 50% tumor cells with moderate or combined moderate and strong c-met staining intensity. C-met expression staining intensity is determined relative to c-met staining intensity of control cell pellets. Cell line H1155 has negative c-met staining and cell line HEK-293 has low c-met staining intensity. In some embodiments of any of the inventions disclosed herein, low c-met biomarker expression is met diagnostic negative clinical status as defined in accordance with Table A herein.

The invention also relates to methods for selecting a therapy for a patient with cancer comprising determining expression of c-met biomarker in a sample from the patient, and selecting a cancer medicament based on the level of expression of the biomarker as defined in the claims. In one embodiment, the patient is selected for treatment with a c-met antagonist (e.g., anti-c-met antibody) if the cancer sample expresses c-met biomarker at a high level. In some embodiments, the patient is treated for cancer using therapeutically effective amount of the c-met antagonist. Thus, in some embodiments, the patient is selected for treatment with a c-met antagonist (e.g., anti-c-met antibody) if the patient's cancer sample expresses c-met biomarker at a high level, and (following the selection) the patient is treated for cancer using therapeutically effective amount of the c-met antagonist. In another embodiment, the patient is selected for treatment with a cancer medicament other than c-met antagonist if the cancer sample expresses c-met biomarker at a low level (e.g., the cancer sample expresses low or substantially undetectable levels of the biomarker). In some embodiments, the patient is treated for cancer using therapeutically effective amounts of the cancer medicament other than c-met antagonist. Thus, in some embodiments, the patient is selected for treatment with a cancer medicament other than c-met antagonist (e.g., EGFR antagonist, e.g., erlotinib) if the cancer sample expresses c-met biomarker at a low (i.e., low or substantially undetectable) level, and (following the selection) the patient is treated for cancer using therapeutically effective amount of the c-met antagonist.

Moreover, the disclosure concerns methods for advertising a cancer medicament (e.g., a c-met antagonist) comprising promoting, to a target audience, the use of the cancer medicament for treating a patient with cancer based on expression of c-met biomarker. Promotion may be conducted by any means available. In some embodiments, the promotion is by a package insert accompanying a commercial formulation of the c-met antagonist (such as an anti-c-met antibody). The promotion may also be by a package insert accompanying a commercial formulation of a second medicament (when treatment is combination therapy with a c-met antagonist and a second medicament, e.g., an EGFR antagonist such as erlotinib). Promotion may be by written or oral communication to a physician or health care provider. In some embodiments, the promotion is by a package insert where the package insert provides instructions to receive therapy with c-met antagonist, and in some embodiments, in combination with a second medicament, such as an EGFR antagonist (such as erlotinib) or, in other embodiments, an anti-VEGF antibody. In some embodiments, the promotion is followed by the treatment of the patient with the c-met antagonist with or without the second medicament (e.g., erlotinib). In some embodiments, the promotion is followed by the treatment of the patient with the second medicament with or without treatment with c-met antagonist. In some embodiments, the package insert indicates that the c-met antagonist is to be used to treat the patient if the patient's cancer sample expressed high c-met biomarker. In some embodiments, the package insert indicates that the c-met antagonist is not to be used to treat the patient if the patient's cancer sample expresses low c-met biomarker. In some embodiments, high c-met biomarker means likelihood of increased PFS and/or OS when the patient is treated with the c-met antagonist (or in some embodiments, treated with c-met antagonist in combination with EGFR antagonist). In some embodiments, low c-met biomarker means likelihood of decreased PFS and OS when the patient is treated with the c-met antagonist (or in some embodiments, treated with c-met antagonist in combination with EGFR antagonist). In some embodiments, the PFS and/or OS is decreased relative to a patient who is not treated with the c-met antagonist (or in some embodiments, treated with c-met antagonist in combination with EGFR antagonist). In some embodiments, the promotion is by a package insert where the package inset provides instructions to receive therapy with anti-c-met antibody in combination with an EGFR antagonist. In some embodiments, the promotion is followed by the treatment of the patient with the anti-c-met antibody with or without the second medicament.

In some aspects, the disclosure features methods of instructing a patient with cancer (such as NSCLC) expressing high levels of c-met biomarker by providing instructions to receive treatment with a c-met antagonist (for example, an anti-c-met antibody), and in some embodiments, treatment with a second medicament (such as EGFR antagonist, e.g. erlotinib), for example, to increase survival of the patient, to decrease the patient's risk of cancer recurrence and/or to increase the patient's likelihood of survival. In some embodiments, the treatment comprises administering to the NSCLC patient an anti-c-met antibody (*e.g.,* MetMAb) administered in combination with an EGFR antagonist, such as erlotinib. In some embodiments the method further comprises providing instructions to receive treatment with at least one chemotherapeutic agent. In certain embodiments the patient is treated as instructed by the method of instructing. In some embodiments, the package insert indicates that the c-met antagonist is to be used to treat the patient if the patient's cancer sample expressed high c-met biomarker. In some embodiments, the instructions indicate that the c-met antagonist is not to be used to treat the patient if the patient's cancer sample expresses low c-met biomarker, wherein low c-met biomarker means decreased PFS and OS when the patient is treated with the c-met antagonist (or in some embodiments, treated with c-met antagonist in combination with EGFR antagonist). In some embodiments, the PFS and/or OS is decreased relative to a patient who is not treated with the c-met antagonist (or in some embodiments, treated with c-met antagonist in combination with EGFR antagonist).

The disclosure also provides business methods, comprising marketing an c-met antagonist (e.g., anti-c-met antibody) for treatment of cancer (e.g., NSCLC) in a human patient, wherein the patient's cancer expressed high (elevated) c-met biomarker expression, for example, to increase survival, decrease the patient's likelihood of cancer recurrence, and/or increase the patient's likelihood of survival. In some embodiments, the treatment comprises administering to a cancer patient an anti-c-met antibody (e.g., MetMAb), and in some embodiment, a second medicament (e.g., an EGFR antagonist, such as erlotinib). In some embodiments, the marketing is followed by the treatment of the patient with the c-met antagonist (such as anti-c-met antibody) and in some embodiments, treatment with anti-c-met antibody and/or EGFR antagonist. In some aspects, the invention features a method of instructing a patient with cancer (such as NSCLC) expressing low (i.e., low or substantially undetectable) levels of c-met biomarker by providing instructions to receive treatment with a cancer medicament other than a c-met antagonist. In certain embodiments the patient is treated as instructed by the method of instructing.

In one aspect, the invention provides a c-met antagonist for use for the treatment of a cancer patient, wherein the patient expresses a high amount of c-met biomarker as defined in the claims. In some embodiments, the patient's cancer expresses a high amount of c-met biomarker.

In one aspect, the invention provides in vitro use of a c-met IHC assay for identifying a cancer patient suitable for treatment with a c-met antagonist, wherein the patient expresses a high amount of c-met biomarker. In some embodiments, the patient's cancer expresses a high amount of c-met biomarker.

In one aspect, the invention concerns diagnostic kits comprising one or more reagent for determining expression of a c-met biomarker in a sample from a cancer (e.g., NSCLC) patient. The diagnostic kit is suitable for use with any of the methods described herein. In some embodiments, detection of high c-met biomarker means increased PFS and/or OS when the patient is treated with a c-met antagonist. In some embodiments, detection of low c-met biomarker means a decreased PFS and/or OS when the patient is treated with the c-met antagonist. In some embodiments, the kit further comprises instructions to use the kit to select a c-met medicament to treat the NSCLC patient.

In one aspect, the invention provides use of a diagnostic kit for identifying a cancer patient suitable for treatment with a c-met antagonist, wherein the patient expresses a high amount of c-met biomarker as defined in the claims. In some embodiments, the patient's cancer expresses a high amount of c-met biomarker.

The invention also concerns articles of manufacture comprising, packaged together, a c-met antagonist in a pharmaceutically acceptable carrier and a package insert indicating that the c-met antagonist is for treating a patient with cancer based on expression of c-met biomarker. Treatment methods include any of the treatment methods disclosed herein. In some embodiments, the package insert indicates that the c-met antagonist is to be used to treat the patient if the patient's cancer sample expressed high c-met biomarker. In some embodiments, the PFS and/or OS is likely increased relative to a patient who is not treated with the c-met antagonist (or in some embodiments, treated with c-met antagonist in combination with EGFR antagonist). In some embodiments, the package insert indicates that the c-met antagonist is not to be used to treat the patient if the patient's cancer sample expresses low c-met biomarker. In some embodiments, low c-met biomarker means decreased PFS and OS when the patient is treated with the c-met antagonist (or in some embodiments, treated with c-met antagonist in combination with EGFR antagonist). In some embodiments, the PFS and/or OS is likely decreased relative to a patient who is not treated with the c-met antagonist (or in some embodiments, treated with c-met antagonist in combination with EGFR antagonist).

In a related aspect, the invention concerns methods for manufacturing an article of manufacture comprising combining in a package a pharmaceutical composition comprising a cancer medicament and a package insert indicating that the pharmaceutical composition is for treating a patient with cancer based on expression of c-met biomarker. Treatment methods include any of the treatment methods disclosed herein. In some embodiments, the package insert indicates that the c-met antagonist is to be used to treat the patient if the patient's cancer sample expressed high c-met biomarker. In some embodiments, the package insert indicates that the c-met antagonist is not to be used to treat the patient if the patient's cancer sample expresses low c-met biomarker. In some embodiments, low c-met biomarker means increased likelihood of decreased PFS and OS when the patient is treated with the c-met antagonist (or in some embodiments, treated with c-met antagonist in combination with EGFR antagonist). In some embodiments, the PFS and/or OS is decreased relative to a patient who is not treated with the c-met antagonist (or in some embodiments, treated with c-met antagonist in combination with EGFR antagonist).

In certain embodiments of any of the inventions described herein, the cancer may be non-small cell lung cancer (including *e.g.*, squamous cell carcinoma (SCC)), renal cell cancer, pancreatic cancer, gastric carcinoma, bladder cancer, esophageal cancer, mesothelioma, melanoma, breast cancer (including triple-negative breast cancer), thyroid cancer, colorectal cancer, head and neck cancer, osteosarcoma, prostate cancer, or glioblastoma. In some embodiments, the cancer is NSCLC. In some embodiments, the NSCLC is second-line or third-line locally advanced or metastatic non-small cell lung cancer. In some embodiments, the NSCLC is adenocarcinoma. In some embodiments, the NSCLC is squamous cell carcinoma. Other exemplary cancers are described herein. In some embodiments, the NSCLC is locally advanced or metastatic NSCLC after failure of at least one prior chemotherapy regimen.

In certain embodiments, the patient did not receive more than two prior treatments for Stage IIIB/IV. In some embodiments, the patient did not receive more than 30 days of exposure to an investigational or marketed agent that can act by EGFR inhibition, or a known EGFR-related toxicity resulting in dose modifications. EGFR inhibitors include (but are not limited to) gefitinib, erlotinib, and cetuximab. In some embodiments, the patient did not receive chemotherapy, biologic therapy, radiotherapy or investigational drug within 28 days prior to randomization (except that optionally, kinase inhibitors may be used within two weeks prior to randomization provided any drug related toxicity was adequately resolved). In some embodiments, the patient is not a patient with untreated and/or active (progressing or requiring anticonvulsants or corticosteroids for symptomatic control) CNS metastasis. In some embodiments, a sample of the patient's cancer has been shown to have wildtype EGFR. In some embodiments, a sample of the patient's cancer has not been shown to have mutated EGFR. Other patient exclusion criteria are described in the Examples, and the present inventions contemplate use of one or more of the exclusions described therein.

C-met antagonists, *e.g.,* suitable for use in any of the inventions described herein, are known in the art and some are further described herein. In certain embodiments, the c-met antagonist is an antagonist anti-c-met antibody. In certain embodiments, the anti-c-met antibody comprises a (a) HVR1 comprising sequence GYTFTSYWLH (SEQ ID NO: 1); (b) HVR2 comprising sequence GMIDPSNSDTRFNPNFKD (SEQ ID NO: 2); (c) HVR3-HC comprising sequence ATYRSYVTPLDY (SEQ ID NO: 3); (d) HVR1-LC comprising sequence KSSQSLLYTSSQKNYLA (SEQ ID NO: 4); (e) HVR2-LC comprising sequence WASTRES (SEQ ID NO: 5); and (f) HVR3-LC comprising sequence QQYYAYPWT (SEQ ID NO: 6). In certain embodiments, the anti-c-met antibody is monovalent and comprises (a) a first polypeptide comprising a heavy chain, said polypeptide comprising the sequence of SEQ ID NO: 11; (b) a second polypeptide comprising a light chain, the polypeptide comprising the sequence of SEQ ID NO: 12; and a third polypeptide comprising a Fc sequence, the polypeptide comprising the sequence of SEQ ID NO: 13, wherein the heavy chain variable domain and the light chain variable domain are present as a complex and form a single antigen binding arm, wherein the first and second Fc polypeptides are present in a complex and form a Fc region that increases stability of said antibody fragment compared to a Fab molecule comprising said antigen binding arm. In certain embodiments, the c-met antibody is MetMAb (interchangeably termed "onartuzumab"). In certain embodiments, the c-met antagonist is any one or more of crizotinib, tivantinib, carbozantinib, MGCD-265, ficlatuzumab, humanized TAK-701, rilotumumab, foretinib, h224G11, DN-30, MK-2461, E7050, MK-8033, PF-4217903, AMG208, JNJ-38877605, EMD1204831, INC-280, LY-2801653, SGX-126, RP1040, LY2801653, BAY-853474, and/or LA480. Other c-met antagonists suitable for use in the present inventions are described herein.

Cancer medicaments can be used either alone or in combination with other cancer medicaments. For example, in some embodiments, a c-met antagonist *(e.g.,* anti-c-met antibody) is used in combination with an EGFR antagonist *(e.g.,* erlotinib). In certain embodiments, erlotinib is administered at a dose of 150 mg, each day of a three week cycle. In certain embodiments, erlotinib is administered at a dose of 100 mg, each day of a three week cycle. In certain embodiments, erlotinib is administered at a dose of 50 mg, each day of a three week cycle. An exemplary protocol is administering to a NSCLC patient (a) an anti-c-met antibody (such as MetMAb) at a dose of about 15 mg/kg every three weeks; and (b) erlotinib (N-(3-ethynylphenyl)-6,7-bis(2-methoxyethoxy)-4-quinazolinamine) at a dose of 150 mg, each day of a three week cycle. In other embodiments, a c-met antagonist *(e.g.,* anti-c-met antibody) is used in combination with an anti-VEGF antibody and chemotherapy *(e.g.,* a taxane). An exemplary protocol is administering to a triple-negative metastatic breast cancer patient an anti-c-met antibody *(e.g.,* MetMAb) administered at a dose of 10 mg/kg on Day 1 and Day 15 of a 28-day cycle, anti-VEGF antibody *(e.g.,* bevacizumab) administered at a dose of 10 mg/kg on Day 1 and Day 15 of the 28-day cycle and paclitaxel administered at a dose of 90 mg/m$^2$ by IV infusion on Day 1, Day 8, and Day 15 of the 28-day cycle. Another exemplary protocol is administering to a triple-negative metastatic breast cancer patient an anti-c-met antibody *(e.g.,* MetMAb) administered at a dose of 10 mg/kg on Day 1 and Day 15 of a 28-day cycle, and paclitaxel administered at a dose of 90 mg/m$^2$ by IV infusion on Day 1, Day 8, and Day 15 of the 28-day cycle. In certain embodiments, MetMAb is administered at a dose of about 15 mg/kg every three weeks, or at a dose of about 10 mg/kg every two weeks. In some embodiments, crizotinib is used in combination with an EGFR antagonist (in some embodiments, erlotinib). In some embodiments, carbozantinib is used in combination with an EGFR antagonist (in some embodiments, erlotinib). In some embodiments, foretinib is used in combination with an EGFR antagonist (in some embodiments, erlotinib). In some embodiments, tivantinib is used in combination with an EGFR antagonist (in some embodiments, erlotinib). In some embodiments, MGCD-265 is used in combination with an EGFR antagonist (in some embodiments, erlotinib). In some embodiments, rilotumumab is used in combination with an EGFR antagonist (in some embodiments, erlotinib). In some embodiments, ficlatuzumab is used in combination with an EGFR antagonist (in some embodiments, erlotinib). In some embodiments, humanized anti-HGF antibody TAK-701 is used in combination with an EGFR antagonist (in some embodiments, erlotinib). Other cancer medicaments are described herein.

Detection of c-met biomarker is disclosed and exemplified herein. In some embodiments of any of the inventions described herein, high expression of a c-met biomarker in a patent's cancer is high protein expression and, in further embodiments, is determined using IHC. In some embodiments, the IHC score is 2 or 3. In some embodiments, the IHC score is 3. In some embodiments, the IHC score is 2. In some embodiments, high c-met biomarker is (means) 50% or more tumor cells with moderate c-met staining intensity, combined moderate/high c-met staining intensity or high c-met staining intensity. In some embodiments, high-c-met biomarker is 50% or more of tumor cells with moderate or high c-met staining intensity. In some embodiments, high expression of a c-met biomarker is high mRNA expression (and in some embodiments, detected using qualitative RT-PCR or in situ hybridization). In some embodiments, high expression of a c-met biomarker is c-met gene amplification (and in some embodiments, detected using FISH). In other embodiments, gene expression profiling, PCR (such as rtPCR), RNN-seq, microarray analysis, SAGE, MassARRAY technique, or FISH is used to detect c-met biomarker. In some embodiments, the PFS and/or OS is likely increased *(i.e.,* there is a likelihood of increased PFS and/or OS) relative to a patient who is not treated with the c-met antagonist (or in some embodiments, treated with c-met antagonist in combination with EGFR antagonist). In some embodiments of any of the inventions disclosed herein, high c-met biomarker expression is met diagnostic positive clinical status as defined in accordance with Table A herein.

In some embodiments of any of the inventions described herein, low expression of a c-met biomarker in a patient's cancer is low protein expression and is determined using IHC. In some embodiments, the IHC score is 1. In some embodiments, the IHC score is 0. In some embodiments, the IHC score is 0 or 1. In some embodiments, low c-met biomarker is negative c-met staining, less than 50% of tumor cells with weak or combined weak and moderate c-met staining intensity, or 50% or more tumor cells with weak or combined weak and moderate c-met staining intensity but less than 50% tumor cells with moderate or combined moderate and strong c-met staining intensity. In some embodiments, low c-met biomarker means increased likelihood of decreased PFS and OS when the patient is treated with the c-met antagonist (or in some embodiments, treated with c-met antagonist in combination with EGFR antagonist). In some embodiments, the PFS and/or OS is likely decreased *(i.e.,* there is a likelihood of decreased PFS and/or OS) relative to a patient who is not treated with the c-met antagonist (or in some embodiments, treated with c-met antagonist in combination with EGFR antagonist). In some embodiments of any of the inventions disclosed herein, low c-met biomarker expression is met diagnostic negative clinical status as defined in accordance with Table A herein.

Optionally, additional biomarkers may be detected in a patient's sample. In some embodiments, the patient's cancer has been found to express wildtype EGFR (in some embodiments, further expresses c-met gene amplification, and in still further embodiments, does not express c-met gene amplification)..In certain embodiments, the patient's cancer has been found to express a biomarker selected from kras and EGFR. In some embodiments, the patient's cancer has been found to express mutated kras. In some embodiments, the patient's cancer has been found to express wildtype kras. In some embodiments, the patient's cancer has been found to express mutated EGFR. In some embodiments, the patient's cancer *(e.g.,* the patient's NSCLC) has been found to express an anaplastic lymphoma kinase (ALK) translocation. In some embodiments, the ALK translocation is an EML4-ALK translocation. In some embodiments, the patient's cancer has been found to express mutated c-met. In some embodiments, the patient's cancer has been found to express wildtype c-met.

Further embodiments regarding determination of biomarker (e.g. c-met biomarker) expression are disclosed herein.

In another aspect, the disclosure provides a method for evaluating adverse events in a patient associated with treatment of a cancer that expresses a high amount of c-met biomarker, wherein treatment is with a c-met antagonist (e.g.,) MetMAb (onartuzumab)) and the method comprises the steps of monitoring the number and/or severity of one or more adverse events. Exemplary adverse events are disclosed herein.

## BRIEF DESCRIPTION OF THE FIGURES

The patent or application file contains at least one drawing executed in color. Copies of this patent or patent application publication with color drawing(s) will be provided by the Office upon request and payment of the necessary fee.

Figure 1 shows exemplary IHC analysis of control cell pellets.

Figure 2 shows an example of IHC analysis of NSCLC tumor samples.

Figure 3 shows analysis of treatment of Met high patients with Erlotinib + placebo (solid line) verses Erlotinib + MetMAb (dashed line).

Figure 4 shows analysis of treatment of Met low patients with Erlotinib + placebo (solid line) verses Erlotinib + MetMAb (dashed line).

Figure 5 shows analysis of treatment of all patients with Erlotinib + placebo (solid line) verses Erlotinib + MetMAb (dashed line),

Figure 6 shows PFS examined by subgroups.

Figure 7 shows OS examined by subgroups.

Figure 8 shows analysis of prognosis by Met expression in erlotinib + placebo treated patients. Met low = dashed line; Met high = solid line.

Figure 9 shows subgroup analysis of PFS in Met High patients.

Figure 10 shows subgroup analysis of OS in Met High patients.

Figure 11 shows subgroup analysis of PFS in Met Low patients.

Figure 12 shows subgroup analysis of OS in Met Low patients.

Figure 13 shows final analysis of treatment of Met diagnostic positive patients with Erlotinib + placebo (solid line) verses Erlotinib + MetMAb (dashed line).

Figure 14 shows final analysis of treatment of Met diagnostic negative patients with Erlotinib + placebo (solid line) verses Erlotinib + MetMAb (dashed line).

Figure 15 shows final analysis of treatment of all patients with Erlotinib + placebo (solid line) verses Erlotinib + MetMAb (dashed line).

Figure 16 shows OS examined by subgroups.

Figure 17 shows final analysis of OS in Met diagnostic negative patients

Figure 18 shows OS analysis on certain subpopulations of patients.

Figure 19 shows that Met expression was associated with worse outcome in Erlotinib + placebo treated patients.

Figure 20 shows the relationship of *MET* mRNA levels with met IHC clinical score.

Figure 21 shows the treatment effect of MetMAb in combination with erlotinib evaluated in patients defined using less stringent Met expression cutoff and more stringent Met expression cutoffs. All Hazard ratios were estimated from unstratified analyses.

## DETAILED DESCRIPTION OF EMBODIMENTS OF THE INVENTION

### I. DEFINITIONS

Herein, a "patient" is a human patient. The patient may be a "cancer patient," *i.e.* one who is suffering or at risk for suffering from one or more symptoms of cancer. Moreover, the patient may be a previously treated cancer patient. The patient may be a "NSCLC cancer patient," *i.e.* one who is suffering or at risk for suffering from one or more symptoms of NSCLC. Moreover, the patient may be a previously treated NSCLC patient.

The term "c-met" or "Met", as used herein, refers, unless indicated otherwise, to any native or variant (whether native or synthetic) c-met polypeptide. The term "wild type c-met" generally refers to a polypeptide comprising the amino acid sequence of a naturally occurring c-met protein. The term "wild type c-met sequence" generally refers to an amino acid sequence found in a naturally occurring c-met.

An "anti-c-met antibody" is an antibody that binds to c-met with sufficient affinity and specificity. The antibody selected will normally have a sufficiently strong binding affinity for c-met, for example, the antibody may bind human c-met with a $K_d$ value of between 100 nM-1 pM. Antibody affinities may be determined by a surface plasmon resonance based assay (such as the BIAcore assay as described in PCT Application Publication No. WO2005/012359); enzyme-linked immunoabsorbent assay (ELISA); and competition assays (e.g. RIA's), for example. In certain embodiments, the anti-c-met antibody can be used as a therapeutic agent in targeting and interfering with diseases or conditions wherein c-met activity is involved. Also, the antibody may be subjected to other biological activity assays, e.g., in order to evaluate its effectiveness as a therapeutic. Such assays are known in the art and depend on the target antigen and intended use for the antibody.

A "c-met antagonist" (interchangeably termed "c-met inhibitor") is an agent that interferes with c-met activation or function. Examples of c-met inhibitors include c-met antibodies; HGF antibodies; small molecule c-met antagonists; c-met tyrosine kinase inhibitors; antisense and inhibitory RNA *(e.g.,* shRNA) molecules (see, for example, WO2004/87207). Preferably, the c-met inhibitor is an antibody or small molecule which binds to c-met. In a particular embodiment, a c-met inhibitor has a binding affinity (dissociation constant) to c-met of about 1,000 nM or less. In another embodiment, a c-met inhibitor has a binding affinity to c-met of about 100 nM or less. In another embodiment, a c-met inhibitor has a binding affinity to c-met of about 50 nM or less. In a particular embodiment, a c-met inhibitor is covalently bound to c-met. In a particular embodiment, a c-met inhibitor inhibits c-met signaling with an IC50 of 1,000 nM or less. In another embodiment, a c-met inhibitor inhibits c-met signaling with an IC50 of 500 nM or less. In another embodiment, a c-met inhibitor inhibits c-met signaling with an IC50 of 50 nM or less.

"C-met activation" refers to activation, or phosphorylation, of the c-met receptor. Generally, c-met activation results in signal transduction (e.g. that caused by an intracellular kinase domain of a c-met receptor phosphorylating tyrosine residues in c-met or a substrate polypeptide). C-met activation may be mediated by c-met ligand (HGF) binding to a c-met receptor of interest. HGF binding to c-met may activate a kinase domain of c-met and thereby result in phosphorylation of tyrosine residues in the c-met and/or phosphorylation of tyrosine residues in additional substrate polypeptides(s).

A "population" of subjects refers to a group of subjects with cancer, such as in a clinical trial, or as seen by oncologists following FDA approval for a particular indication, such as breast cancer therapy.

The phrase "does not possess substantial biomarker expression" or "substantially no biomarker expression" with respect to a biomarker, as used herein, means the biomarker does not exhibit an expression level that is above background level that is of statistical significance. The phrase "little to no biomarker expression" with respect to a biomarker, as used herein, means the biomarker does not display a biologically meaningful amount of expression. As would be understood in the art, amount of expression may be determined quantitatively or qualitatively, so long as a comparison between a biomarker sample and a reference counterpart can be done. The expression can be measured or detected according to any assay or technique known in the art, including, *e.g.,* those described herein (such as IHC).

The term "gene amplification" refers to a process by which multiple copies of a gene or gene fragment are formed in a particular cell or cell line.

For the methods of the invention, the term "instructing" a patient means providing directions for applicable therapy, medication, treatment, treatment regimens, and the like, by any means, but preferably in writing, such as in the form of package inserts or other written promotional material.

For the methods of the invention, the term "promoting" means offering, advertising, selling, or describing a particular drug, combination of drugs, or treatment modality, by any means, including writing, such as in the form of package inserts. Promoting herein refers to promotion of therapeutic agent(s), such as an anti-c-met antibody and/or erlotinib, for an indication, such as NSCLC treatment, where such promoting is authorized by the Food and Drug Administration (FDA) as having been demonstrated to be associated with statistically significant therapeutic efficacy and acceptable safety in a population of subjects

The term "marketing" is used herein to describe the promotion, selling or distribution of a product (e.g., drug). Marketing specifically includes packaging, advertising, and any business activity with the purpose of commercializing a product.

For the purposes herein, a "previously treated" cancer patient has received prior cancer therapy.

"Refractory" cancer progresses even though an anti-tumor agent, such as a chemotherapeutic agent, is being administered to the cancer patient.

A "cancer medicament" is a drug effective for treating cancer. Examples of cancer medicaments include the chemotherapeutic agents and chemotherapy regimens noted below; c-met antagonists, including anti-c-met antibodies, such as MetMAb.

The term "biomarker" or "marker" as used herein refers generally to a molecule, including a gene, mRNA, protein, carbohydrate structure, or glycolipid, the expression of which in or on a tissue or cell or secreted can be detected by known methods (or methods disclosed herein) and is predictive or can be used to predict (or aid prediction) for a cell, tissue, or patient's responsiveness to treatment regimes. The biomarker of particular interest herein is c-met. In some embodiments, the c-met biomarker does not include amplification of c-met gene *(e.g.,* an average in a population of cells of 3 or more, 4 or more, or 5 or more copies of c-met gene, or more, such as an average of 8 or more, or 10 or more copies of c-met gene).

By "patient sample" is meant a collection of similar cells obtained from a cancer patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may contain compounds which are not naturally intermixed with the tissue in nature such as preservatives, anticoagulants, buffers, fixatives, nutrients, antibiotics, or the like. Examples of tumor samples herein include, but are not limited to, tumor biopsies, circulating tumor cells, serum or plasma, circulating plasma proteins, ascitic fluid, primary cell cultures or cell lines derived from tumors or exhibiting tumor-like properties, as well as preserved tumor samples, such as formalin-fixed, paraffin-embedded tumor samples or frozen tumor samples. In one embodiment the sample comprises NSCLC *(e.g.,* squamous subtype or nonsquamous subtype) tumor sample.

An "effective response" of a patient or a patient's "responsiveness" to treatment with a medicament and similar wording refers to the clinical or therapeutic benefit imparted to a patient at risk for, or suffering from, cancer *(e.g.,* NSCLC) upon administration of the cancer medicament. Such benefit includes any one or more of: extending survival (including overall survival and progression free survival); resulting in an objective response (including a complete response or a partial response); or improving signs or symptoms of cancer, etc. In one embodiment, the biomarker *(e.g.,* c-met expression, for example, as determined using IHC) is used to identify the patient who is expected to have greater progression free survival (PFS) when treated with a medicament *(e.g.,* anti-c-met antibody), relative to a patient who does not express the biomarker at the same level. In one embodiment, the biomarker is used to identify the patient who is expected to have reduced PFS when treated with a medicament, relative to a patient treated with the medicament who does not express the biomarker at the same level, or relative to a patient who is not treated with the medicament who does not express the biomarker at the same level. In one embodiment, the biomarker is used to identify the patient who is expected to have greater overall survival (OS) when treated with a medicament, relative to a patient who does not express the biomarker at the same level. In one embodiment, the biomarker is used to identify the patient who is expected to have reduced overall survival (OS), relative to a patient who is treated with the medicament who does not express the biomarker at the same level, or relative to a patient who is not treated with the medicament who does not express the biomarker at the same level. The incidence of biomarker(s) herein effectively predicts, or predicts with high sensitivity, such effective response.

"Survival" refers to the patient remaining alive, and includes overall survival as well as progression free survival.

"Overall survival" refers to the patient remaining alive for a defined period of time, such as 1 year, 5 years, etc from the time of diagnosis or treatment.

"Progression free survival" refers to the patient remaining alive, without the cancer progressing or getting worse.

By "extending survival" is meant increasing overall or progression free survival in a treated patient relative to an untreated patient (*i.e.* relative to a patient not treated with the medicament), or relative to a patient who does not express a biomarker at the designated level, and/or relative to a patient treated with an approved anti-tumor agent (such as chemotherapy regimen of erlotinib.

An "objective response" refers to a measurable response, including complete response (CR) or partial response (PR).

By "complete response" or "CR" is intended the disappearance of all signs of cancer in response to treatment. This does not always mean the cancer has been cured.

"Partial response" or "PR" refers to a decrease in the size of one or more tumors or lesions, or in the extent of cancer in the body, in response to treatment.

The "amount" or "level" of a biomarker associated with an increased clinical benefit to a cancer *(e.g.* NSCLC) patient refers to a detectable level in a biological sample wherein the level of biomarker is associated with increased patient clinical benefit. These can be measured by methods known to the expert skilled in the art and also disclosed by this invention. The expression level or amount of biomarker assessed can be used to determine the response to the treatment. In some embodiments, the amount or level of biomarker is determined using IHC *(e.g.,* of patient tumor sample). In some embodiments, amount or level of a c-met biomarker associated with an increased clinical benefit in a cancer patient is an IHC score of 2, an IHC score of 3, or an IHC score of 2 or 3. In some embodiments, amount or level of a c-met biomarker associated with an increased clinical benefit in a cancer patient is 50% or more tumor cells with moderate c-met staining intensity, combined moderate/high c-met staining intensity or high c-met staining intensity. In some embodiments, amount or level of a c-met biomarker associated with an increased clinical benefit in a cancer patient is 50% or more of tumor cells with moderate or high c-met staining intensity.

The "amount" or "level" of a biomarker associated with a decreased clinical benefit to a cancer *(e.g.* NSCLC) patient refers to lack of detectable biomarker or a low detectable level in a biological sample, wherein the level of biomarker is associated with decreased clinical benefit to the patient. These can be measured by methods known to the expert skilled in the art and also disclosed by this invention. The expression level or amount of biomarker assessed can be used to determine the response to the treatment. In some embodiments, the amount or level of biomarker is determined using IHC *(e.g.,* of patient tumor sample). In some embodiments, amount or level of a c-met biomarker associated with a decreased clinical benefit in a cancer patient is an IHC score of 0, an IHC score of 1, or an IHC score of 0 or 1. In some embodiments, an amount or a level of biomark associated with a decreased clinical benefit in a cancer patient is negative c-met staining, less than 50% of tumor cells with weak or combined weak and moderate c-met staining intensity, or 50% or more tumor cells with weak or combined weak and moderate c-met staining intensity but less than 50% tumor cells with moderate or combined moderate and strong c-met staining intensity.

The terms "level of expression" or "expression level" in general are used interchangeably and generally refer to the amount of a polynucleotide, mRNA, or an amino acid product or protein in a biological sample. "Expression" generally refers to the process by which gene-encoded information is converted into the structures present and operating in the cell. Therefore, according to the invention "expression" of a gene may refer to transcription into a polynucleotide, translation into a protein, or even posttranslational modification of the protein. Fragments of the transcribed polynucleotide, the translated protein, or the post-translationally modified protein shall also be regarded as expressed whether they originate from a transcript generated by alternative splicing or a degraded transcript, or from a post-translational processing of the protein, *e.g.,* by proteolysis. In some embodiments, "level of expression" refers to amount of a protein in a biological sample as determined using IHC.

The phrase "based on expression of" when used herein means that information about expression level of the one or more biomarkers herein is used to inform a treatment decision, information provided on a package insert, or marketing/promotional guidance etc. In the case of high expression of the biomarker, patients may be treated with a cancer *(e.g.* NSCLC) medicament such as a c-met antagonist *(e.g.,* anti-c-met antibody, *e.g.,* MetMAb). In the case of reduced level of expression of the biomarker, patients may be treated with a cancer medicament other than c-met antagonist *(e.g.,* anti-c-met antibody, *e.g.* MetMAb).

"Treatment" refers to both therapeutic treatment and prophylactic or preventative measures. Those in need of treatment include those already having a benign, pre-cancerous, or non-metastatic tumor as well as those in which the occurrence or recurrence of cancer is to be prevented.

The term "therapeutically effective amount" refers to an amount of a therapeutic agent to treat or prevent a disease or disorder in a mammal. In the case of cancers, the therapeutically effective amount of the therapeutic agent may reduce the number of cancer cells; reduce the primary tumor size; inhibit *(i.e.,* slow to some extent and preferably stop) cancer cell infiltration into peripheral organs; inhibit *(i.e.,* slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, and/or quality of life. The terms "cancer" and "cancerous" refer to or describe the physiological condition in mammals that is typically characterized by unregulated cell growth. Included in this definition are benign and malignant cancers. By "early stage cancer" or "early stage tumor" is meant a cancer that is not invasive or metastatic or is classified as a Stage 0, I, or II cancer. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include squamous cell cancer *(e.g.* epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer. In some embodiments, the cancer is triple-negative metastatic breast cancer, including any histologically confirmed triple-negative (ER-, PR-, HER2-) adenocarcinoma of the breast with locally recurrent or metastatic disease (where the locally recurrent disease is not amenable to resection with curative intent).

A cancer or biological sample which "displays c-met expression" is one which, in a diagnostic test, expresses (including overexpresses) a c-met receptor.

A cancer or biological sample which "displays c-met amplification" is one which, in a diagnostic test, has amplified c-met gene. In some embodiments, amplified c-met gene is an average (in a population of cell) of greater than or equal to 5 or more copies of the c-met gene, or an average of eight or more copies of a c-met gene, or more.

A cancer or biological sample which "does not display c-met amplification" is one which, in a diagnostic test, does not have amplified c-met gene. In some embodiments, a sample which does not display c-met amplification is a sample which has an average of fewer than 4 copies of c-met gene.

By "EGFR" is meant the receptor tyrosine kinase polypeptide Epidermal Growth Factor Receptor which is described in Ullrich et al, Nature (1984) 309:418425, alternatively referred to as Her-1 and the c-erbB gene product, as well as variants thereof such as EGFRvIII. Variants of EGFR also include deletional, substitutional and insertional variants, for example those described in Lynch et al (New England Journal of Medicine 2004, 350:2129), Paez et al (Science 2004, 304:1497), Pao et al (PNAS 2004, 101:13306).

An "EGFR antagonist" (interchangeably termed "EGFR inhibitor") is an agent that interferes with EGFR activation or function. Examples of EGFR inhibitors include EGFR antibodies; EGFR ligand antibodies; small molecule EGFR antagonists; EGFR tyrosine kinase inhibitors; antisense and inhibitory RNA (e.g., shRNA) molecules (see, for example, WO2004/87207). Preferably, the EGFR inhibitor is an antibody or small molecule which binds to EGFR. In some embodiments, the EGFR inhibitor is an EGFR-targeted drug. In a particular embodiment, an EGFR inhibitor has a binding affinity (dissociation constant) to EGFR of about 1,000 nM or less. In another embodiment, an EGFR inhibitor has a binding affinity to EGFR of about 100 nM or less. In another embodiment, an EGFR inhibitor has a binding affinity to EGFR of about 50 nM or less. In a particular embodiment, an EGFR inhibitor is covalently bound to EGFR. In a particular embodiment, an EGFR inhibitor inhibits EGFR signaling with an IC50 of 1,000 nM or less. In another embodiment, an EGFR inhibitor inhibits EGFR signaling with an IC50 of 500 nM or less. In another embodiment, an EGFR inhibitor inhibits EGFR signaling with an IC50 of 50 nM or less. In certain embodiments, the EGFR antagonist reduces or inhibits, by at least 10%, 20%, 30%, 40%, 50%, 60%, 70%, 80%, 90% or more, the expression level or biological activity of EGFR.

"EGFR activation" refers to activation, or phosphorylation, of EGFR. Generally, EGFR activation results in signal transduction (e.g. that caused by an intracellular kinase domain of EGFR receptor phosphorylating tyrosine residues in EGFR or a substrate polypeptide). EGFR activation may be mediated by EGFR ligand binding to a EGFR dimer comprising EGFR. EGFR ligand binding to a EGFR dimer may activate a kinase domain of one or more of the EGFR in the dimer and thereby results in phosphorylation of tyrosine residues in one or more of the EGFR and/or phosphorylation of tyrosine residues in additional substrate polyp eptides(s).

As used herein, the term "EGFR-targeted drug" refers to a therapeutic agent that binds to EGFR and inhibits EGFR activation. Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF (see WO98/50433, Abgenix); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding; and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). Examples of small molecules that bind to EGFR include ZD1839 or Gefitinib (IRESSA; Astra Zeneca); CP-358774 or Erlotinib (TARCEVA™; Genentech/OSI); and AG1478, AG1571 (SU 5271; Sugen); EMD-7200.

The technique of "polymerase chain reaction" or "PCR" as used herein generally refers to a procedure wherein minute amounts of a specific piece of nucleic acid, RNA and/or DNA, are amplified as described in U.S. Pat. No. 4,683,195 issued 28 July 1987. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage or plasmid sequences, etc. See generally Mullis et al., Cold Spring Harbor Symp. Quant. Biol., 51: 263 (1987); Erlich, ed., PCR Technology, (Stockton Press, NY, 1989). As used herein, PCR is considered to be one, but not the only, example of a nucleic acid polymerase reaction method for amplifying a nucleic acid test sample, comprising the use of a known nucleic acid (DNA or RNA) as a primer and utilizes a nucleic acid polymerase to amplify or generate a specific piece of nucleic acid or to amplify or generate a specific piece of nucleic acid which is complementary to a particular nucleic acid.

"Quantitative real time polymerase chain reaction" or "qRT-PCR" refers to a form of PCR wherein the amount of PCR product is measured at each step in a PCR reaction. This technique has been described in various publications including Cronin et al., Am. J. Pathol. 164(1):35-42 (2004); and Ma et al., Cancer Cell 5:607-616 (2004).

The term "microarray" refers to an ordered arrangement of hybridizable array elements, preferably polynucleotide probes, on a substrate.

The term "polynucleotide," when used in singular or plural, generally refers to any polyribonucleotide or polydeoxyribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple- stranded regions comprising RNA or DNA or both RNA and DNA. The strands in such regions may be from the same molecule or from different molecules. The regions may include all of one or more of the molecules, but more typically involve only a region of some of the molecules. One of the molecules of a triplehelical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double- stranded DNAs. Oligonucleotides, such as single- stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

A "chemotherapeutic agent" is a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics *(e. g.,* calicheamicin, especially calicheamicin gamma1I and calicheamicin omega1 (see, *e.g.,* Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; antiadrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; dacarbazine; mannomustine; mitobronitol; mitolactol; pipobroman; gacytosine; arabinoside *("Ara-C");* thiotepa; taxoid, *e.g.,* paclitaxel (TAXOL®), albuminengineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin, and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovovin; novantrone; edatrexate; daunomycin; aminopterin; ibandronate; topoisomerase inhibitor RFS 2000; difluorometlhylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor *(e.g.,* LURTOTECAN®); rmRH *(e.g.,* ABARELIX®); BAY439006 (sorafenib; Bayer); SU-11248 (Pfizer); perifosine, COX-2 inhibitor *(e.g.* celecoxib or etoricoxib), proteosome inhibitor *(e.g.* PS341); bortezomib (VELCADE®);

CCI-779; tipifarnib (R11577); orafenib, ABT510; Bcl-2 inhibitor such as oblimersen sodium (GENASENSE®); pixantrone; EGFR inhibitors (see definition below); tyrosine kinase inhibitors (see definition below); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

Herein, chemotherapeutic agents include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

Specific examples of chemotherapeutic agents or chemotherapy regimens herein include: alkylating agents *(e.g.* chlorambucil, bendamustine, or cyclophosphamide); nucleoside analogues or antimetabolites *(e.g.* fludarabine), fludarabine and cyclophosphamide (FC); prednisone or prednisolone; akylator-containing combination therapy, including cyclophosphamide, vincristine, prednisolone (CHOP), or cyclophosphamide, vincristine, prednisolone (CVP), etc.

A "target audience" is a group of people or an institution to whom or to which a particular medicament is being promoted or intended to be promoted, as by marketing or advertising, especially for particular uses, treatments, or indications, such as individual patients, patient populations, readers of newspapers, medical literature, and magazines, television or internet viewers, radio or internet listeners, physicians, drug companies, *etc.*

A "package insert" is used to refer to instructions customarily included in commercial packages of therapeutic products, that contain information about the indications, usage, dosage, administration, contraindications, other therapeutic products to be combined with the packaged product, and/or warnings concerning the use of such therapeutic products, *etc.*

The term "antibody" herein is used in the broadest sense and encompasses various antibody structures, including but not limited to monoclonal antibodies, polyclonal antibodies, multispecific antibodies *(e.g.,* bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies; linear antibodies; single-chain antibody molecules *(e.g.* scFv); and multispecific antibodies formed from antibody fragments.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g.,* $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "cytotoxic agent" as used herein refers to a substance that inhibits or prevents a cellular function and/or causes cell death or destruction. Cytotoxic agents include, but are not limited to, radioactive isotopes *(e.g.,* $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents or drugs *(e.g.,* methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987)). Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991)) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008)). Unless otherwise indicated, HVR residues and other residues in the variable domain (e.g., FR residues) are numbered herein according to Kabat et al., supra. In one embodiment, the c-met antibody herein comprises the HVRs of SEQ ID NOs: 1-6.

An "immunoconjugate" is an antibody conjugated to one or more heterologous molecule(s), including but not limited to a cytotoxic agent.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (*e.g.,* a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "pharmaceutical formulation" refers to a sterile preparation that is in such form as to permit the biological activity of the medicament to be effective, and which contains no additional components that are unacceptably toxic to a subject to which the formulation would be administered.

A "sterile" formulation is aseptic or free from all living microorganisms and their spores.

A "kit" is any manufacture (*e.g* a package or container) comprising at least one reagent, *e.g.,* a medicament for treatment of cancer (*e.g.,* NCSLC or triple-negative breast cancer), or a reagent (*e.g.,* antibody) for specifically detecting a biomarker gene or protein of the invention. The manufacture is preferably promoted, distributed, or sold as a unit for performing the methods of the present invention.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

## II. CANCER MEDICAMENTS

In one aspect, the invention concerns selecting patients who can be treated with cancer medicaments based on expression of one or more of the biomarkers disclosed herein. Examples of cancer medicaments include, but are not limited to:

- c-met antagonists, including anti-c-met antibodies.
- Chemotherapeutic agents and chemotherapy regimens.
- Other medicaments or combinations thereof in development, or approved, for treating cancer, *e.g.,* NSCLC.

In one embodiment, the medicament is an antibody, *e.g.* directed against or which binds to c-met. The antibody herein includes: monoclonal antibodies, including a chimeric, humanized or human antibodies. In one embodiment, the antibody is an antibody fragment, *e.g.,* a Fv, Fab, Fab', one-armed antibody, scFv, diabody, or F(ab')$_2$ fragment. In another embodiment, the antibody is a full length antibody, *e.g.,* an intact IgG1 antibody or other antibody class or isotype as defined herein. In one embodiment, the antibody is monovalent. In another embodiment, the antibody is a one-armed antibody *(i.e.,* the heavy chain variable domain and the light chain variable domain form a single antigen binding arm) comprising an Fc region, wherein the Fc region comprises a first and a second Fc polypeptide, wherein the first and second Fc polypeptides are present in a complex and form a Fc region that increases stability of said antibody fragment compared to a Fab molecule comprising said antigen binding arm. The one-armed antibody may be monovalent.

In one embodiment, the c-met antagonist is an anti-c-met antibody. In another embodiment, the anti-c-met antibody is MetMAb (onartuzumab) or a biosimilar version thereof. MetMAb is disclosed in, for example, WO2006/015371; Jin et al, Cancer Res (2008) 68:4360. In another embodiment, the anti-c-met antibody comprises a heavy chain variable domain comprising one or more of (a) HVR1 comprising sequence GYTFTSYWLH (SEQ ID NO:1); (b) HVR2 comprising sequence GMIDPSNSDTRFNPNFKD (SEQ ID NO: 2); and/or (c) HVR3-HC comprising sequence ATYRSYVTPLDY (SEQ ID NO: 3). In some embodiments, the antibody comprises a light chain variable domain comprising one or more of (a) HVR1-LC comprising sequence KSSQSLLYTSSQKNYLA (SEQ ID NO: 4); HVR2-LC comprising sequence WASTRES (SEQ ID NO: 5); and/or (c) HVR3-LC comprising sequence QQYYAYPWT (SEQ ID NO: 6). In some embodiments the anti-c-met antibody comprises a heavy chain variable domain comprising (a) HVR1 comprising sequence GYTFTSYWLH (SEQ ID NO: 1); (b) HVR2 comprising sequence GMIDPSNSDTRFNPNFKD (SEQ ID NO: 2); and (c) HVR3-HC comprising sequence ATYRSYVTPLDY (SEQ ID NO: 3) and a light chain variable domain comprising (a) HVR1-LC comprising sequence KSSQSLLYTSSQKNYLA (SEQ ID NO: 4); HVR2-LC comprising sequence WASTRES (SEQ ID NO: 5); and (c) HVR3-LC comprising sequence QQYYAYPWT (SEQ ID NO: 6).

In any of the above embodiments, for example, an anti-c-met antibody can be humanized. In one embodiment, an anti- c-met antibody comprises HVRs as in any of the above embodiments, and further comprises an acceptor human framework, e.g. a human immunoglobulin framework or a human consensus framework.

In another aspect, an anti- c-met antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:7. In certain embodiments, a VH sequence having at least 90%, 91%, 92%, 93%, 94%, 95%, 96%, 97%, 98%, or 99% identity contains substitutions *(e.g.,* conservative substitutions), insertions, or deletions relative to the reference sequence, but an anti- c-met antibody comprising that sequence retains the ability to bind to human c-met. In certain embodiments, a total of 1 to 10 amino acids have been substituted, altered inserted and/or deleted in SEQ ID NO:7. In certain embodiments, substitutions, insertions, or deletions occur in regions outside the HVRs *(i.e.,* in the FRs). Optionally, the anti-c-met antibody comprises the VH sequence in SEQ ID NO:7, including post-translational modifications of that sequence.

In another aspect, an anti- c-met antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:8. In certain embodiments, a VL sequence having at least 90%, 91%, 92%, 93%, 94%, 95%, 96%, 97%, 98%, or 99% identity contains substitutions *(e.g.,* conservative substitutions), insertions, or deletions relative to the reference sequence, but an anti- c-met antibody comprising that sequence retains the ability to bind to c-met. In certain embodiments, a total of 1 to 10 amino acids have been substituted, inserted and/or deleted in SEQ ID NO:8. In certain embodiments, the substitutions, insertions, or deletions occur in regions outside the HVRs *(i.e.,* in the FRs). Optionally, the anti- c-met antibody comprises the VL sequence in SEQ ID NO: 8, including post-translational modifications of that sequence.

In yet another embodiment, the anti- c-met antibody comprises a VL region having at least 90%, 91%, 92%, 93%, 94%, 95%, 96%, 97%, 98%, 99%, or 100% sequence identity to the amino acid sequence of SEQ ID NO:8 and a VH region having at least 90%, 91%, 92%, 93%, 94%, 95%, 96%, 97%, 98%, 99%, or 100% sequence identity to the amino acid sequence of SEQ ID NO:7. In yet a further embodiment, the anti- c-met antibody comprises a HVR-L1 comprising amino acid sequence SEQ ID NO: 1; an HVR-L2 comprising amino acid sequence SEQ ID NO: 2; an HVR-L3 comprising amino acid sequence SEQ ID NO: 3; an HVR-H1 comprising amino acid sequence SEQ ID NO: 4; an HVR-H2 comprising amino acid sequence SEQ ID NO: 5; and an HVR-H3 comprising amino acid sequence SEQ ID NO: 6.

In another aspect, an anti- c-met antibody is provided, wherein the antibody comprises a VH as in any of the embodiments provided above, and a VL as in any of the embodiments provided above.

In a further aspect, the invention provides an antibody that binds to the same epitope as an anti- c-met antibody provided herein. For example, in certain embodiments, an antibody is provided that binds to the same epitope as or can by competitively inhibited by an anti- c-met antibody comprising a VH sequence of SEQ ID NO:7 and a VL sequence of SEQ ID NO:8.

In a further aspect of the invention, an anti- c-met antibody according to any of the above embodiment can be a monoclonal antibody, including a monovalent, chimeric, humanized or human antibody. In one embodiment, an anti-c-met antibody is an antibody fragment, e.g., a one-armed, Fv, Fab, Fab', scFv, diabody, or F(ab')$_2$ fragment. In another embodiment, the antibody is a full length antibody, e.g., an intact IgG1 or IgG4 antibody or other antibody class or isotype as defined herein. According to another embodiment, the antibody is a bispecific antibody. In one embodiment, the bispecific antibody comprises the HVRs or comprises the VH and VL regions described above.

In some embodiments, the anti-c-met antibody is monovalent, and comprises (a) a first polypeptide comprising a heavy chain variable domain having the sequence: EVQLVESGGGLVQPGGSLRLSCAASGYTFTSYWLHWVRQAPGKGLEWVGMIDPSNS DTRFNPNFKDRFTISADTSKNTAYLQMNSLRAEDTAVYYCATYRSYVTPLDYWGQGT LVTVSS (SEQ ID NO:7), CH1 sequence, and a first Fc polypeptide; (b) a second polypeptide comprising a light chain variable domain having the sequence:DIQMTQSPSSLSASVGDRVTITCKSSQSLLYTSSQKNYLAWYQQKPGKAPKLLIYWAST R ESGVPSRFSGSGSGTDFTLTISSLQPEDFATYYCQQYYAYPWTFGQGTKVEIKR (SEQ ID NO: 8), and CL1 sequence; and (c) a third polypeptide comprising a second Fc polypeptide, wherein the heavy chain variable domain and the light chain variable domain are present as a complex and form a single antigen binding arm, wherein the first and second Fc polypeptides are present in a complex and form a Fc region that increases stability of said antibody fragment compared to a Fab molecule comprising said antigen binding arm. In some embodiments, the first polypeptide comprises Fc sequence

CPPCPAPELLGGPSVFLFPPKPKDTLMISRTPEVTCVVVDVSHEDPEVKFNWYVDGVE

VHNAKTKPREEQYNSTYRVVSVLTVLHQDWLNGKEYKCKVSNKALPAPIEKTISKAK

GQPREPQVYTLPPSREEMTKNQVSLSCAVKGFYPSDIAVEWESNGQPENNYKTTPPVL

DSDGSFFLVSKLTVDKSRWQQGNVFSCSVMHEALHNHYTQKSLSLSPGK (SEQ ID NO: 9)

and the second polypeptide comprises the Fc sequence

CPPCPAPELLGGPSVFLFPPKPKDTLMISRTPEVTCVVVDVSHEDPEVKFNWYVDGVE

VHNAKTKPREEQYNSTYRVVSVLTVLHQDWLNGKEYKCKVSNKALPAPIEKTISKAK

GQPREPQVYTLPPSREEMTKNQVSLWCLVKGFYPSDIAVEWESNGQPENNYKTTPPVL

DSDGSFFLYSKLTVDKSRWQQGNVFSCSVMHEALHNHYTQKSLSLSPGK (SEQ ID NO: 10).

In another embodiments, the anti-c-met antibody is monovalent and comprises (a) a first polypeptide comprising a heavy chain, said polypeptide comprising the sequence:

EVQLVESGGGLVQPGGSLRLSCAASGYTFTSYWLHWVRQAPGKGLEWVGMIDPSNS DTRFNPNFKDRFTISADTSKNTAYLQMNSLRAEDTAVYYCATYRSYVTPLDYWGQGT LVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPRE PQVYTLPPSREEMTKNQVSLSCAVKGFYPSDIAVEWESNGQPENNYKTTPPVLDSDGS FFLVSKLTVDKSRWQQGNVFSCSVMHEALHNHYTQKSLSLSPGK (SEQ ID NO: 11); (b)

(b) a second polypeptide comprising a light chain, the polypeptide comprising the sequence

DIQMTQSPSSLSASVGDRVTITCKSSQSLLYTSSQKNYLAWYQQKPGKAPKLLIYWAST RESGVPSRFSGSGSGTDFTLTISSLQPEDFATYYCQQYYAYPWTFGQGTKVEIKRTVAA PSVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC (SEQ ID NO: 12); and a third polypeptide comprising a Fc sequence, the polypeptide comprising the sequence

DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTI

SKAKGQPREPQVYTLPPSREEMTKNQVSLWCLVKGFYPSDIAVEWESNGQPENNYKT TPPVLDSDGSFFLYSKLTVDKSRWQQGNVFSCSVMHEALHNHYTQKSLSLSPGK (SEQ ID NO: 13),

wherein the heavy chain variable domain and the light chain variable domain are present as a complex and form a single antigen binding arm, wherein the first and second Fc polypeptides are present in a complex and form a Fc region that increases stability of said antibody fragment compared to a Fab molecule comprising said antigen binding arm.

Other anti-c-met antibodies suitable for use in the methods of the invention are described herein and known in the art. For example, anti-c-met antibodies disclosed in WO05/016382 (including but not limited to antibodies 13.3.2, 9.1.2, 8.70.2, 8.90.3); an anti-c-met antibodies produced by the hybridoma cell line deposited with ICLC number PD 03001 at the CBA in Genoa, or that recognizes an epitope on the extracellular domain of the β chain of the HGF receptor, and said epitope is the same as that recognized by the monoclonal antibody); anti-c-met antibodies disclosed in WO2007/126799 (including but not limited to 04536, 05087, 05088, 05091, 05092, 04687, 05097, 05098, 05100, 05101, 04541, 05093, 05094, 04537, 05102, 05105, 04696, 04682); anti c-met antibodies disclosed in WO2009/007427 (including but not limited to an antibody deposited at CNCM, Institut Pasteur, Paris, France, on March 14, 2007 under the number I-3731, on March 14, 2007 under the number I-3732, on July 6, 2007 under the number I-3786, on March 14, 2007 under the number I-3724; an anti-c-met antibody disclosed in 20110129481; an anti-c-met antibody disclosed in US20110104176; an anti-c-met antibody disclosed in WO2009/134776; an anti-c-met antibody disclosed in WO2010/059654; an anti-c-met antibody disclosed in WO2011020925 (including but not limited to an antibody secreted from a hybridoma deposited at the CNCM, Institut Pasteur, Paris, France, on march 12, 2008 under the number I-3949 and the hybridoma deposited on January 14, 2010 under the number I-4273).

In one aspect, the anti-c-met antibody comprises at least one characteristic that promotes heterodimerization, while minimizing homodimerization, of the Fc sequences within the antibody fragment. Such characteristic(s) improves yield and/or purity and/or homogeneity of the immunoglobulin populations. In one embodiment, the antibody comprises Fc mutations constituting "knobs" and "holes" as described in WO2005/063816. For example, a hole mutation can be one or more of T366A, L368A and/or Y407V in an Fc polypeptide, and a cavity mutation can be T366W.

In some embodiments, the c-met antagonist is an anti-hepatocyte growth factor (HGF) antibody, for example, humanized anti-HGF antibody TAK701, rilotumumab, Ficlatuzumab, and/or humanized antibody 2B8 described in WO2007/143090. In some embodiments, the anti-HGF antibody is the anti-HGF antibody described in US7718174B2.

In some embodiments, the c-met antagonist is a c-met small molecule inhibitor. In some embodiments, the c-met small molecule inhibitor is a selective c-met small molecule inhibitor.

In one embodiment, the c-met antagonist binds c-met extracellular domain. In some embodiments, the c-met antagonist binds c-met kinase domain. In some embodiments, the c-met antagonist competes for c-met binding with hepatocyte growth factor (HGF). In some embodiments, the c-met antagonist binds HGF. In certain embodiments, the c-met antagonist inhibits cell proliferation, *e.g.,* HGF-induced cell proliferation of cell line EBC-1, H441 and/or KP4. In some embodiments, cell proliferation is inhibited with a Ki of 600 nM or less (more potent), 500 nM or less, 400 nM or less, 300 nM or less or more potent. In certain embodiments, the c-met antagonist inhibits c-met signaling *(e.g.,* phospho-c-met, phospho-AKT, phospho-MAPK) when EBC-1 cells are treated with c-met antagonist in the presence of 10% fetal bovine serum. In some embodiments, c-met signaling is inhibited with a Ki of 600 nM or less (more potent), 500 nM or less, 400 nM or less, 300 nM or less (more potent). In certain embodiments, the c-met antagonist treats (is capable of treating) squamous cell carcinoma. In certain embodiments, the c-met antagonist treats (is capable of treating) NSCLC that expresses wild-type k-ras.

In certain embodiments, the c-met inhibitor is not a non-ATP-competitive small molecule. In certain embodiments, the c-met antagonist inhibits cell proliferation, e.g., HGF-induced cell proliferation of cell line EBC-1, H441 and/or KP4. In certain embodiments, the c-met antagonist inhibits c-met signaling (e.g., phospho-c-met, and downstream c-met signaling pathways, *e.g.,* phospho-AKT, phospho-MAPK) when EBC-1 cells are treated with c-met antagonist in the presence of 10% fetal bovine serum. In certain embodiments, the c-met antagonist does not inhibit cell proliferation of cell lines MDA-MB-231 and HT29 (in the presence or absence of exogenous HGF). In certain embodiments, the c-met antagonist does not inhibit cell proliferation of cell lines MDA-MB-231 and HT29 in the presence of 10% fetal bovine serum. Methods for assaying cell proliferation are well-known in the art, and some methods are described in WO2009/111691; WO2006/015371; and Jin et al, Cancer Res (2008) 68:4360. In certain embodiments, the c-met antagonist treats (is capable of treating) squamous cell carcinoma. In certain embodiments, the c-met antagonist treats (is capable of treating) NSCLC that expresses wild-type k-ras. In certain embodiments, the c-met antagonist is not Tivantinib (ARQ-197). In a particular embodiment, a c-met antagonist inhibits c-met signaling with an IC50 of 1,000 nM or less *(i.e.,* more potent). In another embodiment, a c-met antagonist inhibits c-met signaling with an IC50 of 400 nm or less, 500 nM or less, 600 nm or less, 700 nm or less. In another embodiment, a c-met antagonist inhibits c-met signaling with an IC50 of 50 nM or less. In certain embodiments, the c-met antagonist is not crizotinib. In certain embodiments, the c-met antagonist is not foretinib. In certain embodiments, the c-met antagonist is not ficlatuzumab.

In certain embodiments, the c-met antagonist is any one of: SGX-523, Crizotinib (PF-02341066; 3-[(1R)-1-(2,6-dichloro-3-fluorophenyl)ethoxy]-5-(1-piperidin-4-ylpyrazol-4-yl)pyridin-2-amine; CAS no. 877399-52-5); JNJ-38877605 (CAS no. 943540-75-8), BMS-698769, PHA-665752 (Pfizer), SU5416, INC-280 (Incyte; SU11274 (Sugen; [(3Z)-N-(3-chlorophenyl)-3-({3,5-dimethyl-4-[(4-methylpiperazin-1-yl)carbonyl]-1H-pyrrol-2-yl}methylene)-N-methyl-2-oxoindoline-5-sulfonamide; CAS no. 658084-23-2]), Foretinib (GSK1363089), XL880 (CAS no. 849217-64-7; XL880 is a inhibitor of met and VEGFR2 and KDR); MGCD-265 (MethylGene; MGCD-265 targets the c-MET, VEGFR1, VEGFR2, VEGFR3, Ron and Tie-2 receptors; CAS no. 875337-44-3), Tivantinib (ARQ 197; (-)-(3R,4R)-3-(5,6-dihydro-4H-pyrrolo[3,2,1-ij]quinolin-1-yl)-4-(1H-indol-3-yl)pyrrolidine-2,5-dione; see Munchi et al, Mol Cancer Ther June 2010 9; 1544; CAS no. 905854-02-6), LY-2801653 (Lilly), LY2875358 (Lilly), MP-470, Rilotumumab (AMG 102, anti-HGF monoclonal antibody), antibody 223C4 or humanized antibody 223C4 (WO2009/007427), humanized L2G7 (humanized TAK701; humanized anti-HGF monoclonal antibody); EMD 1214063 (Merck Sorono), EMD 1204831 (Merck Serono), NK4, Cabozantinib (XL-184, CAS no. 849217-68-1; carbozantinib is a dual inhibitor of met and VEGFR2), MP-470 (SuperGen; is a novel inhibitor of c-KIT, MET, PDGFR, Flt3, and AXL), Comp-1, Ficlatuzumab (AV-299; anti-HGF monoclonal antibody), E7050 (Cas no. 1196681-49-8; E7050 is a dual c-met and VEGFR2 inhibitor (Esai); MK-2461 (Merck; N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide; CAS no. 917879-39-1); MK8066 (Merck), PF4217903 (Pfizer), AMG208 (Amgen), SGX-126, RP1040, LY2801653, AMG458, EMD637830, BAY-853474, DP-3590. In certain embodiments, the c-met antagonist is any one or more of crizotinib, tivantinib, carbozantinib, MGCD-265, ficlatuzumab, humanized TAK-701, rilotumumab, foretinib, h224G11, DN-30, MK-2461, E7050, MK-8033, PF-4217903, AMG208, JNJ-38877605, EMD1204831, INC-280, LY-2801653, SGX-126, RP1040, LY2801653, BAY-853474, and/or LA480. In certain embodiments, the c-met antagonist is any one or more of crizotinib, tivantinib, carbozantinib, MGCD-265, ficlatuzumab, humanized TAK-701, rilotumumab, and/or foretinib.

EGFR antagonists include antibodies such as humanized monoclonal antibody known as nimotuzumab (YM Biosciences), fully human ABX-EGF (panitumumab, Abgenix Inc.) as well as fully human antibodies known as E1.1, E2.4, E2.5, E6.2, E6.4, E2.11, E6. 3 and E7.6. 3 and described in US 6,235,883; MDX-447 (Medarex Inc). Pertuzumab (2C4) is a humanized antibody that binds directly to HER2 but interferes with HER2-EGFR dimerization thereby inhibiting EGFR signaling. Other examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF (see WO98/50433, Abgenix); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding; and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, *e.g.*, EP659,439A2, Merck Patent GmbH).

Anti-EGFR antibodies that are useful in the methods of the invention include any antibody that binds with sufficient affinity and specificity to EGFR and can reduce or inhibit EGFR activity. The antibody selected will normally have a sufficiently strong binding affinity for EGFR, for example, the antibody may bind human c-met with a Kd value of between 100 nM-1 pM. Antibody affinities may be determined by a surface plasmon resonance based assay (such as the BIAcore assay as described in PCT Application Publication No. WO2005/012359); enzyme-linked immunoabsorbent assay (ELISA); and competition assays (e.g. RIA's), for example. Preferably, the anti-EGFR antibody of the invention can be used as a therapeutic agent in targeting and interfering with diseases or conditions wherein EGFR/EGFR ligand activity is involved. Also, the antibody may be subjected to other biological activity assays, e.g., in order to evaluate its effectiveness as a therapeutic. Such assays are known in the art and depend on the target antigen and intended use for the antibody.

Bispecific antibodies are antibodies that have binding specificities for at least two different epitopes. Exemplary bispecific antibodies may bind to EGFR and to c-met. In another example, an exemplary bispecific antibody may bind to two different epitopes of the same protein, e.g., c-met protein. Alternatively, a c-met or EGFR arm may be combined with an arm which binds to a triggering molecule on a leukocyte such as a T-cell receptor molecule (e.g. CD2 or CD3), or Fc receptors for IgG (FcγR), such as FcγRI (CD64), FcγRII (CD32) and FcγRIII (CD16) so as to focus cellular defense mechanisms to the c-met or EGFR-expressing cell. Bispecific antibodies may also be used to localize cytotoxic agents to cells which express EGFR or c-met. These antibodies possess a EGFR or c-met-binding arm and an arm which binds the cytotoxic agent (e.g. saporin, anti-interferon-α, vinca alkaloid, ricin A chain, methotrexate or radioactive isotope hapten). Bispecific antibodies can be prepared as full length antibodies or antibody fragments (e.g. F(ab')2 bispecific antibodies). In one embodiment, the bispecific antibody is any of the bispecific MET-EGFR antibodies disclosed in US20100254989A.

EGFR antagonists also include small molecules such as compounds described in US5616582, US5457105, US5475001, US5654307, US5679683, US6084095, US6265410, US6455534, US6521620, US6596726, US6713484, US5770599, US6140332, US5866572, US6399602, US6344459, US6602863, US6391874, WO9814451, WO9850038, WO9909016, WO9924037, WO9935146, WO0132651 US6344455, US5760041, US6002008, US5747498. Particular small molecule EGFR antagonists include OSI-774 (CP-358774, erlotinib, OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); Iressa® (ZD1839, gefitinib, AstraZeneca); ZM 105180 ((6-amino-4-(3-methylphenylamino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo [2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide); lapatinib (Tykerb, GlaxoSmithKline); ZD6474 (Zactima, AstraZeneca); CUDC-101 (Curis); canertinib (CI-1033); AEE788 (6-[4-[(4-ethyl-1-piperazinyl)methyl]phenyl]-N-[(1R)-1-phenylethyl]-7H-pyrrolo[2,3-d]pyrimidin-4-amine, WO2003013541, Novartis) and PKI166 4-[4-[[(1R)-1-phenylethyl]amino]-7H-pyrrolo[2,3-d]pyrimidin-6-yl]-phenol, WO9702266 Novartis).

In one embodiment, the antibody, e.g. the antibody used in the methods herein may incorporate any of the features, singly or in combination, as described in Sections 1-6 below:

### 1. Antibody Fragments

In certain embodiments, an antibody provided herein is an antibody fragment. Antibody fragments include, but are not limited to, Fab, Fab', Fab'-SH, F(ab')₂, Fv, and scFv fragments, a one-armed antibody, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')$_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

One-armed antibodies (i.e., the heavy chain variable domain and the light chain variable domain form a single antigen binding arm) are disclosed in, for example, WO2005/063816; Martens et al, Clin Cancer Res (2006), 12: 6144. For treatment of pathological conditions requiring an antagonistic function, and where bivalency of an antibody results in an undesirable agonistic effect, the monovalent trait of a one-armed antibody (i.e., an antibody comprising a single antigen binding arm) results in and/or ensures an antagonistic function upon binding of the antibody to a target molecule. Furthermore, the one-armed antibody comprising a Fc region is characterized by superior pharmacokinetic attributes (such as an enhanced half life and/or reduced clearance rate *in vivo*) compared to Fab forms having similar/substantially identical antigen binding characteristics, thus overcoming a major drawback in the use of conventional monovalent Fab antibodies. Techniques for making one-armed antibodies include, but are not limited to, "knob-in-hole" engineering (see, e.g., U.S. Patent No. 5,731,168). MetMAb is an example of a one-armed antibody. [add other one armed monovalent formats? Genmab?]

Antibody fragments can be made by various techniques, including but not limited to proteolytic digestion of an intact antibody as well as production by recombinant host cells (e.g. *E. coli* or phage), as described herein.

### *2. Chimeric and Humanized Antibodies*

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Frost. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 3. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 4. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

*5. Multispecific Antibodies*

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for c-met and the other is for any other antigen. In certain embodiments, bispecific antibodies may bind to two different epitopes of c-met. Bispecific antibodies may also be used to localize cytotoxic agents to cells which express c-met. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); crosslinking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to c-met as well as another, different antigen, such as EGFR (see, US 2008/0069820, for example).

*6. Antibody Variants*

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

In certain embodiments, antibody variants having one or more amino acid substitutions are provided. Sites of interest for substitutional mutagenesis include the HVRs and FRs. Amino acid substitutions may be introduced into an antibody of interest and the products screened for a desired activity, e.g., retained/improved antigen binding, decreased immunogenicity, or improved ADCC or CDC.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

In certain embodiments, one or more amino acid modifications may be introduced into the Fc region of an antibody provided herein, thereby generating an Fc region variant. The Fc region variant may comprise a human Fc region sequence (*e.g.*, a human IgG1, IgG2, IgG3 or IgG4 Fc region) comprising an amino acid modification (*e.g.* a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious.

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.*, either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

In one embodiment, the medicament is an mmunoconjugate comprising an antibody (such as a c-met antibody) conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the radioconjugate is used for detection, it may comprise a radioactive atom for scintigraphic studies, for example tc99m or I123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

## III. DIAGNOSTIC METHODS

In one aspect, the invention provides methods for identifying a cancer patient who is likely to respond to treatment with a c-met antagonist comprising the step of determining whether the patient's cancer has a high amount of c-met biomarker, wherein the c-met biomarker expression indicates that the patient is likely to respond to treatment with the c-met antagonist.

In another aspect, the invention provides methods for determining cancer patient prognosis, comprising the step of determining whether the patient's cancer has a high amount of c-met biomarker, wherein the c-met biomarker expression indicates that the patient is likely to have increased overall survival (OS) and/or progression-free survival (PFS) when the patient is treated with a c-met antagonist.

In another aspect, the invention provides methods for determining c-met biomarker expression, comprising the step of determining whether a patient's cancer has a high amount of c-met biomarker, wherein c-met biomarker expression is protein expression and is determined in a sample from the patient using IHC, wherein high c-met biomarker expression is 50% or more of the tumor cells with moderate c-met staining intensity, combined moderate/high c-met staining intensity or high c-met staining intensity, wherein c-met expression is detected using a c-met antibody, wherein the c-met biomarker expression indicates that the patient is likely to have increased OS and/or PFS when the patient is treated with a c-met antagonist.

In one aspect, the invention herein concerns a method for selecting a therapy for a patient with cancer (e.g., NSCLC) comprising determining expression of a c-met biomarker in a sample from the patient, and selecting a cancer medicament based on the level of expression of the biomarker. In one embodiment, a high level of the biomarker(s) will result in selection of a c-met antagonist, such as a c-met antibody for use in treating the patient. In another embodiment, where the biomarker(s) are present a low (i.e. low or substantially undetectable level), the patient will be selected for a treatment with a cancer medicament other than a c-met antagonist. In some embodiments, the sample is of the patient's cancer (e.g., NSCLC).

In one aspect, the invention provides a method of optimizing therapeutic efficacy comprising determining c-met biomarker expression in a patient cancer sample. In some embodiments, detection of high c-met biomarker means increased PFS and/or OS when the patient is treated with a c-met antagonist. In some embodiments, detection of low c-met biomarker means a decreased PFS and/or OS when the patient is treated with the c-met antagonist. In some embodiments, the cancer is NSCLC.

In one aspect, the invention provides a method of determining patient prognosis, comprising determining level of c-met biomarker in a patient cancer sample. In some embodiments, high c-met biomarker means likelihood of decreased PFS and/or OS compared to a patient whose cancer has low c-met biomarker. In some embodiments, high c-met biomarker expression means increased PFS and/or OS when the patient is treated with c-met antagonist. In some embodiments, low c-met biomarker expression means decreased PFS and/or OS when the patient is treated with c-met antagonist. In some embodiments, the cancer is NSCLC, and high c-met biomarker expression means increased PFS and/or OS when the patient is treated with c-met antagonist in combination with EGFR antagonist. In some embodiments, the cancer is NSCLC, and low c-met biomarker expression means decreased PFS and/or OS when the patient is treated with c-met antagonist in combination with EGFR antagonist.

In one aspect, amount of c-met biomarker is determined using a method comprising: (a) performing IHC analysis of a sample (such as a patient cancer sample) with anti-c-met antibody; and b) determining expression of a c-met biomarker in the sample. In some embodiments, c-met IHC staining intensity is determined relative to a reference value. In some embodiments, c-met biomarker expression is determined using a c-met staining intensity scoring scheme is disclosed herein, e.g., in Table A in Section III.7 below. In some embodiments, the method further comprises stratifying the patients based on IHC score.

In one aspect, amount of c-met biomarker expression is determined using a method comprising the step of determining expression of c-met biomarker in the sample (such as a patient's cancer sample), wherein the patient's sample has been subjected to IHC analysis using an anti-c-met antibody. In some embodiments, c-met IHC staining intensity is determined relative to a reference value. In some embodiments, c-met biomarker expression is determined using a c-met staining intensity scoring scheme is disclosed herein, e.g., in Table A in Section III.7 below.

In some embodiments, IHC analysis further comprises morphological staining, either prior to or thereafter. In one embodiment, hematoxylin is use for staining cellular nucleic of the slides. Hematoxylin is widely available. An example of a suitable hematoxylin is Hematoxylin II (Ventana). When lighter blue nuclei are desired, a bluing reagent may be used following hematoxylin staining.

Detection of c-met biomarker using IHC is disclosed herein, and a c-met staining intensity scoring scheme is disclosed herein, e.g., in Table A in Section III.7 below. As is noted herein, other biomarkers may be detected. Exemplary other biomarkers are disclosed herein. In some embodiments of any of the inventions disclosed herein, high c-met biomarker expression is met diagnostic positive clinical status as defined in accordance with Table A herein. In some embodiments of any of the inventions disclosed herein, low c-met biomarker expression is met diagnostic negative clinical status as defined in accordance with Table A herein.

In one aspect, amount of c-met biomarker is determined using a method comprising: (a) performing gene expression profiling, PCR (such as rtPCR), RNN-seq, microarray analysis, SAGE, MassARRAY technique, or FISH on a sample (such as a patient cancer sample) with anti-c-met antibody; and b) determining expression of a c-met biomarker in the sample. As is noted herein, other biomarkers may be detected. Exemplary other biomarkers are disclosed herein.

A sample from the patient is tested for expression of one or more of the biomarkers herein. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may contain compounds which are not naturally intermixed with the tissue in nature such as preservatives, anticoagulants, buffers, fixatives, nutrients, antibiotics, or the like. Examples of tumor samples herein include, but are not limited to, tumor biopsies, tumor cells, serum or plasma, circulating plasma proteins, ascitic fluid, primary cell cultures or cell lines derived from tumors or exhibiting tumor-like properties, as well as preserved tumor samples, such as formalin-fixed, paraffin-embedded tumor samples or frozen tumor samples. In one embodiment, the patient sample is a formalin-fixed paraffin-embedded (FFPE) tumor sample (e.g., a NSCLC tumor sample or a breast cancer tumor sample). The sample may be obtained prior to the patient's treatment with a cancer medicament (such as an anti-c-met antagonist). The sample may be obtained from the primary tumor or from a metastatic tumor. The sample may be obtained when the cancer is first diagnosed or, for example, after the tumor has metastasized. In some embodiments, the tumor sample is of lung, lymph node, liver or brain.

Various methods for determining expression of mRNA, protein, or gene amplification include, but are not limited to, gene expression profiling, polymerase chain reaction (PCR) including quantitative real time PCR (qRT-PCR), RNA-Seq, FISH, microarray analysis, serial analysis of gene expression (SAGE), MassARRAY, proteomics, immunohistochemistry (IHC), etc. In some embodiments, protein expression is quantified. Such protein analysis may be performed using IHC, e.g., on patient tumor samples.

Various exemplary methods for determining biomarker expression will now be described in more detail.

## 1. Gene Expression Profiling

In general, methods of gene expression profiling can be divided into two large groups: methods based on hybridization analysis of polynucleotides, and methods based on sequencing of polynucleotides. The most commonly used methods known in the art for the quantification of mRNA expression in a sample include northern blotting and *in situ* hybridization (Parker &Barnes, Methods in Molecular Biology 106:247-283 (1999)); RNAse protection assays (Hod, Biotechniques 13:852- 854 (1992)); and polymerase chain reaction (PCR) (Weis et al., Trends in Genetics 8:263-264 (1992)). Alternatively, antibodies maybe employed that can recognize specific duplexes, including DNA duplexes, RNA duplexes, and DNA-RNA hybrid duplexes or DNA- protein duplexes. Representative methods for sequencing-based gene expression analysis include Serial Analysis of Gene Expression (SAGE), and gene expression analysis by massively parallel signature sequencing (MPSS).

## 2. Polymerase Chain Reaction (PCR)

Of the techniques listed above, a sensitive and flexible quantitative method is PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples. General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini- columns. Other commercially available RNA isolation kits include MASTERPURE® Complete DNA and RNA Purification Kit (EPICENTRE®, Madison, Wis.), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

As RNA cannot serve as a template for PCR, the first step in gene expression profiling by PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse- transcribed using a GENEAMP™ RNA PCR kit (Perkin Elmer, Calif., USA), following the manufacturer's instructions. The derived cDNA can then be used as a template in the subsequent PCR reaction. Although the PCR step can use a variety of thermostable DNA-dependent DNA polymerases, it typically employs the Taq DNA polymerase, which has a 5'- 3' nuclease activity but lacks a 3'-5' proofreading endonuclease activity. Thus, TAQMAN® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TAQMAN® PCR can be performed using commercially available equipment, such as, for example, ABI PRISM 7700® Sequence Detection System® (Perkin- Elmer-Applied Biosystems, Foster City, Calif., USA), or Lightcycler (Roche Molecular Biochemicals, Mannheim, Germany). In a preferred embodiment, the 5' nuclease procedure is run on a real-time quantitative PCR device such as the ABI PRISM 7700® Sequence Detection System. The system consists of a thermocycler, laser, charge- coupled device (CCD), camera and computer. The system amplifies samples in a 96-well format on a thermocycler. During amplification, laser- induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Ct).

To minimize errors and the effect of sample-to-sample variation, PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and P-actin.

A more recent variation of the PCR technique is quantitative real time PCR (qRT-PCR), which measures PCR product accumulation through a dual-labeled fluorigenic probe (i.e., TAQMAN® probe). Real time PCR is compatible both with quantitative competitive PCR, where internal competitor for each target sequence is used for normalization, and with quantitative comparative PCR using a normalization gene contained within the sample, or a housekeeping gene for PCR. For further details see, e.g. Held et al., Genome Research 6:986-994 (1996).

The steps of a representative protocol for profiling gene expression using fixed, paraffin-embedded tissues as the RNA source, including mRNA isolation, purification, primer extension and amplification are given in various published journal articles (for example: Godfrey et al., J. Molec. Diagnostics 2: 84-91 (2000); Specht et al., Am. J. Pathol. 158: 419-29 (2001)). Briefly, a representative process starts with cutting about 10 microgram thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by PCR.

According to one aspect of the present invention, PCR primers and probes are designed based upon intron sequences present in the gene to be amplified. In this embodiment, the first step in the primer/probe design is the delineation of intron sequences within the genes. This can be done by publicly available software, such as the DNA BLAT software developed by Kent, W., Genome Res. 12(4):656-64 (2002), or by the BLAST software including its variations. Subsequent steps follow well established methods of PCR primer and probe design.

In order to avoid non-specific signals, it is important to mask repetitive sequences within the introns when designing the primers and probes. This can be easily accomplished by using the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Rozen and Skaletsky (2000) Primer3 on the WWW for general users and for biologist programmers. In: Krawetz S, Misener S (eds) Bioinformatics Methods and Protocols: Methods in Molecular Biology. Humana Press, Totowa, N.J., pp 365-386).

Factors considered in PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases. Tm's between 50 and 80° C., *e.g.* about 50 to 70° C. are typically preferred.

For further guidelines for PCR primer and probe design see, *e.g.* Dieffenbach et al., "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T. N. Primerselect: Primer and probe design. Methods Mol. Biol. 70:520-527 (1997), the entire disclosures of which are hereby expressly incorporated by reference.

### *3. RNN-Seq*

RNA-Seq, also called Whole Transcriptome Shotgun Sequencing (WTSS) refers to the use of high-throughput sequencing technologies to sequence cDNA in order to get information about a sample's RNA content. Publications describing RNA-Seq include: Wang et al. "RNA-Seq: a revolutionary tool for transcriptomics" Nature Reviews Genetics 10 (1): 57-63 (January 2009); Ryan et al. BioTechniques 45 (1): 81-94 (2008); and Maher et al. "Transcriptome sequencing to detect gene fusions in cancer". Nature 458 (7234): 97-101 (January 2009).

### *4. Microarrays*

Differential gene expression can also be identified, or confirmed using the microarray technique. Thus, the expression profile of breast cancer- associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin- embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GENCHIP™ technology, or Incyte's microarray technology.

The development of microarray methods for large-scale analysis of gene expression makes it possible to search systematically for molecular markers of cancer classification and outcome prediction in a variety of tumor types.

### 5. Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, *e.g.* Velculescu et al., Science 270:484-487 (1995); and Velculescu et al., Cell 88:243-51 (1997).

### 6. MassARRA Y Technology

The MassARRAY (Sequenom, San Diego, Calif.) technology is an automated, high-throughput method of gene expression analysis using mass spectrometry (MS) for detection. According to this method, following the isolation of RNA, reverse transcription and PCR amplification, the cDNAs are subjected to primer extension. The cDNA-derived primer extension products are purified, and dispensed on a chip array that is pre- loaded with the components needed for MALTI-TOF MS sample preparation. The various cDNAs present in the reaction are quantitated by analyzing the peak areas in the mass spectrum obtained.

### 7. Immunohistochemistry

Immunohistochemistry ("IHC) methods are also suitable for detecting the expression levels of the biomarkers of the present invention. Immunohistochemical staining of tissue sections has been shown to be a reliable method of assessing or detecting presence of proteins in a sample. Immunohistochemistry techniques utilize an antibody to probe and visualize cellular antigens in situ, generally by chromogenic or fluorescent methods. Thus, antibodies or antisera, preferably polyclonal antisera, and most preferably monoclonal antibodies specific for each marker are used to detect expression. As discussed in greater detail below, the antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromagenic or fluorogenic substrate is added to provide visualization of the antigen. Signal amplification occurs because several secondary antibodies may react with different epitopes on the primary antibody.

The primary and/or secondary antibody used for immunohistochemistry typically will be labeled with a detectable moiety. Numerous labels are available which can be generally grouped into the following categories:

(a) Radioisotopes, such as $^{35}$S, $^{14}$C, $^{125}$I, $^{3}$H, and $^{131}$I. The antibody can be labeled with the radioisotope using the techniques described in Current Protocols in Immunology, Volumes 1 and 2, Coligen et al., Ed. Wiley-Interscience, New York, N.Y., Pubs. (1991) for example and radioactivity can be measured using scintillation counting.
(b) Colloidal gold particles.
(c) Fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE® and SPECTRUM GREEN® and/or derivatives of any one or more of the above. The fluorescent labels can be conjugated to the antibody using the techniques disclosed in Current Protocols in Immunology, supra, for example. Fluorescence can be quantified using a fluorimeter.
(d) Various enzyme-substrate labels are available and U.S. Pat. No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al. Methods for the Preparation of Enzyme-Antibody Conjugates for use in Enzyme Immunoassay, in Methods in Enzym. (ed J. Langone & H. Van Vunakis), Academic press, New York, 73:147-166 (1981).

Examples of enzyme-substrate combinations include, for example:

(i) Horseradish peroxidase (HRPO) with hydrogen peroxidase as a substrate, wherein the hydrogen peroxidase oxidizes a dye precursor [e.g., orthophenylene diamine (OPD) or 3,3',5,5'-tetramethyl benzidine hydrochloride (TMB)]. 3,3-Diaminobenzidine (DAB) may also be used to visualize the HRP-labeled antibody;
(ii) alkaline phosphatase (AP) with para-Nitrophenyl phosphate as chromogenic substrate; and
(iii) β-D-galactosidase (β-D-Gal) with a chromogenic substrate (e.g., p-nitrophenyl-β-D-galactosidase) or fluorogenic substrate (e.g., 4-methylumbelliferyl-β-D-galactosidase).

Numerous other enzyme-substrate combinations are available to those skilled in the art. For a general review of these, see U.S. Pat. Nos. 4,275,149 and 4,318,980.

Sometimes, the label is indirectly conjugated with the antibody. The skilled artisan will be aware of various techniques for achieving this. For example, the antibody can be conjugated with biotin and any of the four broad categories of labels mentioned above can be conjugated with avidin, or vice verse. Biotin binds selectively to avidin and thus, the label can be conjugated with the antibody in this indirect manner. Alternatively, to achieve indirect conjugation of the label with the antibody, the antibody is conjugated with a small hapten and one of the different types of labels mentioned above is conjugated with an anti-hapten antibody. Thus, indirect conjugation of the label with the antibody can be achieved.

Aside from the sample preparation procedures discussed above, further treatment of the tissue section prior to, during or following IHC may be desired. For example, epitope retrieval methods, such as heating the tissue sample in citrate buffer may be carried out [see, e.g., Leong et al. Appl. Immunohistochem. 4(3):201 (1996)].

Following an optional blocking step, the tissue section is exposed to primary antibody for a sufficient period of time and under suitable conditions such that the primary antibody binds to the target protein antigen in the tissue sample. Appropriate conditions for achieving this can be determined by routine experimentation.

The extent of binding of antibody to the sample is determined by using any one of the detectable labels discussed above. Preferably, the label is an enzymatic label (e.g. HRPO) which catalyzes a chemical alteration of the chromogenic substrate such as 3,3'-diaminobenzidine chromogen. Preferably the enzymatic label is conjugated to antibody which binds specifically to the primary antibody (e.g. the primary antibody is rabbit polyclonal antibody and secondary antibody is goat anti-rabbit antibody).

Specimens thus prepared may be mounted and coverslipped. Slide evaluation is then determined, e.g. using a microscope.

IHC may be combined with morphological staining, either prior to or thereafter. After deparaffinization, the sections mounted on slides may be stained with a morphological stain for evaluation. The morphological stain to be used provides for accurate morphological evaluation of a tissue section. The section may be stained with one or more dyes each of which distinctly stains different cellular components. In one embodiment, hematoxylin is use for staining cellular nucleic of the slides. Hematoxylin is widely available. An example of a suitable hematoxylin is Hematoxylin II (Ventana). When lighter blue nuclei are desired, a bluing reagent may be used following hematoxylin staining. One of skill in the art will appreciate that staining may be optimized for a given tissue by increasing or decreasing the length of time the slides remain in the dye.

Automated systems for slide preparation and IHC processing are available commercially. The Ventana® Bench-Mark XT system is an example of such an automated system.

After staining, the tissue section may be analyzed by standard techniques of microscopy. Generally, a pathologist or the like assesses the tissue for the presence of abnormal or normal cells or a specific cell type and provides the loci of the cell types of interest. Thus, for example, a pathologist or the like would review the slides and identify normal cells (such as normal lung cells) and abnormal cells (such as abnormal or neoplastic lung cells). Any means of defining the loci of the cells of interest may be used (e.g., coordinates on an X-Y axis).

Anti-c-met antibodies suitable for use in IHC are well known in the art, and include SP-44 (Ventana), DL-21 (Upstate), ab27492 (Abcam), PA1-37483 (Pierce Antibodies). One of ordinary skill understands that additional suitable anti-c-met antibodies may be identified and characterized by comparing with c-met antibodies using the IHC protocol disclosed herein, for example.

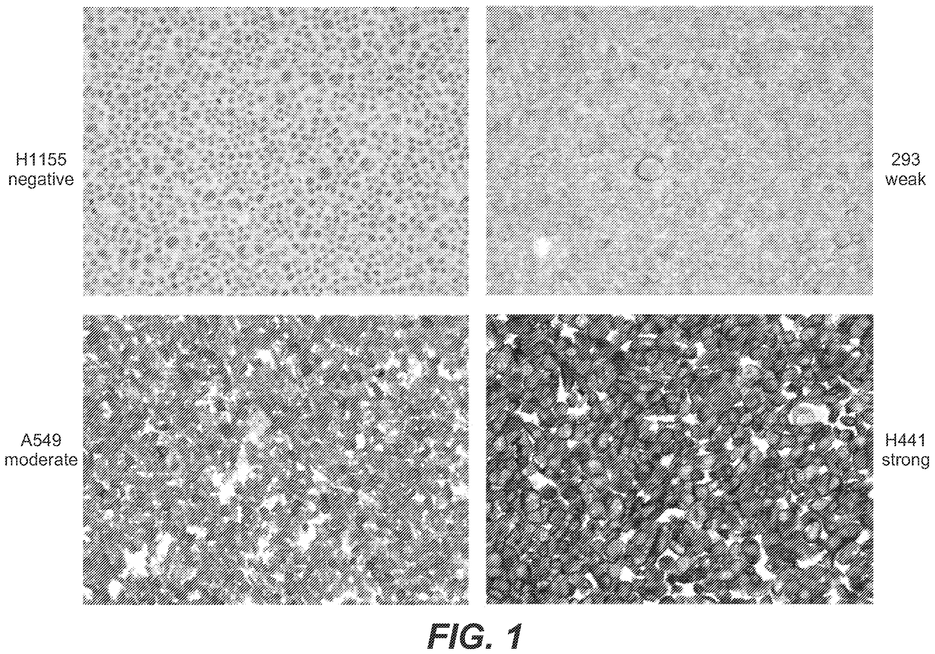

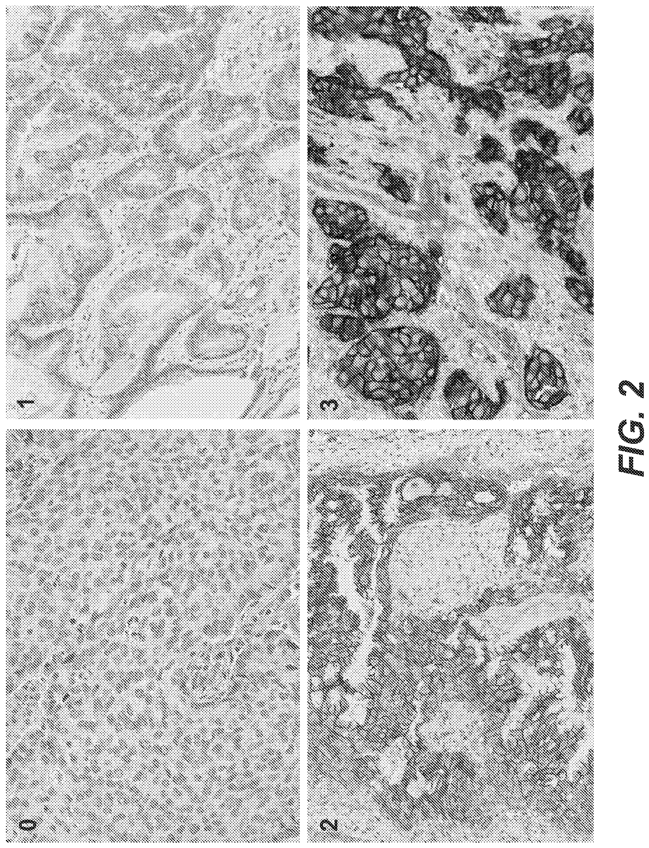

Control cell pellets with various staining intensities may be utilized as controls for IHC analysis as well as scoring controls. For example, H441 (strong c-met staining intensity); A549 (moderate c-met staining intensity); H1703 (weak c-met staining intensity), HEK-293 (293) (weak c-met staining intensity); and TOV-112D (negative c-met staining intensity) or H1155 (negative c-met staining intensity). In some embodiments, Figures 1 and/or 2 herein may be referred to for exemplary c-met IHC scoring intensity. In some embodiments, Figure 1 depicts exemplary 0, 1+, 2+ and 3+ c-met IHC scoring intensity, for example in accordance with the scoring scheme of Table A below. In some embodiments, Figure 2 depicts exemplary c-met IHC score 0, 1, 2, and 3, for example, in accordance with the scoring scheme of Table A below.

A c-met immunohistochemistry protocol and scoring scheme is exemplified herein. C-met staining intensity criteria may be evaluated according to Table A:

**Table A**

| IHC score | Staining criteria |
| --- | --- |
| 0 | samples with negative or equivocal staining, or < 50% tumor cells with weak (1+) or combined weak (1+) & moderate (2+) staining |
| 1 | 50% or more tumor cells with weak (1+) or combined weak (1+) & moderate (2+) staining, but less than 50% tumor cells with moderate (2+) or combined moderate (2+) & strong (3+) staining |
| 2 | 50% or more tumor cells with moderate (2+) or combined moderate (2+) & strong (3+) staining, but less than 50% tumor cells with strong (3+) staining |
| 3 | 50% or more tumor cells with strong (3+) staining |

In some embodiments, clinical "Met diagnostic positive" and "Met diagnostic negative" categories are defined as follows:

Met diagnostic positive: IHC score 2 or 3 (as defined in Table A), and
Met diagnostic negative: IHC score 0 or 1 (as defined in Table A).

In some embodiments, high c-met biomarker associated is an IHC score of 2, an IHC score of 3, or an IHC score of 2 or 3. In some embodiments, high c-met biomarker is 50% or more tumor cells with moderate c-met staining intensity, combined moderate/high c-met staining intensity or high c-met staining intensity. In some embodiments, high-c-met biomarker is 50% or more of tumor cells with moderate or high c-met staining intensity. In some embodiments, at least 50 tumor cells are analyzed in a sample. In some embodiments, at least 10, 20, 30, 40, 50, 60, or 70 or more tumor cells are analyzed in a sample. In some embodiments, high c-met biomarker expression is met diagnostic positive (met diagnostic positive clinical status) as defined in accordance with Table A herein.

In some embodiments, low c-met biomarker is an IHC score of 0, an IHC score of 1 or an IHC score of 0 or 1. In some embodiments, low c-met biomarker is negative c-met staining, less than 50% of tumor cells with weak or combined weak and moderate c-met staining intensity, or 50% or more tumor cells with weak or combined weak and moderate c-met staining intensity but less than 50% tumor cells with moderate or combined moderate and strong c-met staining intensity. In some embodiments, at least 10, 20, 30, 40, 50, 60, or 70 or more tumor cells are analyzed in a sample. In some embodiments, low c-met biomarker expression is met diagnostic negative (met diagnostic negative clinical status) as defined in accordance with Table A herein.

### 8. Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, *e.g.* my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

### 9. Gene amplification

Detecting amplification of the c-met gene is achieved using certain techniques known to those skilled in the art. For example, comparative genome hybridization may be used to produce a map of DNA sequence copy number as a function of chromosomal location. See, e.g., Kallioniemi et al. (1992) Science 258:818-821. Amplification of the c-met gene may also be detected, e.g., by Southern hybridization using a probe specific for the c-met gene or by real-time quantitative PCR.

In certain embodiments, detecting amplification of the c-met gene is achieved by directly assessing the copy number of the c-met gene, for example, by using a probe that hybridizes to the c-met gene. For example, a FISH assay may be performed. In certain embodiments, detecting amplification of the c-met gene is achieved by indirectly assessing the copy number of the c-met gene, for example, by assessing the copy number of a chromosomal region that lies outside the c-met gene but is co-amplified with the c-met gene. Biomarker expression may also be evaluated using an *in vivo* diagnostic assay, e.g. by administering a molecule (such as an antibody) which binds the molecule to be detected and is tagged with a detectable label (*e.g.* a radioactive isotope) and externally scanning the patient for localization of the label.

## IV. MEDICAL USES OF C-MET ANTAGONISTS AND OTHER CANCER MEDICAMENTS IN THERAPEUTIC METHODS

In one aspect, the invention provides a method for treating a patient with cancer, comprising administering a therapeutically effective amount of a c-met antagonist to the patient if the patient has been found to have a high (elevated) amount of c-met biomarker.

The invention also concerns a method for treating a patient with NSCLC comprising administering to the patient a therapeutically effective amount of a c-met antagonist (e.g., an anti-c-met antibody e.g. MetMAb), if the patient has been found to have an high amount of c-met biomarker (for example, if the patient's cancer expresses high c-met biomarker, for example, as determined using IHC). In some embodiments, NSCLC is squamous cell carcinoma. In some embodiments, NSCLC is adenocarcinoma. In some embodiments, the NSCLC is second-line or third-line locally advanced or metastatic NSCLC. In some embodiments, the NSCLC is locally advanced or metastatic NSCLC after failure of at least one prior chemotherapy regimen. Methods for determining c-met expression using IHC are discussed and exemplified herein. In some embodiments, the patient's cancer has been shown to expresses high c-met with an IHC score 2, an IHC score of 3, or an IHC score of at least 2 (2 or 3). In some embodiments, the patient's cancer (a sample from the patient's cancer) has been shown to contain 50% or more tumor cells with moderate c-met staining intensity, combined moderate/high c-met staining intensity or high c-met staining intensity. In some embodiments, high-c-met biomarker is 50% or more of tumor cells with moderate or high c-met staining intensity. In some embodiments, criteria disclosed herein are used to score c-met biomarker status as high or low.

Moreover, the invention provides a method for treating a patient with NSCLC comprising administering to the patient a therapeutically effective amount of a combination of a c-met antagonist (such as a anti-c-met antibody, such as MetMAb) and an EGRF antagonist (such as erlotinib), if the patient has been found to have an high amount of c-met biomarker. The patient treated herein desirably will benefit from (or be more likely to exhibit) greater progression free survival (PFS) and overall survival (OS) relative to a patient who has a reduced amount of the c-met biomarker. Exemplary EGFR antagonists are described herein. In some embodiments, the NSCLC is locally advanced or metastatic NSCLC after failure of at least one prior chemotherapy regimen.

The invention additionally concerns a method for treating a patient with cancer (such as NSCLC) comprising administering to the patient a therapeutically effective amount of a cancer other than a c-met antagonist, if the patient has been found to have a reduced amount of c-met biomarker (for example, if the patient's cancer expresses reduced c-met biomarker, for example, as determined by IHC). In some embodiments, the patient's cancer does not detectably express c-met or expresses c-met with an IHC score of 0, an IHC score of 1, or an IHC score of 0 or 1. In some embodiments, criteria disclosed herein are used to score c-met biomarker status as negative. In some embodiments, the patient's cancer (a sample from the patient's cancer) has been shown to contain negative c-met staining, less than 50% of tumor cells with weak or combined weak and moderate c-met staining intensity, or 50% or more tumor cells with weak or combined weak and moderate c-met staining intensity but less than 50% tumor cells with moderate or combined moderate and strong c-met staining intensity.

Cancer medicaments of the invention can be used either alone or in combination with other cancer medicaments. For instance, an anti-c-met antibody (for example, MetMAb) may be administered at a dose of about 15 mg/kg every three weeks, or at a dose of about 10 mg/kg every two weeks.

For instance, a c-met antibody may be co-administered with at least one additional therapeutic agent, *e.g.* with a chemotherapeutic agent, with other c-met antagonists (such as other c-met antibodies), with an EGFR antagonist (such as erlotinib), or with an anti-VEGF antibody (such a bevacizumab). A c-met antibody may be co-administered with an additional c-met antagonist. Such combination therapies noted above encompass combined administration (where two or more therapeutic agents are included in the same or separate formulations), and separate administration, in which case, administration of a first medicament can occur prior to, simultaneously, and/or following, administration of a second medicament. In one embodiment, an anti-c-met antibody (such as MetMAb) at a dose of about 15 mg/kg every three weeks; is used in combination with erlotinib (N-(3-ethynylphenyl)-6,7-bis(2-methoxyethoxy)-4-quinazolinamine) at a dose of 150 mg, each day of a three week cycle. In other embodiments, a c-met antagonist (e.g., anti-c-met antibody) is used in combination with an anti-VEGF antibody and chemotherapy (e.g., a taxane). An exemplary protocol is administering to a triple-negative metastatic breast cancer patient an anti-c-met antibody (e.g., MetMAb) administered at a dose of 10 mg/kg on Day 1 and Day 15 of a 28-day cycle, anti-VEGF antibody (e.g., bevacizumab) administered at a dose of 10 mg/kg on Day 1 and Day 15 of the 28-day cycle and paclitaxel administered at a dose of 90 mg/m$^2$ by IV infusion on Day 1, Day 8, and Day 15 of the 28-day cycle. An exemplary protocol is administering to a triple-negative metastatic breast cancer patient an anti-c-met antibody (e.g., MetMAb) administered at a dose of 10 mg/kg on Day 1 and Day 15 of a 28-day cycle, and paclitaxel administered at a dose of 90 mg/m$^2$ by IV infusion on Day 1, Day 8, and Day 15 of the 28-day cycle.

Cancer medicaments can also be used in combination with radiation therapy.

The medicament(s) herein can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. However, other dosage regimens may be useful. The progress of this therapy is easily monitored by conventional techniques and assays.

It is understood that any of the above formulations or therapeutic methods may be carried out using an immunoconjugate of the invention in place of or in addition to an antibody as the medicament.

In some embodiments, the patient did not receive more than two prior treatments for Stage IIIB/IV. In some embodiments, the patient did not receive more than 30 days of exposure to an investigational or marketed agent that can act by EGFR inhibition, or a known EGFR-related toxicity resulting in dose modifications. EGFR inhibitors include (but are not limited to) gefitinib, erlotinib, and cetuximab. In some embodiments, the patient did not receive chemotherapy, biologic therapy, radiotherapy or investigational drug within 28 days prior to randomization (except that optionally, kinase inhibitors maybe used within two weeks prior to randomization provided any drug related toxicity was adequately resolved). In some embodiments, the patient is not a patient with untreated and/or active (progressing or requiring anticonvulsants or corticosteroids for symptomatic control) CNS metastasis. Other patient exclusion criteria are described in the Examples, and the present inventions contemplate use of one or more of the exclusions described therein. In some embodiments, a sample of the patient's cancer has been shown to have wildtype EGFR. In some embodiments, a sample of the patient's cancer has not been shown to have mutated EGFR.

## V. ARTICLES OF MANUFACTURE

In another embodiment of the disclosure, an article of manufacture for use in treating cancer (such as NSCLC or breast cancer) is provided. The article of manufacture comprises a container and a label or package insert on or associated with the container. Suitable containers include, for example, bottles, vials, syringes, *etc.* The containers may be formed from a variety of materials such as glass or plastic. The container holds or contains a composition comprising the cancer medicament as the active agent and may have a sterile access port (for example the container may be an intravenous solution bag or a vial having a stopper pierceable by a hypodermic injection needle).

The article of manufacture may further comprise a second container comprising a pharmaceutically-acceptable diluent buffer, such as bacteriostatic water for injection (BWFI), phosphate-buffered saline, Ringer's solution and dextrose solution. The article of manufacture may further include other materials desirable from a commercial and user standpoint, including other buffers, diluents, filters, needles, and syringes.

The article of manufacture also includes information, for example in the form of a package insert, indicating that the composition is used for treating cancer based on expression level of the biomarker(s) herein. The insert or label may take any form, such as paper or on electronic media such as a magnetically recorded medium (e.g., floppy disk) or a CD-ROM. The label or insert may also include other information concerning the pharmaceutical compositions and dosage forms in the kit or article of manufacture.

According to one embodiment, an article of manufacture is provided comprising, packaged together, a c-met antagonist (e.g., an anti-c-met antibody) in a pharmaceutically acceptable carrier and a package insert indicating that the c-met antagonist is for treating a patient with cancer (such as NSCLC) based on expression of a c-met biomarker.

The disclosure also concerns a method for manufacturing an article of manufacture comprising combining in a package a pharmaceutical composition comprising a c-met antagonist (e.g., an anti-c-met antibody) and a package insert indicating that the pharmaceutical composition is for treating a patient with cancer (such as NSCLC) based on expression of a c-met biomarker.

The article of manufacture may further comprise an additional container comprising a pharmaceutically acceptable diluent buffer, such as bacteriostatic water for injection (BWFI), phosphate-buffered saline, Ringer's solution, and/or dextrose solution. The article of manufacture may further include other materials desirable from a commercial and user standpoint, including other buffers, diluents, filters, needles, and syringes.

## VI. DIAGNOSTIC KITS

The invention also concerns diagnostic kits useful for detecting any one or more of the biomarker(s) identified herein. Accordingly, a diagnostic kit is provided which comprises one or more reagents for determining expression of one or more of c-met, k-ras, ALK and EGFR biomarker in a sample from a cancer patient. Optionally, the kit further comprises instructions to use the kit to select a cancer medicament (e.g. a c-met antagonist, such as an anti-c-met antibody) for treating the cancer patient if the patient expresses the c-met biomarker at an high level. In another embodiment, the instructions are to use the kit to select a cancer medicament other than c-met antagonist (or other than an anti-c-met antibody) if the patient expresses the biomarker at a reduced level.

## VII. METHODS OF ADVERTISING

The disclosure herein also concerns a method for advertising a cancer medicament comprising promoting, to a target audience, the use of the cancer medicament (e.g. anti-c-met antibody) for treating a patient with cancer based on expression of c-met biomarker.

Advertising is generally paid communication through a non-personal medium in which the sponsor is identified and the message is controlled. Advertising for purposes herein includes publicity, public relations, product placement, sponsorship, underwriting, and sales promotion. This term also includes sponsored informational public notices appearing in any of the print communications media designed to appeal to a mass audience to persuade, inform, promote, motivate, or otherwise modify behavior toward a favorable pattern of purchasing, supporting, or approving the invention herein.

The advertising and promotion of the diagnostic method herein may be accomplished by any means. Examples of advertising media used to deliver these messages include television, radio, movies, magazines, newspapers, the internet, and billboards, including commercials, which are messages appearing in the broadcast media. Advertisements also include those on the seats of grocery carts, on the walls of an airport walkway, and on the sides of buses, or heard in telephone hold messages or in-store PA systems, or anywhere a visual or audible communication can be placed.

More specific examples of promotion or advertising means include television, radio, movies, the internet such as webcasts and webinars, interactive computer networks intended to reach simultaneous users, fixed or electronic billboards and other public signs, posters, traditional or electronic literature such as magazines and newspapers, other media outlets, presentations or individual contacts by, *e.g.*, e-mail, phone, instant message, postal, courier, mass, or carrier mail, in-person visits, etc.

The type of advertising used will depend on many factors, for example, on the nature of the target audience to be reached, e.g., hospitals, insurance companies, clinics, doctors, nurses, and patients, as well as cost considerations and the relevant jurisdictional laws and regulations governing advertising of medicaments and diagnostics. The advertising may be individualized or customized based on user characterizations defined by service interaction and/or other data such as user demographics and geographical location.

## EXAMPLES

**Materials and Methods**

**Samples:** Pretreatment patient samples were analyzed from a blind, Phase II, randomized, multicenter trial (further described below) designed to evaluate preliminary activity and safety of treatment with MetMAb plus erlotinib versus erlotinib plus placebo in NSCLC. Submission of either a formalin-fixed paraffin-embedded tumor specimens or unstained paraffin slides (15 slides) of representative tumor was required for all patients enrolled into the study.

**Immunohistochemistry (IHC):** Formalin-fixed, paraffin-embedded tissue sections were deparaffinized prior to antigen retrieval, blocking and incubation with primary anti-c-Met antibodies. Following incubation with secondary antibody and enzymatic color development, sections were counterstained and dehydrated in series of alcohols and xylenes before coverslipping.

The following protocol was used for IHC. The Ventana Benchmark XT system was used to perform c-met IHC staining using the following reagents and materials:

Primary antibody: Ventana anti-Total cMET (SP44) Rabbit Monoclonal Primary Antibody (cat# M3444.R)
Specimen Type: Formalin-fixed paraffin embedded (FFPE) samples and control cell pellets of varying staining intensities
Procedure Species: Human
Instrument: BenchMark XT
Epitope Recovery Conditions: Cell Conditioning 1 (CC1, Ventana, cat # 950-124)
Primary Antibody Conditions: 10 ug/ml /16 Min at 37C
Diluent: Ventana antibody dilution buffer (Tris HCl buffer, cat# 95119)
Naive Antibody for negative control: Naive Rabbit IgG at 3 ug/ml (Ventana Confirm negative control rabbit IgG , cat# 760-1029)
Detection: Ultraview Universal DAB Detection kit (Benchmark Reagent, polymer system, Ventana cat # 760-500) used according to manufacturer's instructions.
Counterstain: Ventana Hematoxylin II (cat # 790-2208)/ with Bluing reagent (Cat # 760-2037)

The Benchmark XT Protocol was as follows:

1. paraffin (Selected)
2. Deparaffinization (Selected)
3. Cell Conditioning (Selected)
4. Conditioner #1 (Selected)
5. Mild CC1 (Selected)
6. Standard CC1 (Selected)
7. Ab Incubation Temperatures (Selected)
8. 37C Ab Inc. (Selected)
9. Titration (Selected)
10. Hand Apply (Primary Antibody), and Incubate for (0Hr 16 Min)
11. Countstain (Selected)
12. Apply One Drop of (Hematoxylin II) (Countstain), Apply Coverslip, and Incubate for (4 Minutes)
13. Post Countstain (Selected)
14. Apply One Drop of (BLUING REAGENT) (Post Countstain), Apply Coverslip, and Incubate for (4 Minutes)
15. Wash slides in soap water to remove oil\
16. Rinse slides with water
17. Dehydrate slides through 95% Ethanol, 100% Ethanol to xylene (Leica autostainer program #9)
18. Cover slip.

**Scoring c-met expression by IHC:** The presence or absence of c-met expression in tumor specimens was evaluated using IHC. There was a wide dynamic range of c-met staining intensities in NSCLC, with tumor cells staining with negative, weak, moderate, or strong intensity. In addition, c-met expression within NSCLC tumor tissue was often heterogeneous; that is, tumor cells exhibited different levels of met expression within a sample. Both the intensity of IHC staining (negative, weak, moderate, or strong) and the proportion of tumor cells that stained at different intensity levels were considered when evaluating c-met expression by IHC. The criteria for defining met diagnostic positive tumors and met diagnostic negative tumors were defined before study unblinding, as determined by IHC following the scoring system below.

Tumor cells were scored for c-Met staining. In the majority of the cases, the staining was primarily membranous with some cytoplasmic signals (M, c), however, predominant cytoplasmic staining (C, m) was also observed in 5-10% samples. The staining was classified as strong (3+), moderate (2+), weak (1+), equivocal (+/-) or negative (-) staining intensity. Strong staining intensity was characterized by dark brown to black cytoplasm and/or thickened, darkened membranes of similar intensity. Moderate staining intensity was characterized by brown cytoplasm and/or membranes. The moderate staining lacked the blackness seen in strong signal intensity and membranes were thinner. Weak signal intensity was characterized by light brown cytoplasm. The weak signal lacked the rich brown color seen in moderate staining intensity and membranes were thinner. Negative or equivocal signal intensity was characterized by an absence of any detectable signal or a pale gray or tan signal, rather than brown, and without evidence of enhanced membrane staining.

In addition to evaluating staining intensity, percentages of various staining intensities/patterns were visually estimated in the samples with heterogeneous signals.

The following control cell pellets with various staining intensities were included as controls for IHC analysis as well as scoring controls: H441 (+++ (strong) staining); A549 (++ (moderate) staining); H1703 (+ (weak) staining); and TOV-112D (- (negative) staining) or H1155 (- (negative) staining). For lung samples, bronchial epithelium was also used as an internal control, as it exhibited moderate (2+) membranous staining. Isotype control antibody was also used to determine the basal background staining of testing samples. Positive control antibody, such as Ki-67, was used to evaluate tissue quality. Figure 2 shows an example of NSCLC tissue samples with IHC scores of 0, 1, 2, or 3, prepared according to the IHC protocol above. As shown in Figure 2, c-met staining signal may be distributed homogenously having a uniform level of intensity throughout the neoplastic portions of the tumor, or distributed heterogeneously having more than one intensity level. Staining may be heterogeneous, for example, samples may have more than one intensity level. Figure 1 shows exemplary control cell pellets prepared according to the IHC protocol above.

After evaluating IHC staining, an IHC score was reported based on analysis of at least 50 tumor cells with the following scoring cutoffs:

|  | IHC score* | Staining criteria |
| --- | --- | --- |
| Diagnostic negative | 0 | samples with negative or equivocal staining, or < 50% tumor cells with weak (1+) or combined weak (1+) & moderate (2+) staining |
|  | 1 | 50% or more tumor cells with weak (1+) or combined weak (1+) & moderate (2+) staining, but less than 50% tumor cells with moderate (2+) or combined moderate (2+) & strong (3+) staining |
| Diagnostic positive | 2 | 50% or more tumor cells with moderate (2+) or combined moderate (2+) & strong (3+) staining, but less than 50% tumor cells with strong (3+) staining |
|  | 3 | 50% or more tumor cells with strong (3+) staining |
| *interchangeably termed "IHC clinical score" or "clinical score" | | |

Vascular staining was documented, but not used for IHC score, partly due to lack of sufficient vasculature in some biopsy samples.

Clinical "Met diagnostic positive" and "Met diagnostic negative" categories were defined as follows:

Met diagnostic negative: IHC score 0 or 1
Met diagnostic positive: IHC score 2 or 3.

For clarity, it is noted that the present application uses the following terminology:

| IHC score | Clinical diagnostic category |
| --- | --- |
| 0 or 1 | Met diagnostic negative, interchangeably termed Met Dx-, Met low, Met-Low, Met Low |
| 2 or 3 | Met diagnostic positive, interchangeably termed Met Dx+, Met high, Met-High, Met High |

In addition, in US patent application No. 61/378,911, filed August 31, 2010, a related patent application to the present application, the terms "met positive" and "met negative" were used to refer to IHC score 2 or 3, and IHC score 0 or 1, respectively.

**Clinical trial**

Lung cancer is the leading cause of cancer death globally: it kills more people than breast, colon, kidney, liver, melanoma and prostate cancers combined. Each year 1.18 million people die as a result of the disease (Parkin DM. CA Cancer J Clin (2005) 55:74-108). The majority of patients with lung cancer are diagnosed when the disease is at an advanced stage and has spread to other parts of the body.

Non-small cell lung cancer (NSCLC) is the most common form of the disease, accounting for approximately 85% of all cases. Only 7.5% of people diagnosed with advanced (stage IV) NSCLC are expected to be alive after five years. NSCLC can be classified as being an adenocarcinoma, squamous cell carcinoma or large cell carcinoma. Adenocarcinoma is the most common form of NSCLC. There is growing evidence that the two major NSCLC histologic subtypes, adenocarcinoma (accounting for approximately 40% of lung cancers) and SCC (accounting for approximately 25% of lung cancer) exhibit unique biologic properties resulting in differential responses to similar therapies. Pemtrexed, while active in adenocarcinoma, has shown relatively lower efficacy in SCC (Scagliotti et al, 2008, J Clin Oncol. 26(21):3543-51. Epub 2008 May 27). Treatment with bevacizumab in NSCLC patients with centrally located SCC has resulted in increased bleeding risk (Sandler et al 2006, N Engl J Med. 355(24):2542-50). Finally, SCC patients with metastatic disease historically have worse overall survival compared with adenocarcinoma. Thus, a need to identify effective regimens for this histologic subset remains.

The original protocol for this study was described, e.g., in WO2010/045345; however, the study was later modified to add enrollment of 50 patients with squamous cell histology as follows. Patients were randomized in a 1:1 ratio to one of the two treatment arms: MetMAb + erlotinib versus erlotinib + placebo. Once 120 patients, comprising the "overall" population were enrolled, eligibility was restricted to patients with squamous cell carcinoma (SCC) histology to ensure that a total of approximately 80 patients with SCC were enrolled in the study. Randomization for the first 120 patients comprising the "overall" population was stratified by smoking status, performance status, and histology, and randomization for the next 50 SCC patients was to be stratified by smoking status and performance status. During this study, patients and treating individuals, including the investigators, were blinded to the treatment assignment of study drug (MetMAb or placebo). A protocol-specific analysis of data from this study obtained on or before June 8, 2010 (n = 128 patients) suggested that patients whose tumors expressed lower levels of c-met did not derive benefit from MetMAb. On the basis of these data, screening and enrolment of new patient into the study was stopped and the trial was modified as follows:

- MetMAb was discontinued in patient who had c-met diagnostic-negative tumors, with the exception of certain patients if in the judgment of the investigator, the patient was deriving benefit from the study drug.
- Patients with c-met diagnostic tumors who discontinued MetMAb for reasons other than disease progression could be allowed to continue receiving erlotinib monotherapy.
- Patient with c-met diagnostic negative tumors randomized to the placebo+erlotinib arm who exhibited disease progression were only allowed to cross over to receive MetMAb (in addition to continuing erlotinib) if fresh tissue biopsy was obtained at disease progression and IHC results showed tumor was c-met diagnostic-positive.
- All other patient continued to receive treatment according to protocol.

The primary objective of the amended study was to evaluate progression-free survival (PFS) of MetMAb plus Erlotinib, relative to Erlotinib plus placebo, in patients with Met positive tumors (as determined by immunohistochemistry), in patients with squamous cell histology, as well as all patients (i.e., including patients with Met negative tumors).

The secondary objectives of this study were: (a) to evaluate progression free survival (PFS) in patients with squamous cell histology; (b) to determine the overall RECIST 1.0 response rate and duration of response in patients with c-met positive tumors, squamous cell histology, as well as overall patient population; (b) to characterize the safety and tolerability of MetMAb plus Erlotinib in patients with NSCLC; and (c) to evaluate minimum concentration (Cmin) and maximum concentration (Cmax) of both MetMAb and erlotinib in patients with NSCLC.

Additional objectives of this study were to (a) to evaluate overall survival, in patients with squamous cell histology, c-met positive tumors as well as in the overall population; (b) to evaluate the FDG-PET response rate by treatment group and in patients with c-met positive tumors, as well as overall; (c) to evaluate progression-free survival (PFS) in FDG-PET responders versus non-responders, by treatment group and in Met positive tumors, squamous cell histology, as well as in the overall population; (d) to evaluate the relationship between Response Evaluation Criteria In Solid Tumors (RECIST) 1.0 response at first tumor assessment and PFS; (e) to evaluate the relationship between response and changes in biomarkers (or baseline expression of) related to the HGF/Met and/or EGFR signaling pathways (including, but not limited to IL8 and serum HGF); (f) to evaluate potential mechanisms of resistance in patients who progress on study; and (g) evaluate time to progression in patients with c-met positive tumors as well as overall.

Study design. This study was a Phase II, double-blind, randomized, multicenter trial designed to evaluate the preliminary activity and safety of treatment with MetMAb plus Erlotinib versus Erlotinib plus placebo in second and third-line NSCLC. Approximately 120 histologically unspecified patients from approximately 40 multinational sites were randomized in a 1:1 ratio to one of the two treatment arms: MetMAb plus Erlotinib vs. Erlotinib plus placebo. Once 120 patients, comprising the "overall" population were enrolled, eligibility was restricted to patients with squamous cell carcinoma (SCC) histology to ensure that a total of approximately 80 patients with SCC are enrolled in the study. Randomization for the first 120 patients comprising the "overall" population was stratified by smoking status (non-smokers and smokers who have quit more than 10 years ago versus current smokers and smokers who have quit less than 10 years ago), performance status and histology, and randomization for the next 50 SCC patients will be stratified by smoking status and performance status. Treatment in each arm was continued until progression of disease, unacceptable toxicity, or any other discontinuation criterion was met. Upon disease progression, patients randomized to the Erlotinib plus placebo arm were given the option to receive MetMAb (in addition to continuing Erlotinib), provided they continue to meet eligibility criteria. Safety data collected from this cross-over was summarized for hypothesis generating purposes.

As noted above, a protocol-specific analysis of data from this study obtained on or before June 8, 2010 (n = 128 patients) suggested that patients whose tumors expressed lower levels of c-met did not derive benefit from MetMAb. On the basis of these data, screening and enrollment of new patient into the study was stopped and the trial was modified as noted above.

During the study, data on tumor measurement and survival status were collected for evaluation of PFS, overall survival (OS) and overall response rate (ORR). CT scans were obtained at baseline and for the first four cycles at an approximately every 6 week intervals (i.e., every two three-week cycles of MetMAb/placebo). After four cycles, routine CT scans were performed approximately every 9 weeks (every 3 cycles of MetMAb/placebo). FDG-PET imaging is obtained at baseline and at Day 10-14 of Cycle 1. During the course of this study, an Image Reading facility (IRF) evaluated FDG-PET results and determined whether FDG-PET imaging should continue in all participating sites or whether FDG-PET imaging should be confined to a few sites based on data received.

In some patients, exploratory serum and plasma samples were collected to determine the effect of MetMAb plus Erlotinib on circulating levels of potential markers of activity, including but not limited to IL-8 and HGF. Correlating these and other markers with clinical outcomes assists in identifying predictive biomarkers, e.g., markers in circulation that may reflect drug activity or response to therapy. Blood for serum and plasma was drawn from consenting patients at pre-specified times and evaluated for levels of these exploratory markers.

Expression of c-met and/or EGFR was determined in a pre-treatment sample of the tumor. C-met and/or EGFR expression was determined by IHC and/or FISH analysis.

Because of the well-established survival benefit of Eastern Asians when treated with EGFR-directed therapies, this study did not allow more than 20% of the evaluable study population to be Eastern Asians.

Outcome measures. The primary outcome measure of this study was progression free survival (PFS) defined by the Response Evaluation Criteria In Solid Tumors (RECIST)) 1.0 or death from any cause within thirty days of the last treatment.

The secondary outcome measures for this study were as follows:

(a) overall response (OR) (partial response plus complete response) as determined using RECIST 1.0 in Met positive tumors and overall; and
(b) duration of OR.

Exploratory outcome measures included the following:

(a) FDG-PET response rates, as determined based on the definitions of the European Organization for Research of Cancer (EORTC);
(b) Incidence, nature and severity of adverse events and serious adverse events, and changes in vital signs, physical findings, and clinical laboratory results during and following study drug administration will be monitored; and
(c) Overall survival (time from randomization until death from any cause within 30 days of the last study treatment).

Primary and secondary outcome measures will be assessed in patients with c-met positive tumors, in SCC patients, and in the "overall" population.

Serum samples will be collected for analysis of MetMAb and erlotinib pharmacokinetics and pharmacodynamics.

Patient selection criteria. Adult patients were eligible to participate in this study if they have inoperable locally advanced or metastatic (Stage IIIb/IV) NSCLC (e.g., as determined by histological studies) and have received at least one, but no more than two prior regimens for Stage IIIb/IV NSCLC disease. Availability at the site of a representative formalin-fixed paraffin-embedded tumor specimen that enabled the definitive diagnosis of NSCLC with adequate viable tumor cells in a tissue block (preferred) or 15 unstained serial slides, accompanied by an associated pathology report, was required prior to randomization. Cytological samples or fine-needle aspiration samples were not acceptable. The patient may still have be eligible if the patient provided at least greater than or equal to 5 unstained serial slides or was willing to consent to and undergo a pre-treatment core or excisional biopsy of the tumor. Cytological or fine-needle aspiration samples were not acceptable.

In this study, cancer staging followed the American Joint Committee on Cancer's AJCC Cancer Staging Manual, 6th edition. Patients who received neo-adjuvant and/or adjuvant therapy for Stage I-IIIa disease prior to their first-line regiment (for Stage IIIb/IV) were eligible for study participation, provided they also received first-line therapy for Stage IIIb/IV disease. In some embodiments, at least one of the chemotherapy containing regimens (for any stage) was platinum-based. Patients must have had measurable disease as determined by RECIST. In some embodiments, patients had at least one measurable lesion on a pre-treatment FDG-PET scan that is also a target lesion on CT according to RECIST. In some embodiments, patients provided a pre-treatment tumor specimen, and possessed at least one measurable lesion on a pre-treatment FDG-PET scan that is also a target lesion on CT according to RESIST.

In some embodiments, excluded subjects were subjects who had more than two prior treatments for Stage IIIB/IV. In some embodiments, excluded subjects included subjects with more than 30 days of exposure to an investigational or marketed agent that can act by EGFR inhibition, or a known EGFR-related toxicity resulting in dose modifications. EGFR inhibitors include (but are not limited to) gefitinib, erlotinib, and cetuximab. In some embodiments, excluded subjects included subjects who received chemotherapy, biologic therapy, radiotherapy or investigational drug within 28 days prior to randomization (except that kinase inhibitors may be used within two weeks prior to randomization provided any drug related toxicity was adequately resolved), or subjects with untreated and/or active (progressing or requiring anticonvulsants or corticosteroids for symptomatic control) CNS metastasis. In some embodiments, subjects with history of brain metastasis were eligible for study participation, as long as they met the following criteria: (a) measurable disease outside the CNS, as defined by RECIST; (b) no radiographic evidence of interim progression between the completion of CNS-directed therapy and the screening radiographic study; (c) CNS-directed treatment which may include neurosurgery or stereotactic radiosurgery; (d) the screening of CNS radiographic study was $\geq$ 4 weeks since completion of radiotherapy and $\geq$ 2 weeks since the discontinuation of corticosteroids and anticonvulsants; (e) radiotherapy and stereotactic radiosurgery was completed $\geq$ 4 weeks prior to Day 1; and (f) neurosurgery was completed $\geq$ 24 weeks prior to Day 1, and brain biopsy was completed $\geq$ 12 weeks prior to Day 1.

In some embodiment, excluded subject also included subjects with history of serious systemic disease, including myocardial infarction within the last 6 months prior to randomization, uncontrolled hypertension (persistent blood pressure > 150/100 mmHg on antihypertensives), unstable angina, New York Heart Association (NYHA) Grade II or greater congestive heart failure, unstable symptomatic arrhythmia requiring medication (patients with chronic atrial arrhythmia, i.e., atrial fibrillation or paroxysmal supraventricular tachycardia are eligible), or Grade II or greater peripheral vascular disease; uncontrolled diabetes as evidenced by fasting serum glucose level > 200mg/dL; major surgical procedure or significant traumatic injury within 28 days prior to randomization; anticipation of need for a major surgical procedure during the course of the study; local palliative radiotherapy within 7 or 14 days prior to randomization or persistent adverse effects from radiotherapy that have not been resolved to Grade II or less prior to randomization; inability to take oral medication or requirement for IV alimentation or total parenteral nutrition with lipids, or prior surgical procedures affecting gastrointestinal absorption. In some embodiments, excluded subjects included subjects having any of the following uncorrected abnormal hematologic values (within 2 weeks prior to randomization): ANC < 1,500 cells/$\mu$L, Platelet count < 100,000 cells/$\mu$L, Hemoglobin < 9.0 g/dL, following RBC transfusion, Other baseline laboratory values (within 2 weeks prior to randomization), Serum bilirubin > 1.5xULN, Serum creatinine > 1.5xULN, Uncontrolled hypercalcemia (> 11.5 mg/dL or > 1.5 ionized calcium). In some embodiments, excluded subject included subjects having uncontrolled diabetes and subjects having symptomatic hypercalcemia requiring continued use of bisphosphonate therapy.

In some embodiments, excluded subjects included pregnant or breast-feeding women; subjects having other malignancies that have undergone a putative surgical or radiotherapy cure (e.g., intraepithelial carcinoma of the cervix uteri, localized prostate cancer post prostatectomy, or basal/squamous cell carcinoma of the skin) within 5 years prior to randomization could be discussed with the medical monitor; or evidence of confusion or disorientation, or history of major psychiatric illness. See also additional exclusions on the label of erlotinib.

Statistical methods and efficacy analysis. Primary and secondary efficacy analyses included all randomized patients, with patients allocated to the treatment arm to which they were randomized. Safety analyses included all randomized patients who received at least one dose of study treatment, with patients allocated to the treatment arm associated with the regimen actually received. Demographic and baseline characteristics (e.g. age and sex) were summarized using means, standard deviations, medians, and ranges for continuous variables and proportions for categorical variables, as appropriate. Summaries were presented by overall patient population and by treatment arm. The baselines value of any variable were defined as the last available value prior to the first administration of study treatment.

Kaplan-Meier methodology was used to estimate the median PFS for each treatment arm. The stratification factors were determined by the computer readable form (CRF) data, not by data collected by the interactive voice readable system at the time of randomization unless the CRF data was missing. Estimation of the hazard ration (i.e., the magnitude of the treatment effect and the 95% confidence interval) was determined using a stratified Cox regression model with an indicator variable for MetMAb treatment. The same analysis methods as those described for PFS in "overall" population patients were applied to patients with met positive tumors and to patients with SCC histology. All deaths from any cause within 30 days of the last treatment were included as PFS events. Objective response was defined as a complete or partial response determined on two consecutive occasions greater than or equal to four weeks apart. Patients without a post-baseline tumor assessment were considered non-responders. An estimate of the objective response rate and 95% confidence interval (Blyth-Still-Casella) was calculated for each treatment arm. Confidence intervals for the difference in tumor response rate (Satnes and Snell 1980; Berger and Boos 1994) were calculated. For patients with an objective response, duration of objective response was defined as the time from the initial response to disease progression or death from any cause within 30 days of the last treatment. Methods for handling censoring and for analysis were the same as described for PFS. All secondary efficacy endpoints were assessed for patients with met positive tumors, patients with SCC histology and by "overall" patient population.

Trial drugs. MetMAb is a known recombinant, humanized, monovalent monoclonal antibody directed against c-met. MetMAb is supplied as a sterile liquid in a single-use 15-cc vial. Each vial contains 600mg of MetMAb in 10ml at a concentration of 60mg/ml in 10mM histidine acetate, 120nM trehalose, 0.02% polysorbate 20, pH 5.4. MetMAb vials are refrigerated at 2C-8C and remain refrigerated until just prior to use. MetMAb is administered intravenously, after dilution in normal saline (0.9%).

Erlotinib (TARCEVA®) is provided as a conventional, immediate-release tablet containing erlotinib as the hydrochloride salt. In addition to the active ingredient, erlotinib, tablets contain lactose (hydrous), microcrystalline cellulose, sodium starch glycolate, sodium lauryl sulfate and magnesium stearate. Tablets containing 25mg, 100mg and 150mg of Erlotinib are available.

Placebo consisted of 250 cc 0.9% NSS (Saline IV solution, 0.9%).

Study treatment. The dose of MetMAb was 15 mg/kg intravenously on Day 1 of a 3-week cycle. The weight at screening was used to determine the actual dose of MetMAb. The dose of erlotinib was 150 mg by mouth each day of a 3-week cycle. Dosage level for erlotinib may have been reduced to 100 mg (first reduction) or 50 mg (second reduction) for toxicity likely attributable to erlotinib (e.g., rash, diarrhea).

*MET* and *EGFR* copy number: *MET* and *EGFR* gene copy numbers were evaluated by fluorescent in situ hybridization (FISH). Greater than or equal to 5 copies of MET/cell were designated as FISH positive (see, Cappuzzo et al, J Clin Oncol (2009) 27:1667-9). True MET amplification was defined as tight gene cluster of greater than or equal to 15 copies of MET in greater than or equal to 10% of tumor cells or a MET/CEP7 ratio of greater than or equal to 2. For experiments in which both MET and EGFR copy number were evaluated, tumors were considered MET and EGFR FISH positive based on a scoring criterion used in multiple clinical studies (see, e.g., Varella-Garcia et al, J Clin Pathol (2009) 62, 970-7; Capuzzo et al, JNCI (2005); 97:643-55.): FISH positive: gene amplification or high polysomy, FISH negative: low polysomy, trisomy or disomy, Gene amplification: tight gene clusters or ≥15 copies of MET in ≥10% of tumor cells or MET to CEP7 ratio of ≥2, High polysomy: ≥4 copies of MET in ≥ 40% of tumor cells.

*EGFR, KRAS* and *MET* genotyping: DNA was isolated from macro-dissected tumor tissue slides. *EGFR* and *KRAS* mutations were evaluated using the DxS genotyping kits, *MET* exon 14 variants were evaluated by DHPLC (Transgenomics Inc, Omaha NE) and a *MET* N275S polymorphism was evaluated by pyrosequencing.

mRNA profiling: *MET, HGF, EGFR, AREG, and EREG* mRNA expression was evaluated on RNA extracted from macro-dissected tumor tissue slides using the Fluidigm platform. Transcript levels were normalized to the mean of two reference genes that are stably expressed in lung tissue and results are expressed as normalized expression values $(2^{-\Delta Ct})$.

Plasma HGF: HGF levels in plasma collected prior to treatment were evaluated by capture ELISA.

### Results of analysis based on data cut-off date of on or before June 8, 2010

128 patients with second or third line NSCLC were enrolled and randomized at 25 global sites from March 2009 to March 2010 to: (a) MetMAb (15mg/kg IV q3wks) plus erlotinib treatment (interchangeably termed "ME") (n=64) or (b) placebo plus erlotinib treatment (interchangeably termed "PE") (n=64). The data cut-off date used in this analysis was on or before June 8, 2010.

Patient disposition is shown in Table 1.

Table 1: Patient Disposition

| n (%) | Erlotinib +Placebo (n=64) | Erlotinib +MetMAb (n=64) | Total (n=128) |
|---|---|---|---|
| Discontinued Blinded Treatment | 48 (75.0) | 49 (76.6) | 97 (75.8) |
| Disease progression | 39 (60.9) | 34 (53.1) | 73 (57.0) |
| Adverse events | 2 (3.1) | 7 (10.9) | 9 (7.0) |
| Deaths | 4 (6.3) | 2 (3.1) | 6 (4.7) |
| Other | 3 (4.7) | 6 (9.4) | 9 (7.0) |

Patient demographics are shown in Table 2.

Table 2: Patient Demographics

| | Erlotinib+Placebo n=64 (%) | Erlotinib+MetMAb n=64 (%) |
|---|---|---|
| Median age (range) | 62 (42-83) | 66 (30-83) |

(continued)

| | Erlotinib+Placebo n=64 (%) | Erlotinib+MetMAb n=64 (%) |
|---|---|---|
| Sex: male, n (%) | 39 (60.9) | 36 (56.3) |
| Race: White, n (%) | 58 (90.6) | 56 (87.5) |
| ECOG: 0/1, n (%) | 62 (96.9) | 60 (93.8) |
| Non-squamous, n (%) | 48 (75) | 49 (76.6) |
| Squamous, n (%) | 16 (25) | 15 (23.4) |
| Never smoker, n (%) | 8 (12.5) | 10 (15.6) |
| Met high*, n (%) | 30 (50.8) | 35 (56.5) |
| Kras mutant**, n (%) | 13 (23.2) | 13 (23.2) |
| EGFR mutant**, n (%) | 6 (10.7) | 7 (12.5) |
| * Of 121 patients with evaluable tissue samples<br>** Of 112 patients with evaluable tissue samples. | | |

Baseline characteristics were well-balanced in the overall (ITT) population including prevalence of patients with Met high tumors (51%/56.5%; PE/ME), Kras mutation (23%/23%) and EGFR mutation (11%/12.5%). Tissue was evaluable for Met IHC analysis in 121 patients (95% of patients), and for EGFR and KRAS mutations in 112 patients.

Baseline characteristics by Met status are shown in Table 3.

Table 3: Baseline characteristics by Met status.

| | Met High (n=65) | | Met Low (n=56) | |
|---|---|---|---|---|
| | MetMAb+<br>Erlotinib (n=35) | Placebo+<br>Erlotinib (n=30) | MetMAb+<br>Erlotinib (n=27) | Placebo+<br>Erlotinib (n=29) |
| Age (yr):<br>　Median (range) | 66 (30 - 83) | 63 (44 - 82) | 66 (45 - 82) | 60(42-83) |
| >= 65 yrs | 18 (51.4%) | 14 (46.7%) | 14 (51.9%) | 11 (37.9%) |
| Sex:<br>　Male | 18 (51.4%) | 19 (63.3%) | 17 (63.0%) | 16 (55.2%) |
| Baseline ECOG:<br>　0/1 | 34 (97.1%) | 28 (93.3%) | 24 (88.9%) | 29 (100%) |
| Histopathology:<br>Adenocarcinoma Squamous | 26 (73.3%) | 21 (70.0%) | 12 (44.4%) | 17 (58.6%) |
| | 5 (14.3%) | 4 (13.3%) | 10 (37.0%) | 10 (34.5%) |
| Smoking History:<br>　Never smoker | 7 (20.0%) | 6 (20%) | 2 (7.4%) | 1 (3.4%) |
| 　Kras mutant * | 7 (22.6%) | 6(23.1%) | 6 (24.0%) | 7 (25.0%) |
| 　EGFR mutant* | 7 (22.6%) | 2 (7.7%) | 0 | 4 (14.3%) |
| * Of 112 patients with evaluable tissue samples | | | | |

In this study, tissue was obtained from 100% of patients. 95% of patients had adequate tissue for evaluation of Met by IDC. 54% of patients had "Met high" NSCLC.

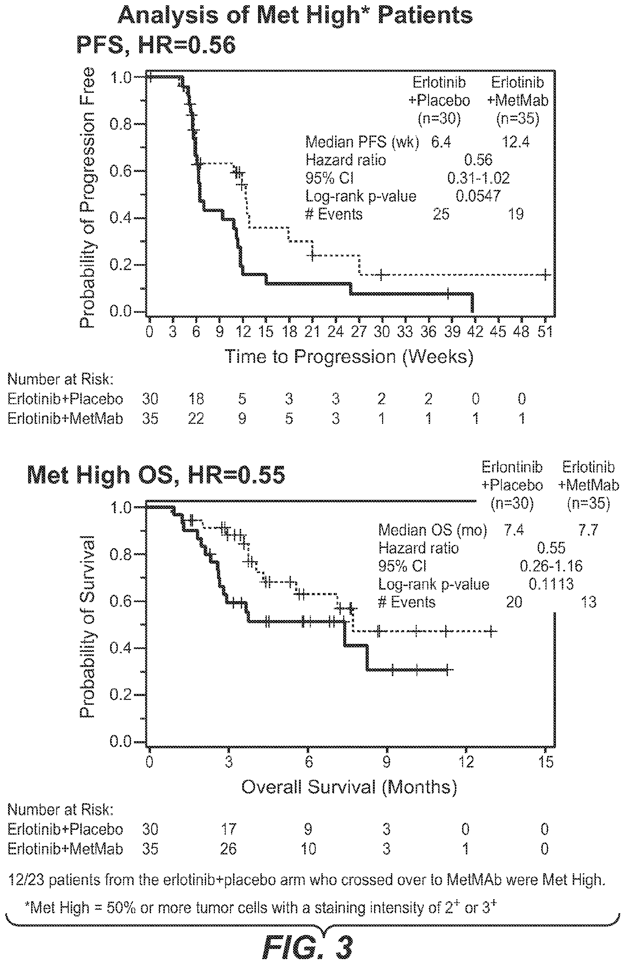

Treatment with MetMAb and erlotinib provided a clinically meaningful benefit to patients with Met high NSCLC (Figure 3). Both a PFS benefit (Hazard ratio (HR) 0.56; 95% CI 0.31, 1.02; p=0.05,) and an OS benefit (HR 0.55; 95% CI 0.25, 1.16; p=0.11) were observed in the Met-high patients treated with MetMAb plus erlotinib. Early and sustained separation of the curves was observed. The addition of MetMAb to erlotinib nearly doubled the progression free survival and overall survival in patients with met high NSCLC, compared to treatment with erlotinib + placebo (PFS in ME was 12.4 weeks, verses 6.4 weeks in PE; OS in ME was 7.7 weeks verses 7.4 weeks in PE). 23 patients from the erlotinib + placebo arm crossed over to MetMAb and 12 of the 23 patients who crossed over the ME had Met high biomarker expression.

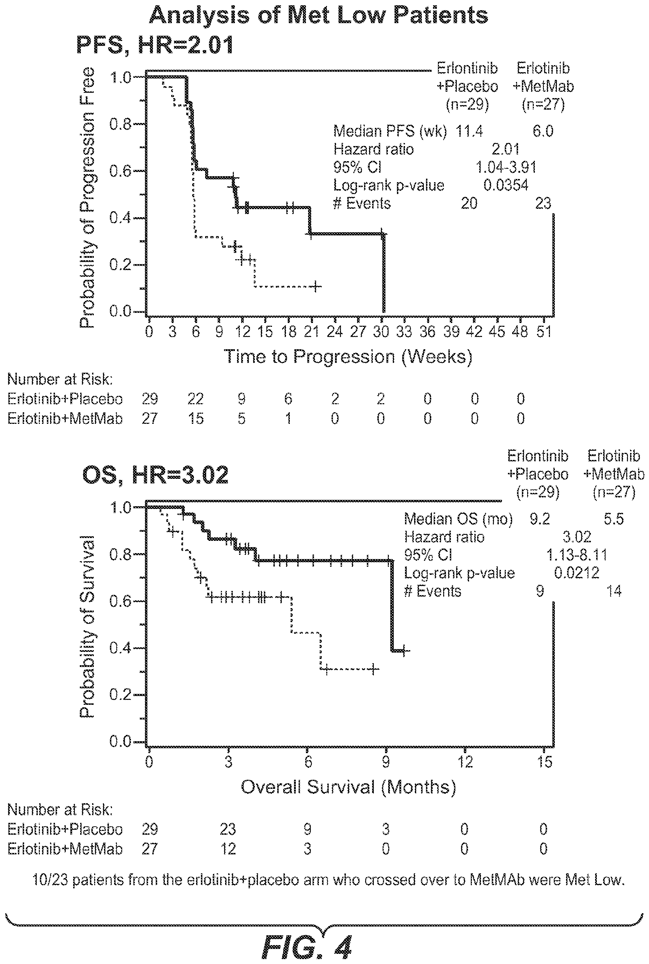

Met Low NSCLC patients did not benefit from treatment with MetMAb + erlotinib (Figure 4). MetMAb increased the risk of progression and death in Met low NSCLC patient verses treatment with erlotinib and placebo: both PFS (HR 2.01; 95% CI 1.04, 3.91; p=0.04) and OS (HR 3.26; 95% CI 1.20, 8.80; p=0.01) were worse in the ME cohort. ~70 percent of Met low patients treated with MetMAb and erlotinib progressed by the first assessment (CAT scan at week 6).

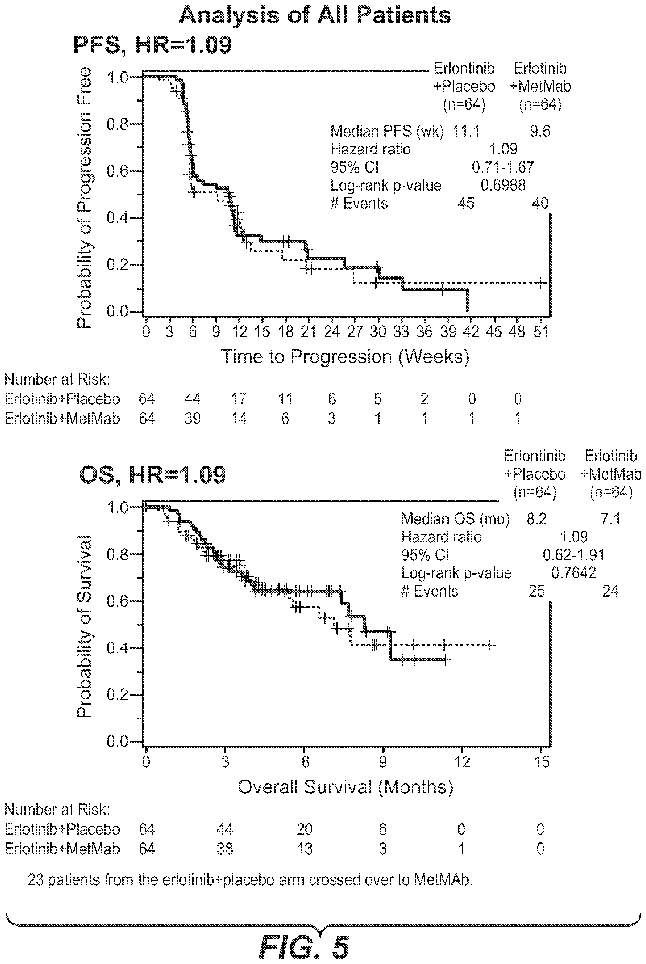

PFS and OS in the overall population (Figure 5). PFS and OS were not significantly different in the MetMAb plus erlotinib and placebo plus erlotinib treatment arms in the overall population. MetMAb plus erlotinib treatment did not show benefit in the overall population relative to placebo plus erlotinib treatment. The HRs for PFS and OS in the overall (interchangeably termed "intent to treat" or ITT) population were 1.09 (95% CI 0.71, 1.67; p=0.70) and 1.09 (95% CI 0.62, 1.91; p=0.76). Median PFS and media OS were consistent with previously reported findings in similar disease settings. 23 patients from the erlotinib + placebo arm crossed over to MetMAb+ erlotinib treatment. Objective response rates were: Erlotinib + Placebo n=3 (4.7%), Erlotinib + MetMAb n=4 (6.3%).

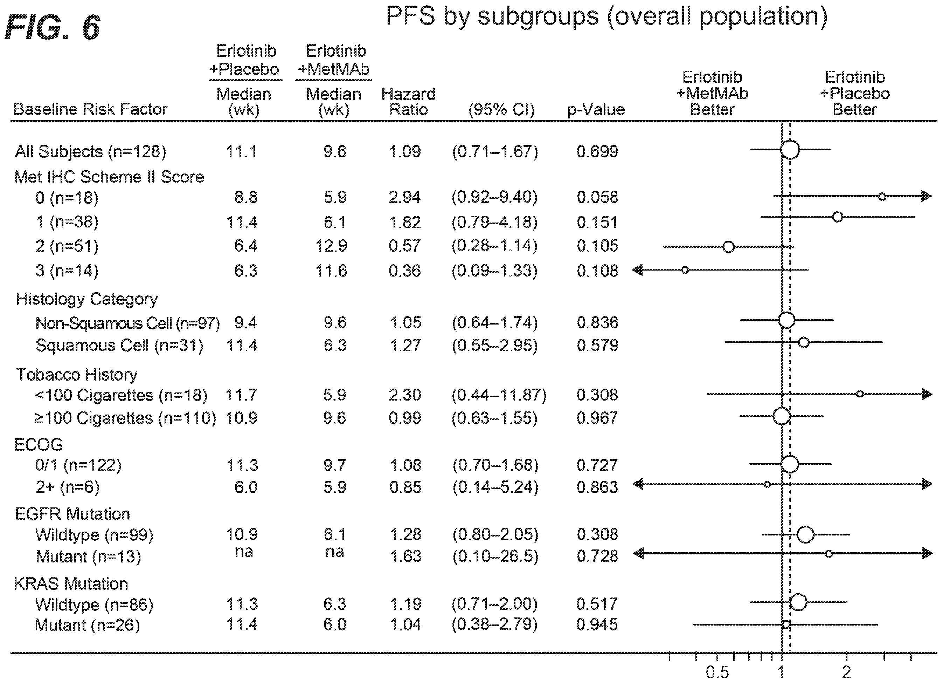

PFS was examined by subgroups (Figure 6). Met IHC status 3 and 2 patients showed benefit from MetMAb + erlotinib treatment, with status 3 patients showing greater benefit. Met IHC status 0 and 1 patients did not benefit from MetMAb plus erlotinib treatment. Status 0 patients did worse on MetMAb + erlotinib than status 1 patients. Selective benefit of MetMAb + erlotinib treatment was not observed in other subgroups, including: histology category (non-squamous verses squamous cell), tobacco history, ECOGCC, EGFR mutation or Kras mutation.

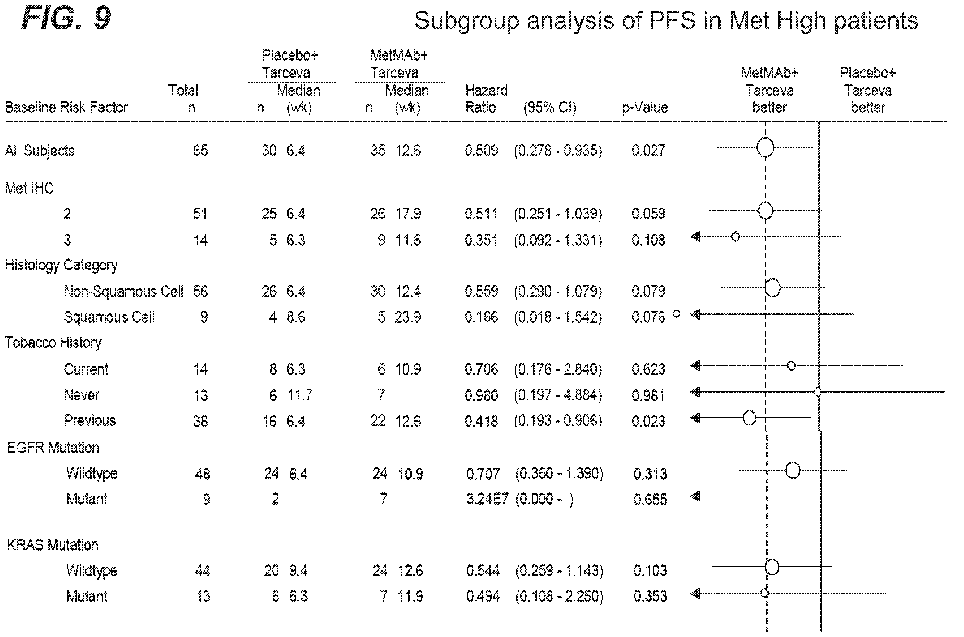

Figure 9 shows subgroup analysis of PFS in Met High patients.

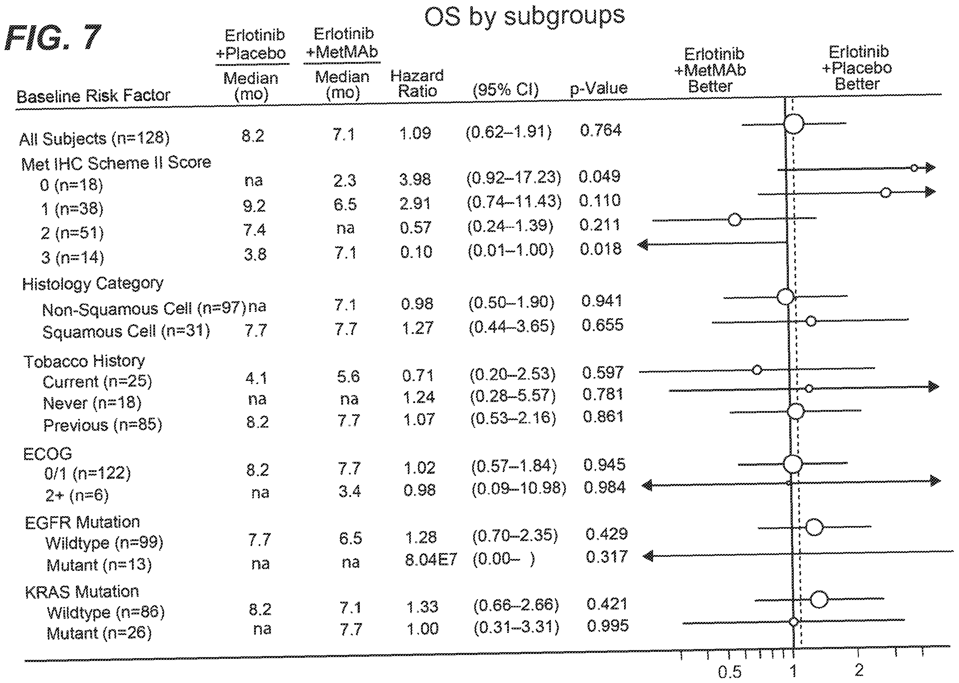

OS was also examined by subgroups (Figure 7). Similar to the PFS results, Met high IHC status 3 and 2 patients showed benefit from MetMAb + erlotinib treatment, with status 3 patients showing greater benefit. Met low IHC status 0 and 1 patients did not benefit from MetMAb plus erlotinib treatment. Status 0 patients did worse on MetMAb + erlotinib than status 1 patients. Selective benefit of MetMAb + erlotinib treatment was not observed in other subgroups, including: histology category (non-squamous verses squamous cell), tobacco history, ECOGCC, EGFR mutation or Kras mutation.

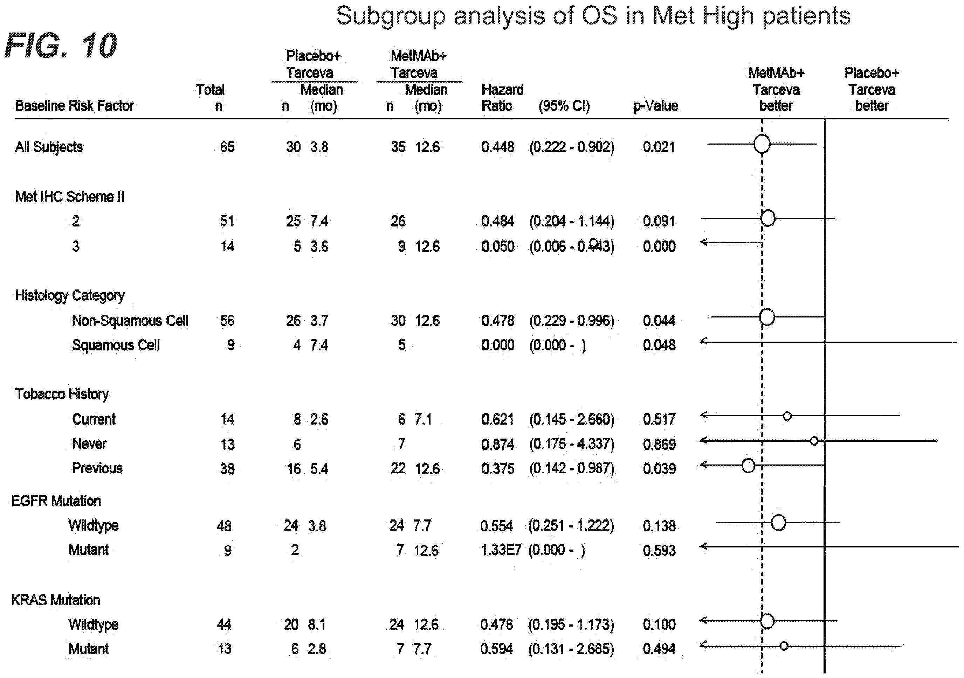

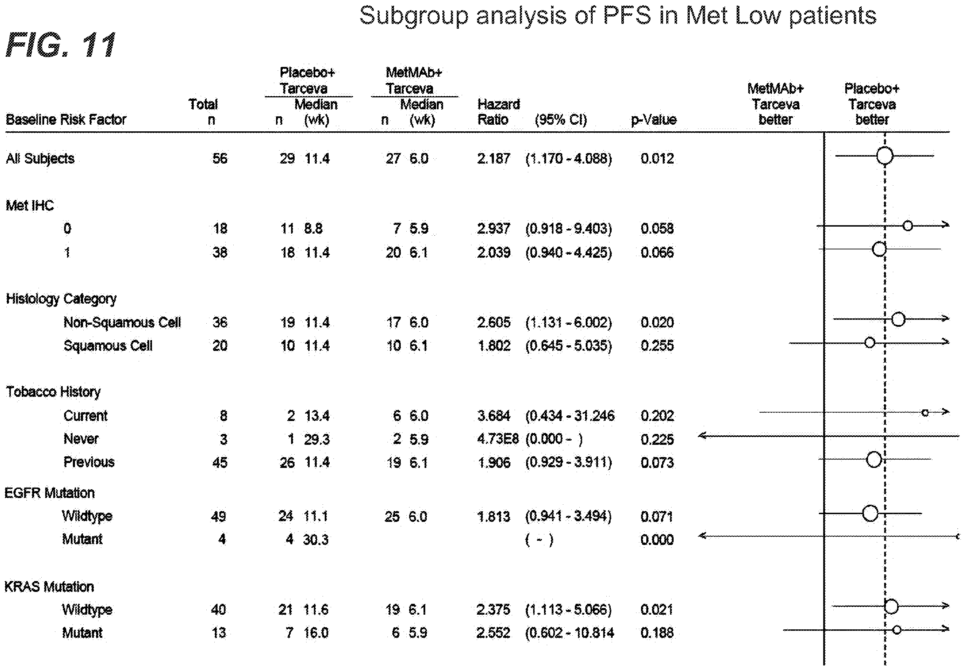

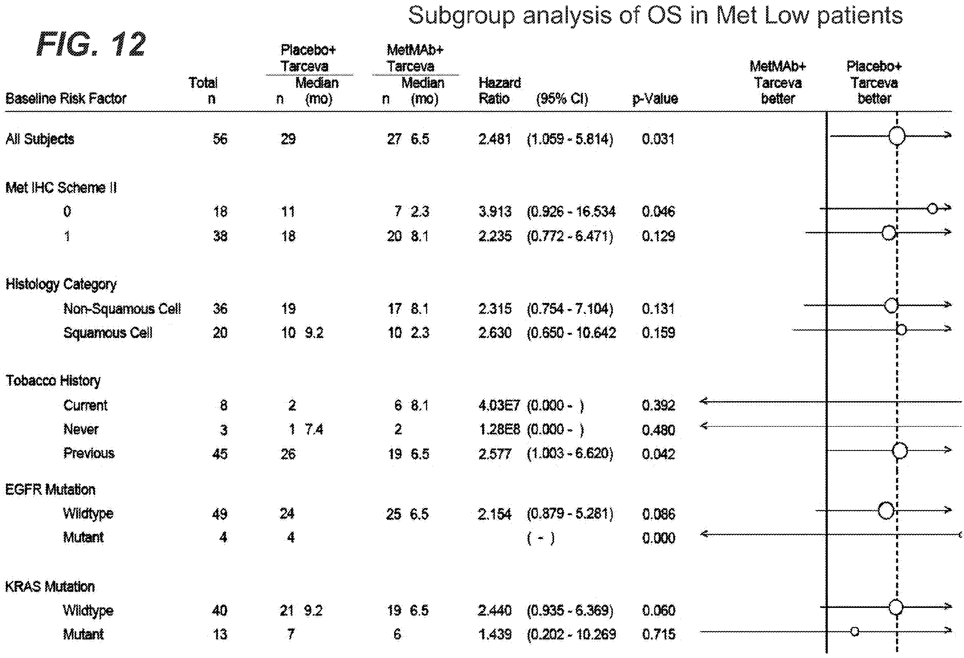

Figure 10 shows subgroup analysis of OS in Met High patients. Figures 11 and 12 show subgroup analysis of PFS and OS, respectively, in Met Low patients.

Overall survival was also analyzed in the Met high population excluding patients with known EGFR mutations. The degree of benefit of MetMAb + erlotinib treatment (relative to placebo + erlotinib) (OS HR =0.55, 95% CI 0.26, 1.20, p = 0.13) was approximately equal to overall survival in the Met high population including patients with known EGFR mutations Thus, the benefit from MetMAb + erlotinib in Met high patients was not driven by EGFR mutation status.

Key prognostic variables by Met status are shown in Table 4.

Table 4: Key prognostic variables by Met status

|  | Met Positive (n=65) | | Met Negative (n=56) | |
| --- | --- | --- | --- | --- |
|  | Erlotinib + Placebo (n=30) | Erlotinib +MetMAb (n=35) | Erlotinib +Placebo (n=29) | Erlotinib +MetMAb (n=27) |
| Histopathology (n=128) | | | | |
| Adenocarcinoma | 21 (70.0) | 26 (73.3) | 17(58.6) | 12 (44.4) |
| Squamous | 4(13.3) | 5 (14.3) | 10 (34.5) | 10 (37.0) |
| Mutational analyses (n=112) | | | | |
| Kras mutant | 6(23.1) | 7(22.6) | 7 (25.0) | 6 (24.0) |
| EGFR mutant | 2(7.7) | 7(22.6) | 4(14.3) | 0 |

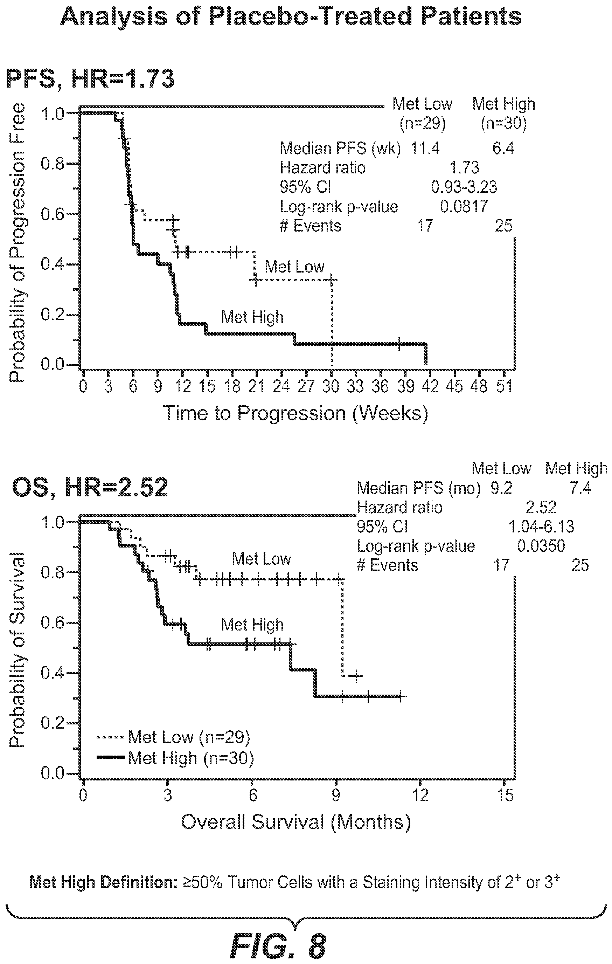

Met expression was prognostic for a worse outcome (Figure 8). In an analysis of the erlotinib + placebo -treated patients, Met high patients had increased risk of progression (HR = 1.73; median PFS of 6.4 weeks) and approximately double the risk of death (HR = 2.52; median OS of 7.4 weeks) relative to Met low patients (median PFS 11.4 weeks; median OS of 9.2 weeks). Thus, Met expression is a prognostic factor for progression and survival in erlotinib-treated second- or third-line NSCLC patients: Met high patients did worse when treated with erlotinib, while Met low patients did better when treated with erlotinib.

Pattern of progression (growth of target verses new lesion) was comparable between treatment groups and by met status (Table 5).

Table 5: Pattern of progression

| | Met High | | Met Low | |
|---|---|---|---|---|
| | Erlotinib +Placebo | Erlotinib +MetMAb | Erlotinib +Placebo | Erlotinib +MetMAb |
| # pts PD by RECIST | n=21 | n=17 | n=15 | n=15 |
| PD on target lesions | 13 (61.9) | 11 (64.7) | 10 (66.7) | 11 (73.3) |
| PD by new lesions | 12 (57.1) | 9 (52.9) | 6 (40.0) | 7 (46.7) |

Pattern of progression (growth of target verses new lesion) was comparable between treatment groups and by Met status.

Efficacy analysis of ORR is shown in Table 6.

Table 6: Efficacy analysis of ORR.

| | | Erlotinib+Placebo | Erlotinib+MetMAb |
|---|---|---|---|
| | | | |
| | Overall | n=64 | n=64 |
| | | | |
| No. of patients with OR (%) | | | |
| | | 3 (4.7) | 4 (6.3) |
| | | | |
| 95% CI for ORR | | 1.3-12.5 | 2.2-15 |
| Difference in ORR, % (95% CI) | | | |
| | | 1.6 (-6.3-9.4) | |
| | | | |
| p-value | | 1.0 | |
| Met High | | n=30 | n=35 |
| No. of patients with OR (%) | | 1 (3.3) | 3 (8.6) |
| 95% CI for ORR | | 0.2%-16.3 | 2.4-21.5 |
| Difference in ORR, % (95% CI) | | 5.2 (-6.0-16.5) | |
| p-value | | 0.62 | |

Exposure to treatment by Met status is shown in Table 7.

Table 7: Exposure to treatment by Met status

| | High Met | | Low Met | |
|---|---|---|---|---|
| | Erlotinib | Erlotinib | Erlotinib | Erlotinib |
| | +Placebo (n=30) | +MetMAb (n=35) | +Placebo (n=29) | +MetMAb (n=27) |
| MetMAb or Placebo | | | | |
| No. Cycles: median (range) | 2(1-14) | 4(1-18) | 4(1-12) | 2 (1-8) |
| Erlotinib | | | | |
| No. Doses: median (range) | 49.5 (4-290) | 61 (14-338) | 85 (21-239) | 42 (1-152) |
| Patients requiring dose modification (%) | 8(26.7) | 14 (40) | 7(24.1) | 11 (40.7) |

Safety: Treatment with Erlotinib + MetMAb was well-tolerated. Table 8 shows all adverse events, regardless of relationship, with reported frequency greater than 10% observed in the study. With the exception of edema (predominantly Grade 1-2), overall toxicity for Erlotinib + MetMAb was comparable to Erlotinib + Placebo.

Table 8: All adverse events

| n (%) | Met High | | Met Low | |
|---|---|---|---|---|
| | Erlotinib +Placebo (n=30) | Erlotinib +MetMAb (n=35) | Erlotinib +Placebo (n=29) | Erlotinib +MetMAb (n=27) |
| Total | 30 (100) | 35 (100) | 29 (100) | 26 (96.3) |
| Rash | 19 (63.3) | 21 (60.0) | 16 (52.2) | 15 (55.6) |
| Diarrhea | 13 (43.3) | 18 (51.4) | 13 (44.8) | 7 (25.9) |
| Fatigue | 12 (40.0) | 15 (42.9) | 10 (34.5) | 6 (22.2) |
| Decreased appetite | 10 (33.3) | 10 (28.6) | 5 (17.2) | 3 (11.1) |
| Nausea | 8 (26.7) | 10 (28.6) | 8 (27.6) | 9 (33.3) |
| Dyspnea | 8 (26.7) | 7 (20.0) | 4 (13.8) | 4 (14.8) |
| Cough | 6 (20.0) | 6 (17.1) | 5 (17.2) | 3 (11.1) |
| Dermititis acneform | 3 (10.0) | 5 (14.3) | 6 (20.7) | 5 (18.5) |
| Dry skin | 5 (16.7) | 5 (14.3) | 5 (17.2) | 2 (7.4) |
| Peripheral edema | 2 (6.7) | 7 (20) | 2 (6.9) | 5 (18.5) |
| Anemia | 5 (16.7) | 4 (11.4) | 3 (10.3) | 3 (11.1) |

Table 9 shows all grade 3-5 adverse events (frequency > 5%) observed in the study. There were no grade 5 events. Rash, diarrhea, and fatigue were comparable between treatment arms in both Met high and Met low subpopulations. The incidence of Grade ≥3 adverse events was similar in ME v. PE in the Met high group (54% v. 53%); however, incidence of Grade ≥3 adverse events was higher in the ME arm in the Met low group (52%vs 35% in the PE arm).

Table 9: Grade 3-5 adverse events

| n (%) | Met high | | Met low | |
|---|---|---|---|---|
| | Erlotinib +Placebo (n=30) | Erlotinib +MetMAb (n=35) | Erlotinib +Placebo (n=29) | Erlotinib +MetMAb (n=27) |
| Total | 16 (53.3) | 19 (54.3) | 10 (34.5) | 14 (51.9) |
| Rash | 1 (3.3) | 3 (8.6) | 1 (3.4) | 1 (3.7) |
| Diarrhea | 2 (6.7) | 4 (11.4) | 1 (3.4) | 1 (3.7) |
| Pneumonia | 0 | 1 (2.9) | 1 (3.4) | 4 (14.8) |
| Fatigue | 1 (3.3) | 3 (8.6) | 1 (3.4) | 2 (7.4) |
| Pulmonary embolism | 1 (3.3) | 2 (5.7) | 0 | 2 (7.4) |

A summary of safety is shown in Table 10.

Table 10: Summary of safety.

| n (%) | Met High | | Met Low | |
|---|---|---|---|---|
| | Erlotinib +Placebo (n=30) | Erlotinib +MetMAb (n=35) | Erlotinib + Placebo (n=29) | Erlotinib +MetMAb (n=27) |
| Any AEs | 30 (100) | 35 (100) | 29 (100) | 26 (96.3) |
| Grade ≥3 AEs | 16 (53.3) | 19 (54.3) | 10 (34.5) | 14 (51.9) |
| SAEs | 11 (36.7) | 14 (40) | 6 (20.7) | 11 (40.7) |
| AEs leading to treatment discontinuation* | 3 (10) | 8 (22.9) | 0 | 1 (3.7) |
| AEs leading to death** | 3 (10) | 1 (2.9) | 0 | 4 (14.8) |
| *For patients in the Erlotinib+MetMAb arm who were Met High: aspiration pneumonia, obstructive hernia, hypoxia, cerebral infarction, esophageal stenosis, fatigue, NOS, and nail toxicity. **AEs leading to death for those who were Met Low: pneumonia, pulmonary embolism, hemoptysis, NSCLC | | | | |

## Conclusions

- Anti-c-met antibody MetMAb was a selective and potent inhibitor of the Met receptor.
- Met high expression was associated with a worse outcome in placebo-treated patients.
- Treatment with the combination of erlotinib and MetMAb benefited patients with Met High NSCLC.
- The poorer outcomes for patients with Met Low NSCLC treated with erlotinib + MetMAb cannot be explained by adverse events.
- Erlotinib + MetMab was well-tolerated and there were no new significant safety findings.
- Results for the overall population verses patients with Met High NSCLC highlight the importance of using a diagnostic.

### *Final Analysis based on data cut off date of on or before November 15, 2010*

128 patients with second or third line NSCLC were enrolled and randomized at 25 global sites from March 2009 to March 2010 to: (a) MetMAb (15mg/kg IV q3wks) plus erlotinib treatment (interchangeably termed "ME") (n=64) or (b) placebo plus erlotinib treatment (interchangeably termed "PE") (n=64). The data cut-off date used in this analysis was November 15, 2010.

Patient disposition is shown in Table 11.

Table 11: Patient Disposition

| No. of patients (%) | Met Diagnostic-Positive | | Met Diagnostic-Negative | | All patients (n=137) |
|---|---|---|---|---|---|
| | Placebo+ erlotinib (n=31) | MetMAb+ erlotinib (n=35) | Placebo+ erlotinib (n=31) | MetMAb+ erlotinib (n=31) | |
| Disease progression | 23 (74.2) | 18 (51.4) | 22 (71.0) | 22 (71.0) | 92 (67.2) |
| Adverse event | 2 (6.5) | 7 (20.0) | 1 (3.2) | 1 (3.2) | 11 (8.0) |
| Death | 4 (12.9) | 0 | 1 (3.2) | 2 (6.5) | 7 (5.1) |
| Physician decision | 1 (3.2) | 2 (5.7) | 5 (16.1) | 3 (9.7) | 12 (8.8) |
| Patient decision | 1 (3.2) | 3 (8.6) | 1 (3.2) | 1 (3.2) | 6 (4.4) |

(continued)

| No. of patients (%) | Met Diagnostic-Positive | | Met Diagnostic-Negative | | All patients (n=137) |
|---|---|---|---|---|---|
| | Placebo+ erlotinib (n=31) | MetMAb+ erlotinib (n=35) | Placebo+ erlotinib (n=31) | MetMAb+ erlotinib (n=31) | |
| Sponsor decision | 0 | 0 | 1 (3.2) | 0 | 1 (0.7) |

Patient demographics are shown in Table 12.

Table 12: Patient Demographics

| | Erlotinib+Placebo n=64 (%) | Erlotinib+MetMAb n=64 (%) |
|---|---|---|
| Median age (range) | 62 (42-83) | 66 (30-83) |
| Sex: male, n (%) | 39 (60.9) | 36 (56.3) |
| Race: White, n (%) | 5 (90.6) | 56 (87.5) |
| ECOG: 0/1, n (%) | 62 (96.9) | 60 (93.8) |
| Non-squamous, n (%) | 48 (75) | 49 (76.6) |
| Squamous, n (%) | 16 (25) | 15 (23.4) |
| Never smoker, n (%) | 8 (12.5) | 10 (15.6) |
| Met Dx pos*, n (%) | 30 (50.8) | 35 (56.5) |
| Kras mutant**, n (%) | 13 (23.2) | 13 (23.2) |
| EGFR mutant**, n (%) | 6 (10.7) | 7 (12.5) |
| * Of 121 patients with evaluable tissue samples<br>** Of 112 patients with evaluable tissue samples. | | |

Baseline characteristics were well-balanced in the overall (ITT) population including prevalence of patients having Met diagnostic positive tumors (51%/56.5%; PE/ME), Kras mutation (23%/23%) and EGFR mutation (11%/12.5%). Tissue was evaluable for Met IHC analysis in 121 patients (95% of patients), and for EGFR and KRAS mutations in 112 patients.
Baseline characteristics by Met status are shown in Table 13.

Table 13: Baseline characteristics by Met status.

| | ITT | | Met Dx Negative | | Met Dx Positive | |
|---|---|---|---|---|---|---|
| | Placebo + erlotinib (n=68) | MetMAb + erlotinib (n=69) | Placebo + erlotinib (n=31) | MetMAb + erlotinib (n=31) | Placebo + erlotinib (n=31) | MetMAb + erlotinib (n=35) |
| Median age (range) | 63 (42-83) | 64 (30-83) | 61 (42-83) | 63 (45-82) | 64 (44-82) | 66 (30-83) |
| Sex: Male | 62% | 58% | 55% | 65% | 65% | 51% |
| Race: White | 90% | 88% | 90% | 87% | 90% | 91% |
| ECOG PS: 0/1 | 97% | 94% | 100% | 90% | 94% | 97% |
| Squamous | 29% | 29% | 39% | 45% | 16% | 14% |
| Never smoker | 12% | 15% | 3% | 7% | 19% | 20% |
| Met Dx positive* | 50% | 53% | 0% | 0% | 100% | 100% |

(continued)

|  | ITT | | Met Dx Negative | | Met Dx Positive | |
|---|---|---|---|---|---|---|
|  | Placebo + erlotinib (n=68) | MetMAb + erlotinib (n=69) | Placebo + erlotinib (n=31) | MetMAb + erlotinib (n=31) | Placebo + erlotinib (n=31) | MetMAb + erlotinib (n=35) |
| KRAS mutant** | 23% | 23% | 25% | 24% | 23% | 23% |
| EGFR mutant** | 11% | 13% | 14% | 0% | 8% | 23% |
| *Of 128 patients with evaluable tissue samples<br>**Of 112 patients with evaluable tissue samples | | | | | | |

In this study, tissue was obtained from 100% of patients. 95% of patients had adequate tissue for evaluation of Met by IDC. 54% of patients had "Met high" NSCLC.

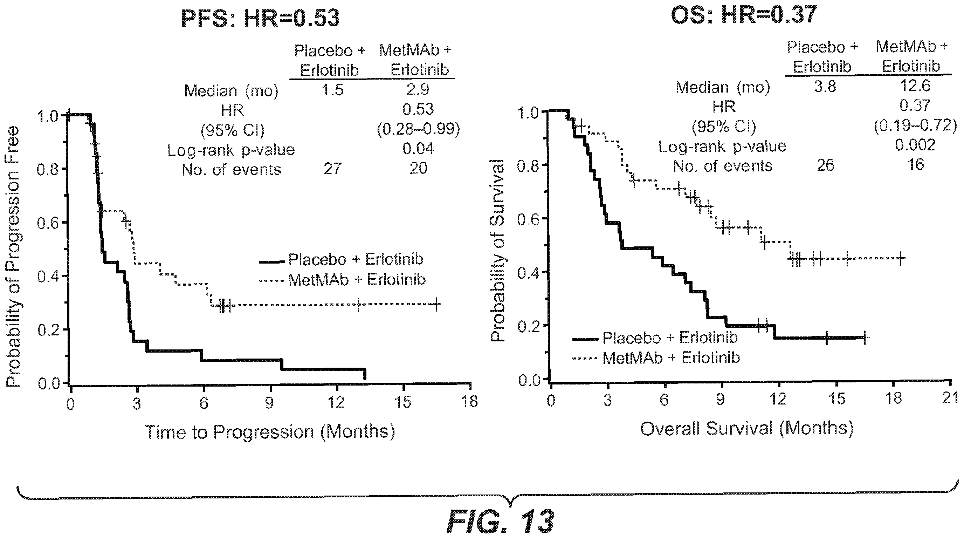

Treatment with MetMAb and erlotinib provided a statistically significant and clinically meaningful benefit to patients with Met diagnostic positive NSCLC (Figure 13). Both a PFS benefit (Hazard ratio (HR) 0.53; 95% CI 0.28-0.99; p=0.04,) and an OS benefit (HR .37; 95% CI 0.19-0.72; p=0.002) were observed in the Met diagnostic positive patients treated with MetMAb plus erlotinib. Early and sustained separation of the curves was observed. The addition of MetMAb to erlotinib resulted in a near three-fold reduction in the risk of death. Median PFS in patients treated with MetMAb + erlotinib (ME) was 2.9 months, verses 1.5 months in patients treated with placebo+ erlotinib (PE); median OS in patients treated with MetMAb + erlotinib was 12.6 months verses 3.8 months in patients treated with placebo + erlotinib).

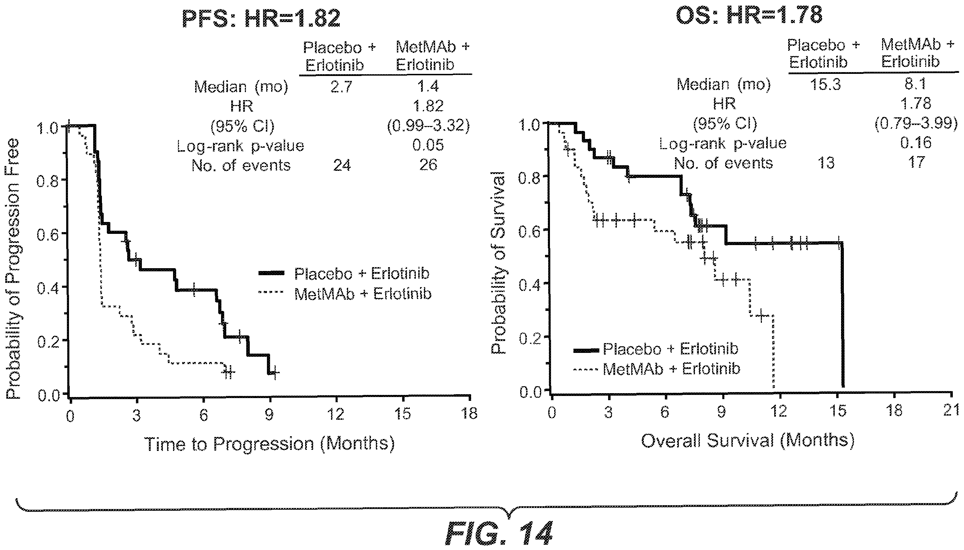

Met diagnostic negative NSCLC patients did not benefit from treatment with MetMAb + erlotinib (Figure 14). MetMAb increased the risk of progression and death in Met low NSCLC patient verses treatment with erlotinib and placebo: both median PFS (HR 1.82; 95% CI 0.99-3.32; p=0.05) and median OS (HR 1.78; 95% CI 0.79 - 3.99; p=0.16) were worse in the MetMAb + erlotinib cohort. ~70 percent of Met low patients treated with MetMAb and erlotinib progressed by the first assessment (CAT scan at week 6).

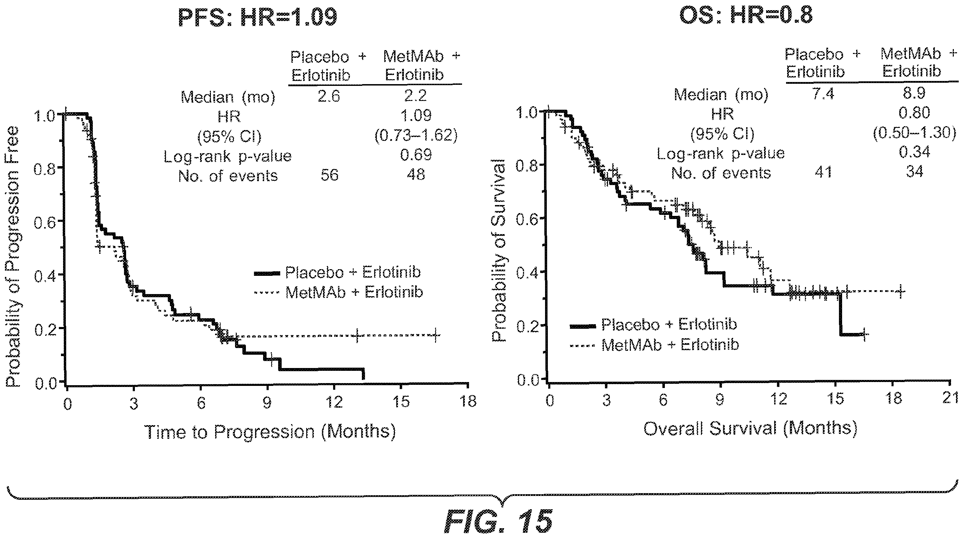

PFS and OS in the overall population are shown in Figure 15. PFS and OS were not significantly different in the MetMAb plus erlotinib and placebo plus erlotinib treatment arms in the overall population. MetMAb plus erlotinib treatment did not show benefit in the overall population relative to placebo plus erlotinib treatment. The Hazard rations for PFS and OS in the overall (interchangeably termed "intent to treat" or ITT) population were 1.09 (95% CI 0.73-1.62; p=0.69) and 0.8 (95% CI 0.5-1.3; p=0.76). Median PFS and median OS were consistent with previously reported findings in similar disease settings.

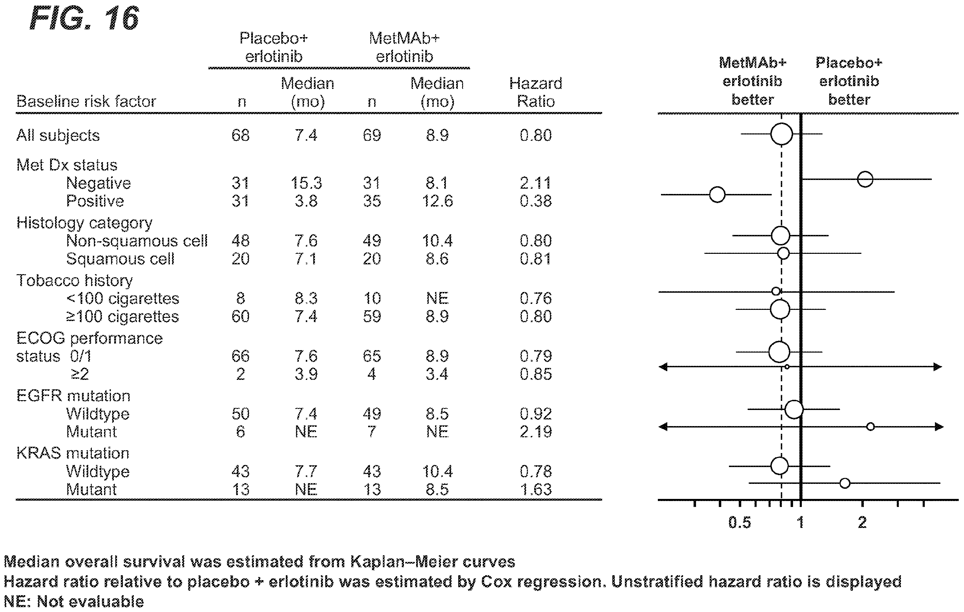

OS was also examined by subgroups (Figure 16). Met diagnostic positive IHC status 3 and 2 patients showed benefit from MetMAb + erlotinib treatment, with status 3 patients showing greater benefit. Met diagnostic negative IHC status 0 and 1 patients did not benefit from MetMAb plus erlotinib treatment, and status 0 patients did worse on MetMAb + erlotinib than status 1 patients. Selective benefit of MetMAb + erlotinib treatment was not observed in other subgroups, including: histology category (non-squamous verses squamous cell), tobacco history, ECOGCC, EGFR mutation or Kras mutation.

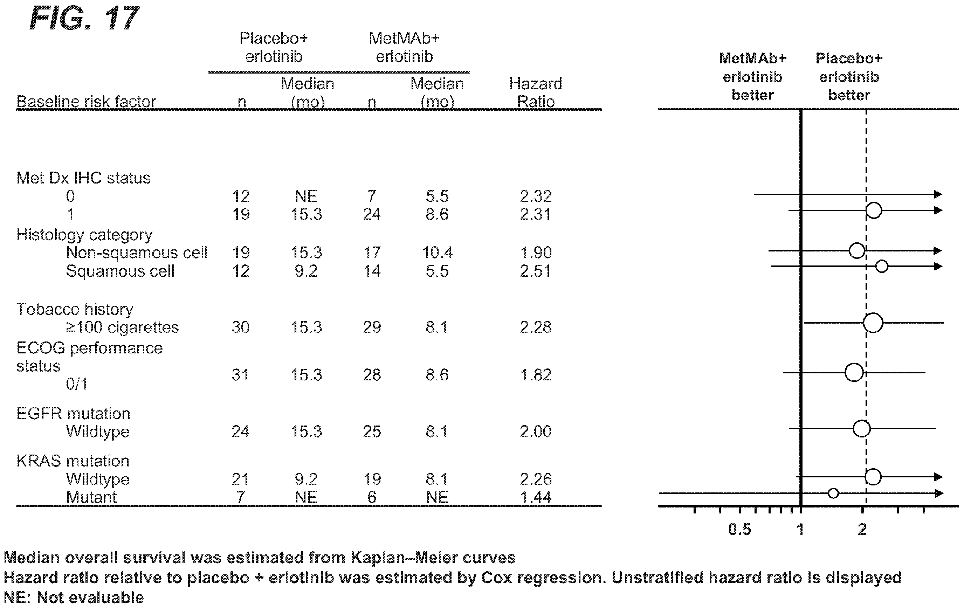

Figure 17 shows subgroup analysis of OS in Met Diagnostic negative patients.

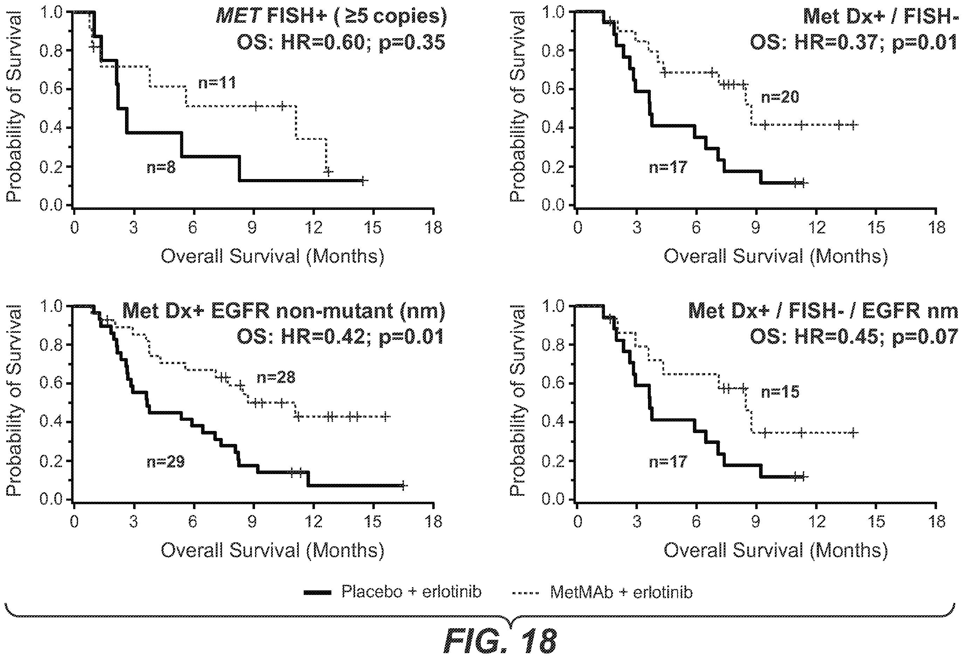

Overall survival was also analyzed in key sub-populations of patients: MET FISH positive patients (defined as greater than or equal to five copies of MET), Met diagnostic positive/*MET* FISH negative, Met diagnostic positive/EGFR wildtype, and Met diagnostic positive/*MET* FISH negative/EGFR wildtype (Figure 18). The benefit of adding MetMAb to erlotinib was not exclusive to *MET* FISH positive patients and was observed in *MET* FISH negative/Met diagnostic positive patients, suggesting that IHC is a more sensitive predictor of benefit from MetMAb. The benefit from MetMAb + erlotinib in Met diagnostic positive patients was not driven by EGFR mutation status.

Key prognostic variables by Met status are shown in Table 14.

Table 14: Key prognostic variables by Met status

|  | Met Positive (n=65) | | Met Negative (n=56) | |
|---|---|---|---|---|
|  | Erlotinib + Placebo (n=30) | Erlotinib +MetMAb (n=35) | Erlotinib +Placebo (n=29) | Erlotinib +MetMAb (n=27) |
| Histopathology (n=128) | | | | |
| Adenocarcinoma | 21 (70.0) | 26 (73.3) | 17(58.6) | 12 (44.4) |

(continued)

| | Met Positive (n=65) | | Met Negative (n=56) | |
|---|---|---|---|---|
| | Erlotinib + Placebo (n=30) | Erlotinib +MetMAb (n=35) | Erlotinib +Placebo (n=29) | Erlotinib +MetMAb (n=27) |
| Histopathology (n=128) | | | | |
| Squamous | 4(13.3) | 5 (14.3) | 10 (34.5) | 10 (37.0) |
| Mutational analyses (n=112) | | | | |
| Kras mutant | 6(23.1) | 7(22.6) | 7 (25.0) | 6 (24.0) |
| EGFR mutant | 2(7.7) | 7(22.6) | 4(14.3) | 0 |

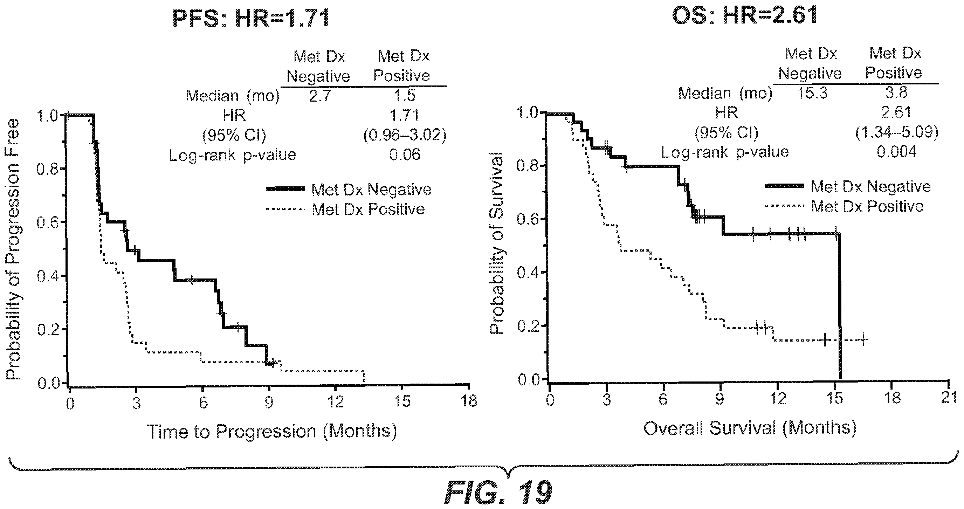

Met expression was prognostic for a worse outcome (Figure 19). In an analysis of the erlotinib + placebo -treated patients, Met diagnostic positive patients had increased risk of progression (HR = 1.7; median PFS of 1.5 months) and increased risk of death (HR = 3.8; median OS of 3.8 months) relative to Met diagnostic negative patients (median PFS 2.7 months; median OS of 15.3 months). Thus, Met expression is a prognostic factor for progression and survival in erlotinib-treated second- or third-line NSCLC patients: Met diagnostic positive patients did worse when treated with erlotinib, while Met diagnostic negative patients did better when treated with erlotinib.

Safety: Treatment with Erlotinib + MetMAb was well-tolerated. Safety data is summarized in Table 15.

Table 15: Summary of safety data.

| | Met Diagnostic Positive | | Met Diagnostic Negative | |
|---|---|---|---|---|
| No. of patients (%) | Placebo + erlotinib (n=31) | MetMAb + erlotinib (n=35) | Placebo + erlotinib (n=31) | MetMAb + erlotinib (n=31) |
| Any adverse event | 31 (100) | 35 (100) | 31 (100) | 31 (100) |
| Grade ≥3 adverse event | 17 (54.8) | 20(57.1) | 13(41.9) | 17 (54.8) |
| Serious adverse event | 11 (35.5) | 15 (42.9) | 9 (29.0) | 13 (41.9) |
| Adverse events leading to MetMAb/placebo discontinuation | 2 (6.5) | 8 (22.9) | 0 | 2 (6.5) |
| Adverse events leading to death | 4(12.9) | 1 (2.9) | 0 | 3(9.7) |

Addition of MetMAb did not substantially increased frequency (by greater than or equal to 10) of any specific adverse event in both diagnostic populations, with the exception of peripheral edema, which was higher in the MetMAb treated arms in both Met diagnostic positive and diagnostic negative populations. MetMAb treatment did not add substantial new toxicities nor exacerbate known erlotinib toxicities regardless of Met diagnostic status, although a higher proportion of Met diagnostic positive patients on MetMAb + erlotinib discontinued due to adverse events and a higher proportion of Met diagnostic negative patients on MetMAb + erlotinib had significant adverse events and Grade 3 or higher adverse events.

Tumor growth and disease progression: The rate of tumor growth was not significantly different between treatment arms of by Met status at Cycle 2, by which time most of the PFS events had occurred.

The overall survival treatment effect of MetMAb + erlotinib treatment was also evaluated in patients using different IHC scoring schemes to define the patients who were scored as "met positive":

- A less stringent cutoff of greater than or equal to 10% of tumor cells with an IHC staining intensity of moderate (2+) or high (3+) ("10% Met diagnostic positive")
- A more stringent cutoff of greater than or equal to 90% of tumor cells with an IHC staining intensity of moderate (2+) or high (3+) ("90% Met diagnostic positive").

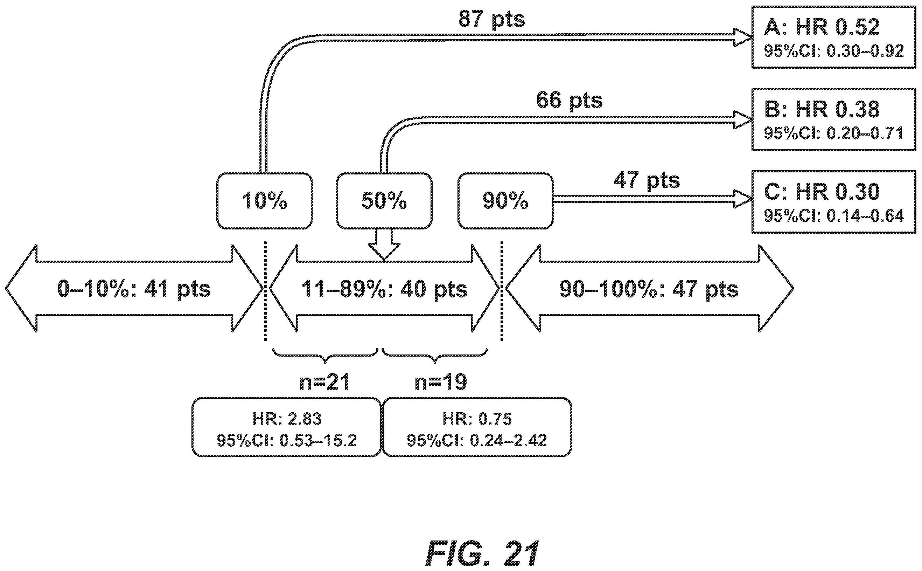

Estimates of the treatment effect in patient subsets were expressed as hazard ratios using an unstratified Cox model, including 95% confidence interval. The results of this analysis are shown in Figure 21. When using 10% as cutoff, the HR in patients selected as 10% Met diagnostic positive (n=87) was 0.52, with 95%CI of 0.30, 0.92. When using 50% as cutoff (i.e., greater than or equal to 50% of tumor cells with an IHC staining intensity of moderate (2+) or high (3+)), the HR in patients selected as Met diagnostic positive (n=66) was 0.38, with 95%CI of 0.20, 0.71. When using 90% as cutoff, the HR in patients selected as 90% Met diagnostic positive (n=47) was 0.30, with 95%CI of 0.14, 0.64. In patients who were selected as (a) 10% Met diagnostic positive and (b) not Met diagnostic positive using the 50% cutoff, (n=21), the HR was 2.83, with 95% CI of 0.53, 15.2. In patients who are selected as (a) Met diagnostic positive using 50% cutoff and (b) not 90% Met diagnostic positive, (n=19), the HR was 0.75, with 95% CI of 0.24, 2.42. This detailed analysis of different IHC scoring schemes supports the use of the definition of Met Diagnostic positive as greater than or equal to 50% of tumor cells staining at a moderate (2+) or high (3+) intensity for Met by IHC.

***Conclusions***

- Anti-c-met antibody MetMAb was a selective and potent inhibitor of the Met receptor.
- Met high expression was associated with a worse outcome in placebo-treated patients.
- Treatment with the combination of erlotinib and MetMAb benefited patients with Met diagnostic positive NSCLC.
- The poorer outcomes for patients with Met diagnostic positive NSCLC treated with erlotinib + MetMAb cannot be explained by adverse events.
- Erlotinib + MetMAb were well-tolerated and there were no new significant safety findings.
- Results for the overall population verses patients with Met diagnostic positive NSCLC highlight the importance of using a diagnostic.

*Exploratory biomarker analyses from OAM4558g: a placebo-controlled Phase II study of erlotinib ±MetMAb in patients with advanced non-small-cell lung cancer (NSCLC)*

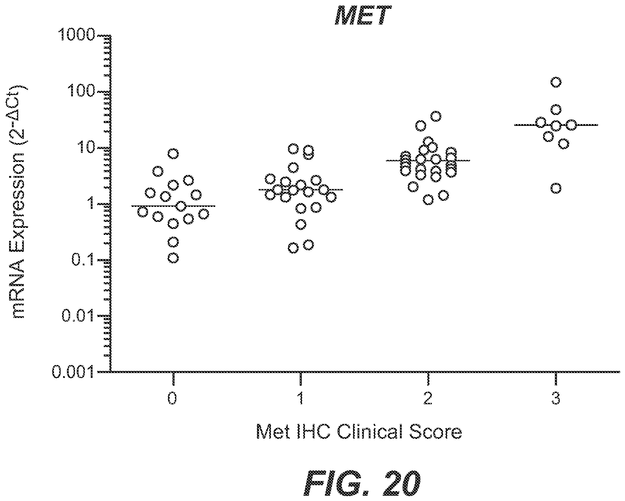

Exploratory tumor biomarkers related to c-Met and/or EGFR signaling were measured by FISH, qRT-PCR or various mutation detection techniques from archival tumor tissue specimens. Plasma HGF levels were measured by ELISA. Analyses with outcome were carried out independent of Met clinical diagnostic status.
***Results:*** Baseline characteristics of patients with evaluable tumor or plasma specimens were generally comparable.
EGFR and KRAS mutations: EGFR and KRAS data were obtained from n=112 (93%) patients, MET exon 14 variants from n=87 (72%) patients and MET N375S snp from n=113 (93%) patients. EGFR mutations predicted for 6/7 objective responses on study and were comparable between treatment arms. KRAS mutations (detected in 23% (26/112) of evaluable specimens) were mutually exclusive with EGFR mutations (detected in 12% (13/112) of evaluable specimens) and did not significantly impact outcome with MetMAb in the ITT or Met diagnostic positive/negative populations.
MET variant data: The MET exon 14 deletion mutation was identified in 1% (n=1) of evaluable specimens. The MET N375S snp was identified in 11% (n=12) of evaluable specimens. Due to the imbalance between treatment arms, outcome analyses were not able to be performed.
MET FISH: FISH data were obtained from n=96 (75%) patients. There was a non-significant trend towards improved outcome in EGFR-wildtype patients that were MET FISH positive, as defined by ≥5 copies of MET/cell, but not a lower thresholds (e.g., ≥4 copies (HR=0.89, p=0.82)). Overall, 74% of FISH positive patients were also Met diagnostic positive. True gene amplification was detected in 8% of patients. However, EGFR-wildtype patients that were Met diagnostic positive and MET FISH negative displayed a trend for improved outcome (OS HR=0.45, p=0.07), indicating that assessing for met gene amplification using FISH potentially missed a large subgroup of patients who may benefit from MetMAb therapy. There was no significant difference in OS in MET and EGFR FISH positive patients.
*MET* and *EGFR* pathway gene analysis by qRT-PCR, and plasma HGF level analysis: mRNA data were obtained from n=67 patients (49%) (Figure 20). A non-statistically significant association of tumor *MET* mRNA levels with the Met IHC clinical score was observed; however, expression of *MET* mRNA or other genes did not independently predict for PFS or OS benefit in the subset of patients with mRNA data (Table 16).

Table1 6. Association of *MET, EGFR, AREG* and *EREG* expression with outcome.

| mRNA | | Placebo+Erlotinib | | MetMAb+Erlotinib | | HR | 95%CI | p-value |
|---|---|---|---|---|---|---|---|---|
| (median) | | n | Median (mo) | n | Median (mo) | | | |
| MET (3.16) | High | 12 | 6.5 | 22 | 8.7 | 0.59 | 0.25-1.4 | 0.23 |
| | Low | 19 | 15.3 | 14 | 11.7 | 1.48 | 0.54-4.1 | 0.45 |
| EGFR (7.75) | High | 13 | 8.3 | 21 | 7.1 | 1.1 | 0.44-2.76 | 0.83 |
| | Low | 18 | 7.1 | 15 | 11.7 | 0.88 | 0.33-2.33 | 0.80 |

(continued)

| mRNA | | Placebo+Erlotinib | | MetMAb+Erlotinib | | HR | 95%CI | p-value |
|---|---|---|---|---|---|---|---|---|
| (median) | | n | Median (mo) | n | Median (mo) | | | |
| AREG (7.11) | High | 13 | 8.3 | 21 | NA | 0.77 | 0.30-1.99 | 0.58 |
| | Low | 18 | 6.9 | 15 | 6.5 | 1.31 | 0.53-3.25 | 0.55 |
| EREG (0.26) | High | 13 | 6.9 | 21 | 10.2 | 0.67 | 0.28-1.63 | 0.37 |
| | Low | 18 | 9.2 | 15 | 8.1 | 1.34 | 0.50-3.60 | 0.57 |

Baseline plasma HGF data were obtained from n=96 patients (70%). A non-significant trend for improved outcome in patients with low HGF protein levels in plasma collected prior to treatment was evident.

*Conclusions:* The data presented herein support Met IHC as the most robust, sensitive and independent predictor of OS benefit from MetMAb treatment. Further investigation of these biomarkers is warranted given the small size of this study.

Although the foregoing invention has been described in some detail by way of illustration and example for purposes of clarity of understanding, the descriptions and examples should not be construed as limiting the scope of the invention.

## Claims

1. A method for identifying a cancer patient who is (a) likely to, or (b) less likely to, respond to treatment with a c-met antagonist comprising the step of determining whether the patient's cancer has (a) a high amount or (b) a low amount of c-met biomarker, wherein the c-met biomarker expression indicates that the patient is (a) likely to respond to treatment with the c-met antagonist, or (b) less likely to respond to treatment with the c-met antagonist, wherein c-met biomarker protein expression is determined in a cancer sample obtained from the patient using immunohistochemistry (IHC) and (a) high c-met biomarker expression is (i) 50% or more of the tumor cells with moderate c-met staining intensity or combined moderate/strong c-met staining intensity but less than 50% of the

65

tumor cells with strong staining intensity (IHC score 2), or (ii) 50% or more of the tumor cells with strong c-met staining intensity (IHC score 3), and (b) low c-met biomarker expression is (i) negative c-met staining, less than 50% of tumor cells with weak or combined weak and moderate c-met staining intensity (IHC score 0), or (ii) 50% or more tumor cells with weak or combined weak and moderate c-met staining intensity but less than 50% tumor cells with moderate or combined moderate and strong c-met staining intensity (IHC score 1),

wherein c-met expression staining intensity is determined relative to c-met staining intensity of control cell pellets, and wherein a control for moderate c-met staining intensity has the c-met staining intensity of cell line A549; a control for strong c-met staining intensity has the c-met staining intensity of cell line H441; a control for negative c-met staining intensity has the c-met staining intensity of cell line H1155; and a control for weak c-met staining intensity has the c-met staining intensity of cell line HEK-293.

2. A method for determining cancer patient prognosis, comprising the step of determining whether the patient's cancer has a high amount of c-met biomarker, wherein the c-met biomarker expression indicates that the patient is likely to have increased overall survival (OS) and/or progression-free survival (PFS) when the patient is treated with a c-met antagonist, wherein c-met biomarker protein expression is determined in a cancer sample obtained from the patient using immunohistochemistry (IHC) and high c-met biomarker expression is (i) 50% or more of the tumor cells with moderate c-met staining intensity or combined moderate/strong c-met staining intensity but less than 50% of the tumor cells with strong staining intensity (IHC score 2), or (ii) 50% or more of the tumor cells with strong c-met staining intensity (IHC score 3),

wherein c-met expression staining intensity is determined relative to c-met staining intensity of control cell pellets, and wherein a control for moderate c-met staining intensity has the c-met staining intensity of cell line A549; a control for strong c-met staining intensity has the c-met staining intensity of cell line H441; a control for negative c-met staining intensity has the c-met staining intensity of cell line H1155; and a control for weak c-met staining intensity has the c-met staining intensity of cell line HEK-293.

3. A method for selecting a therapy for a patient comprising determining expression of a c-met biomarker in a cancer sample obtained from the patient, and selecting a cancer medicament based on the level of expression of the biomarker, wherein the patient is selected for treatment with (i) a c-met antagonist if the cancer sample expresses the biomarker at a high level, or (ii) a cancer medicament other than a c-met antagonist if the cancer sample expresses the biomarker at a low or substantially undetectable level,

wherein c-met biomarker protein expression is determined in a cancer sample from the patient using immunohistochemistry (IHC) and (a) high c-met biomarker expression is (i) 50% or more of the tumor cells with moderate c-met staining intensity or combined moderate/strong c-met staining intensity but less than 50% of the tumor cells with strong staining intensity (IHC score 2), or (ii) 50% or more of the tumor cells with strong c-met staining intensity (IHC score 3), and (b) low c-met biomarker expression is (i) negative c-met staining, less than 50% of tumor cells with weak or combined weak and moderate c-met staining intensity (IHC score 0), or (ii) 50% or more tumor cells with weak or combined weak and moderate c-met staining intensity but less than 50% tumor cells with moderate or combined moderate and strong c-met staining intensity (IHC score 1),

wherein c-met expression staining intensity is determined relative to c-met staining intensity of control cell pellets, and wherein a control for moderate c-met staining intensity has the c-met staining intensity of cell line A549; a control for strong c-met staining intensity has the c-met staining intensity of cell line H441; a control for negative c-met staining intensity has the c-met staining intensity of cell line H1155; and a control for weak c-met staining intensity has the c-met staining intensity of cell line HEK-293.

4. A method for determining c-met biomarker expression, comprising the step of determining c-met biomarker protein expression in a cancer sample obtained from a patient using immunohistochemistry (IHC) with an anti-c-met antibody, comprising determining (a) the percentage of tumor cells in the sample with negative c-met staining intensity, (b) the percentage of tumor cells in the sample with weak c-met staining intensity, (c) the percentage of tumor cells in the sample with moderate c-met staining intensity, and (d) the percentage of tumor cells in the sample with strong c-met staining intensity, wherein c-met biomarker expression has an IHC score of 0 where the sample has negative c-met staining, or less than 50% of tumor cells with weak or combined weak and moderate c-met staining intensity; an IHC score of 1 where the sample has 50% or more tumor cells with weak or combined weak and moderate c-met staining intensity but less than 50% tumor cells with moderate or combined moderate and strong c-met staining intensity; an IHC score of 2 where the sample has 50% or more tumor cells with moderate or combined moderate and strong c-met staining intensity but less than 50% tumor cells with strong staining intensity; and an IHC score of 3 where the sample has 50% or more tumor cells with strong c-met staining intensity,

wherein c-met expression staining intensity is determined relative to c-met staining intensity of control cell pellets, and wherein a control for moderate c-met staining intensity has the c-met staining intensity of cell line A549; a control for strong c-met staining intensity has the c-met staining intensity of cell line H441; a control for negative c-met staining intensity has the c-met staining intensity of cell line H1155; and a control for weak c-met staining intensity has the c-met staining intensity of cell line HEK-293.

5. The method of claim 4, wherein c-met biomarker expression with an IHC score of 2 or 3 indicates that the patient is likely to respond to treatment with a c-met antagonist.

6. The method of claim 4 or 5, comprising the step of determining whether a patient's cancer has a high level of c-met biomarker, wherein high c-met biomarker expression is (i) 50% or more of the tumor cells with moderate c-met staining intensity or combined moderate and strong c-met staining intensity but less than 50% of the tumor cells with strong staining intensity (IHC score 2), or (ii) 50% or more of the tumor cells with strong c-met staining intensity (IHC score 3), wherein c-met expression is detected using a c-met antibody, wherein the c-met biomarker expression indicates that the patient is likely to have increased OS and/or PFS when the patient is treated with a c-met antagonist.

7. The method of any one of claims 1 to 6, wherein the control for moderate c-met staining intensity is cell line A549; the control for strong c-met staining intensity is cell line H441; the control for negative c-met staining intensity is cell line H1155; and the control for weak c-met staining intensity is cell line HEK-293.

8. The method of any one of claims 1, 2, and 6, wherein the patient has greater PFS and/or OS relative to a patient who does not have high c-met biomarker.

9. The method of any one of claims 1 to 8, wherein the sample is obtained (i) prior to treatment with c-met antagonist, (ii) prior to treatment with a cancer medicament, or (iii) after the cancer has metastasized.

10. The method of any one of claims 1-9, wherein the sample is formalin fixed and paraffin embedded.

11. The method of any one of claims 1-10, wherein the c-met IHC is performed using anti-c-met antibody SP44.

12. The method of any one of claims 1-11, wherein the cancer is non-small cell lung cancer, renal cell cancer, pancreatic cancer, gastric carcinoma, bladder cancer, esophageal cancer, mesothelioma, melanoma, breast cancer, thyroid cancer, colorectal cancer, head and neck cancer, osteosarcoma, prostate cancer, or glioblastoma.

13. The method of claim 12, wherein the cancer is non-small cell lung cancer (NSCLC).

14. The method of claim 13, wherein the NSCLC is: second-line or third-line locally advanced or metastatic non-small cell lung cancer; adenocarcinoma; or squamous cell carcinoma.

15. The method of any one of claims 1-3, 5-14, wherein the c-met antagonist is an antagonist anti-c-met antibody.

16. The method of claim 15, wherein the anti-c-met antibody:

(i) comprises a (a) HVR1 comprising sequence GYTFTSYWLH (SEQ ID NO: 1); (b) HVR2 comprising sequence GMIDPSNSDTRFNPNFKD (SEQ ID NO: 2); (c) HVR3-HC comprising sequence ATYRSYVTPLDY (SEQ ID NO: 3); (d) HVR1-LC comprising sequence KSSQSLLYTSSQKNYLA (SEQ ID NO: 4); (e) HVR2-LC comprising sequence WASTRES (SEQ ID NO: 5); and (f) HVR3-LC comprising sequence QQYYAYPWT (SEQ ID NO: 6); or (ii) is monovalent and comprises (a) a first polypeptide comprising a heavy chain, said polypeptide comprising the sequence:

EVQLVESGGGLVQPGGSLRLSCAASGYTFTSYWLHWVRQAPGKGLEWVGMIDPSNSDTR
FNPNFKDRFTISADTSKNTAYLQMNSLRAEDTAVYYCATYRSYVTPLDYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYR

VVSVLTVLHQDWLNGKEYKCKVSNKALPAPIEKTISKAKGQPREPQVYTLPPSREEMTKNQ
VSLSCAVKGFYPSDIAVEWESNGQPENNYKTTPPVLDSDGSFFLVSKLTVDKSRWQQGNV
FSCSVMHEALHNHYTQKSLSLSPGK (SEQ ID NO: 11);

(b) a second polypeptide comprising a light chain, the polypeptide comprising the sequence

DIQMTQSPSSLSASVGDRVTITCKSSQSLLYTSSQKNYLAWYQQKPGKAPKLLIYWASTRE
SGVPSRFSGSGSGTDFTLTISSLQPEDFATYYCQQYYAYPWTFGQGTKVEIKRTVAAPSVFI
FPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 12);

and a third polypeptide comprising a Fc sequence, the polypeptide comprising the sequence

DKTHTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKGQ
PREPQVYTLPPSREEMTKNQVSLWCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 13),

wherein the heavy chain variable domain and the light chain variable domain are present as a complex and form a single antigen binding arm, wherein the first and second Fc polypeptides are present in a complex and form a Fc region that increases stability of said antibody fragment compared to a Fab molecule comprising said antigen binding arm.

17. The method of any one of claims 1-3, 5-15, wherein the c-met antagonist is one or more of crizotinib, tivantinib, carbozantinib, MGCD-265, ficlatuzumab, humanized TAK-701, rilotumumab, foretinib, h224G11, DN-30, MK-2461, E7050, MK-8033, PF-4217903, AMG208, JNJ-38877605, EMD1204831, INC-280, LY-2801653, SGX-126, RP1040, LY2875358, BAY-853474, and/or LA480.

18. The method of any one of claims 1-3, 5-17, wherein treatment is in combination with treatment with an EGFR antagonist.

19. The method of claim 18, wherein the EGFR antagonist is erlotinib.

20. The method of any one of claims 1-3, 5-17, wherein the c-met antagonist is onartuzumab and treatment further comprises treatment with erlotinib, or the c-met antagonist is crizotinib, tivantinib, carbozantinib, MGCD-265, ficlatuzumab, humanized TAK-701, or foretinib, and treatment further comprises treatment with erlotinib.

21. A c-met antagonist for use in a method for treating a patient with non-small cell lung cancer (NSCLC) where the patient's cancer has been found to have a high amount of a c-met biomarker, wherein c-met biomarker protein expression is determined in a cancer sample obtained from the patient using immunohistochemistry (IHC) and high c-met biomarker expression is (i) 50% or more of the tumor cells with moderate c-met staining intensity or combined moderate/strong c-met staining intensity but less than 50% of the tumor cells with strong staining intensity (IHC score 2), or (ii) 50% or more of the tumor cells with strong c-met staining intensity (IHC score 3), wherein c-met expression staining intensity is determined relative to c-met staining intensity of control cell pellets, and wherein a control for moderate c-met staining intensity has the c-met staining intensity of cell line A549; a control for strong c-met staining intensity has the c-met staining intensity of cell line H441; a control for negative c-met staining intensity has the c-met staining intensity of cell line H1155; and a control for weak c-met staining intensity has the c-met staining intensity of cell line HEK-293.

22. The c-met antagonist for use of claim 21, wherein the c-met antagonist is (i) an anti-c-met antibody, or (ii) is onartuzumab.

**23.** The c-met antagonist for use of claim 21 or 22, wherein the patient has greater PFS and/or OS relative to a patient who does not have high c-met biomarker.

**24.** The c-met antagonist for use of any one of claims 21 to 23, wherein the control for moderate c-met staining intensity is cell line A549; the control for strong c-met staining intensity is cell line H441; the control for negative c-met staining intensity is cell line H1155; and the control for weak c-met staining intensity is cell line HEK-293.

**25.** The c-met antagonist for use of any one of claims 21 to 24, wherein the NSCLC is second-line or third-line locally advanced or metastatic non-small cell lung cancer, adenocarcinoma, or squamous cell carcinoma.

**26.** The c-met antagonist for use of claim 21 or 22, wherein the patient is treated with a combination of anti-c-met antibody and an EGFR antagonist.

**27.** The c-met antagonist for use of claim 26, wherein the EGFR antagonist is erlotinib.

**28.** The c-met antagonist for use of claim 27, wherein the patient is treated with (a) onartuzumab at a dose of about 15 mg/kg every three weeks; and (b) erlotinib (N-(3-ethynylphenyl)-6,7-bis(2-methoxyethoxy)-4-quinazolinamine) at a dose of 150 mg, each day of a three week cycle.

**29.** Use of a diagnostic kit comprising one or more reagents for determining expression of a c-met biomarker in a sample from a NSCLC patient, and instructions to use the kit **characterized in that** the use of the kit is for selecting (a) a c-met antagonist to treat the NSCLC patient if a high amount of c-met biomarker is determined or (b) a cancer medicament other than a c-met antagonist to treat the NSCLC patient if a low or substantially undetectable amount of c-met biomarker is determined, wherein c-met biomarker protein expression is determined in a cancer sample from the patient using immunohistochemistry (IHC) and (a) high c-met biomarker expression is (i) 50% or more of the tumor cells with moderate c-met staining intensity or combined moderate/strong c-met staining intensity but less than 50% of the tumor cells with strong staining intensity (IHC score 2), or (ii) 50% or more of the tumor cells with strong c-met staining intensity (IHC score 3), and (b) low c-met biomarker expression is (i) negative c-met staining, less than 50% of tumor cells with weak or combined weak and moderate c-met staining intensity (IHC score 0), or (ii) 50% or more tumor cells with weak or combined weak and moderate c-met staining intensity but less than 50% tumor cells with moderate or combined moderate and strong c-met staining intensity (IHC score 1), wherein c-met expression staining intensity is determined relative to c-met staining intensity of control cell pellets, and wherein a control for moderate c-met staining intensity has the c-met staining intensity of cell line A549; a control for strong c-met staining intensity has the c-met staining intensity of cell line H441; a control for negative c-met staining intensity has the c-met staining intensity of cell line H1155; and a control for weak c-met staining intensity has the c-met staining intensity of cell line HEK-293.

**Patentansprüche**

**1.** Verfahren zum Identifizieren eines Krebspatienten, der (a) wahrscheinlich oder (b) weniger wahrscheinlich auf eine Behandlung mit einem c-met-Antagonisten reagiert, umfassend den Schritt des Bestimmens, ob der Krebs des Patienten (a) einen hohen Wert oder (b) einen niedrigen Wert des c-met-Biomarkers aufweist, wobei die Expression des c-met-Biomarkers angibt, dass der Patient (a) wahrscheinlich auf die Behandlung mit dem c-met-Antagonisten reagieren wird oder (b) weniger wahrscheinlich auf die Behandlung mit dem c-met-Antagonisten reagieren wird, wobei die c-met-Biomarker-Proteinexpression in einer Krebsprobe, die aus dem Patienten erhalten wurde, unter Verwendung von Immunhistochemie (IHC) bestimmt wird und (a) eine starke c-met-Biomarker-Expression (i) 50 % oder mehr der Tumorzellen mit mittelmäßiger c-met-Färbungsintensität oder kombinierter mittelmäßiger/starker c-met-Färbungsintensität, aber weniger als 50 % der Tumorzellen mit starker Färbungsintensität (IHC-Bewertung 2) oder (ii) 50 % oder mehr der Tumorzellen mit starker c-met-Färbungsintensität (IHC-Bewertung 3) ist und (b) die schwache c-met-Biomarker-Expression (i) eine negative c-met-Färbung, weniger als 50 % der Tumorzellen mit schwacher oder kombinierter schwacher und mittelmäßiger c-met-Färbungsintensität (IHC-Bewertung 0) oder (ii) 50 % oder mehr der Tumorzellen mit schwacher oder kombinierter schwacher und mittelmäßiger c-met-Färbungsintensität, aber weniger als 50 % der Tumorzellen mit mittelmäßiger oder kombinierter mittelmäßiger und starker c-met-Färbungsintensität (IHC-Bewertung 1) ist, wobei die c-met-Expressionsfärbungsintensität im Vergleich zur c-met-Expressionsfärbungsintensität von Kontrollzellpellets bestimmt wird und wobei eine Kontrolle für mittelmäßige c-met-Färbungsintensität die c-met-Färbungsintensität von Zelllinie A549 aufweist; eine Kontrolle für eine starke c-met-Färbungsintensität die c-met-Färbungsintensität von Zelllinie H441 aufweist; eine Kontrolle für eine negative c-met-Färbungsintensität die c-met-Färbungsintensität von Zelllinie H1155 aufweist; eine Kontrolle für eine schwache c-met-Färbungsintensität die c-met-Färbungsintensität von Zelllinie HEK-293 aufweist.

2. Verfahren zum Bestimmen der Prognose eines Krebspatienten, umfassend den Schritt des Bestimmens, ob die Krebserkrankung des Patienten eine große Menge eines c-met-Biomarkers aufweist, wobei die c-met-Biomarkerexpression angibt, dass der Patient wahrscheinlich eine verlängerte gesamte Überlebensdauer (OS) und/oder progressionsfreie Überlebensdauer (PFS) hat, wenn der Patient mit einem c-met-Antagonisten behandelt wird, wobei die c-met-Biomarker-Proteinexpression in einer Krebsprobe, die aus dem Patienten erhalten wurde, unter Verwendung von Immunhistochemie (IHC) bestimmt wird und eine starke c-met-Biomarkerexpression (i) 50 % oder mehr der Tumorzellen mit mittelmäßiger c-met-Färbungsintensität oder einer kombinierten mittelmäßigen/starken c-met-Färbungsintensität, aber weniger als 50 % der Tumorzellen mit starker Färbungsintensität (IHC-Bewertung 2) oder (ii) 50 % oder mehr der Tumorzellen mit starker c-met-Färbungsintensität (IHC-Bewertung 3) ist, wobei die c-met-Expressionsfärbungsintensität im Vergleich zu der c-met-Färbungsintensität von Kontrollzellpellets bestimmt wird und wobei eine Kontrolle für eine mittelmäßige c-met-Färbungsintensität die c-met-Färbungsintensität von Zelllinie A549 aufweist; eine Kontrolle für eine starke c-met-Färbungsintensität die c-met-Färbungsintensität von Zelllinie H441 aufweist; eine Kontrolle für eine negative c-met-Färbungsintensität die c-met-Färbungsintensität von Zelllinie H1155 aufweist; und eine Kontrolle für eine schwache c-met-Färbungsintensität die c-met-Färbungsintensität von Zelllinie HEK-293 aufweist.

3. Verfahren zum Auswählen einer Therapie für einen Patienten, umfassend das Bestimmen der Expression eines c-met-Biomarkers in einer Krebsprobe, die aus dem Patienten entnommen wurde, und Auswählen eines Krebsmedikaments basierend auf dem Expressionsausmaß des Biomarkers, wobei der Patient für eine Behandlung mit (i) einem c-met-Antagonisten, falls der Biomarker in der Krebsprobe in einem starken Ausmaß exprimiert wird, oder (ii) einem Krebsmedikament ausgewählt wird, das kein c-met-Antagonist ist, falls der Biomarker in geringem oder im Wesentlichen nicht detektierbarem Ausmaß in der Krebsprobe exprimiert wird, wobei die c-met-Biomarker-Proteinexpression in einer Krebsprobe aus dem Patienten unter Verwendung von Immunhistochemie (IHC) bestimmt wird und (a) eine starke c-met-Biomarker-Expression (i) 50 % oder mehr der Tumorzellen mit mittelmäßiger c-met-Färbungsintensität oder kombinierter mittelmäßiger/starker c-met-Färbungsintensität, aber weniger als 50 % der Tumorzellen mit starker Färbungsintensität (IHC-Bewertung 2) oder (ii) 50 % oder mehr der Tumorzellen mit starker c-met-Färbungsintensität (IHC-Bewertung 3) ist und (b) die schwache c-met-Biomarker-Expression (i) eine negative c-met-Färbung, weniger als 50 % der Tumorzellen mit schwacher oder kombinierter schwacher und mittelmäßiger c-met-Färbungsintensität (IHC-Bewertung 0) oder (ii) 50 % oder mehr der Tumorzellen mit mittelmäßiger oder kombinierter mittelmäßiger und starker c-met-Färbungsintensität (IHC-Bewertung 1) ist, wobei die c-met-Expressionsfärbungsintensität im Vergleich zu der c-met-Färbungsintensität von Kontrollzellpellets bestimmt wird und wobei eine Kontrolle für eine mittelmäßige c-met-Färbungsintensität die c-met-Färbungsintensität von Zelllinie A549 aufweist; eine Kontrolle für eine starke c-met-Färbungsintensität die c-met-Färbungsintensität von Zelllinie H441 aufweist; eine Kontrolle für eine negative c-met-Färbungsintensität die c-met-Färbungsintensität von Zelllinie H1155 aufweist; und eine Kontrolle für eine schwache c-met-Färbungsintensität die c-met-Färbungsintensität von Zelllinie HEK-293 aufweist.

4. Verfahren zum Bestimmen der c-met-Biomarkerexpression, umfassend den Schritt des Bestimmens der c-met-Biomarkerexpression in einer Krebsprobe, die aus einem Patienten entnommen wurde, unter Verwendung von Immunhistochemie (IHC) mit einem Anti-c-met-Antikörper, umfassend das Bestimmen (a) des Prozentanteils von Tumorzellen in der Probe mit negativer c-met-Färbungsintensität, (b) des Prozentanteils von Tumorzellen in der Probe mit schwacher c-met-Färbungsintensität, (c) des Prozentanteils von Tumorzellen in der Probe mit mittelmäßiger c-met-Färbungsintensität und (d) des Prozentsatzes von Tumorzellen in der Probe mit starker c-met-Färbungsintensität, wobei die c-met-Biomarkerexpression eine IHC-Bewertung von 0 aufweist, wenn die Probe eine negative c-met-Färbung aufweist oder weniger als 50 % der Tumorzellen eine schwache oder kombinierte schwache und mittelmäßige c-met-Färbungsintensität aufweisen; eine IHC-Bewertung von 1 aufweist, wenn die Probe 50 % oder mehr Tumorzellen mit schwacher oder kombinierter schwacher und mittelmäßiger c-met-Färbungsintensität, aber weniger als 50 % Tumorzellen mit einer mittelmäßigen oder kombinierten mittelmäßigen und starken c-met-Färbungsintensität aufweist; eine IHC-Bewertung von 2 aufweist, wenn die Probe 50 % oder mehr Tumorzellen mit mittelmäßiger oder kombinierter mittelmäßiger und starker c-met-Färbungsintensität, aber weniger als 50 % Tumorzellen mit einer starken c-met-Färbungsintensität aufweist; eine IHC-Bewertung von 3 aufweist, wenn die Probe 50 % oder mehr Tumorzellen mit starker Färbungsintensität aufweist;
wobei die c-met-Expressionsfärbungsintensität im Vergleich zu der c-met-Färbungsintensität von Kontrollzellpellets bestimmt wird und
wobei eine Kontrolle für eine mittelmäßige c-met-Färbungsintensität die c-met-Färbungsintensität von Zelllinie A549 aufweist; eine Kontrolle für eine starke c-met-Färbungsintensität die c-met-Färbungsintensität von Zelllinie H441 aufweist; eine Kontrolle für eine negative c-met-Färbungsintensität die c-met-Färbungsintensität von Zelllinie H1155 aufweist; und eine Kontrolle für eine schwache c-met-Färbungsintensität die c-met-Färbungsintensität von Zelllinie HEK-293 aufweist.

5. Verfahren nach Anspruch 4, wobei die c-met-Biomarkerexpression mit einer IHC-Bewertung von 2 oder 3 angibt, dass der Patient wahrscheinlich auf die Behandlung mit einem c-met-Antagonisten ansprechen wird.

6. Verfahren nach Anspruch 4 oder 5, umfassend den Schritt des Bestimmens, ob die Krebserkrankung eines Patienten einen hohen Wert eines c-met-Biomarkers aufweist, wobei eine starke c-met-Biomarkerexpression (i) 50 % oder mehr der Tumorzellen mit mittelmäßiger c-met-Färbungsintensität oder kombinierter mittelmäßiger und starker c-met-Färbungsintensität, aber weniger als 50 % der Tumorzellen mit starker Färbungsintensität (IHC-Bewertung 2) oder (ii) 50 % oder mehr der Tumorzellen mit starker Färbungsintensität (IHC-Bewertung 3) ist, wobei die c-met-Expression unter Verwendung eines c-met-Antikörpers detektiert wird, wobei die c-met-Biomarkerexpression angibt, dass ein Patient wahrscheinlich eine längere OS und/oder PFS aufweist, wenn der Patient mit einem c-met-Antagonisten behandelt wird.

7. Verfahren nach einem der Ansprüche 1 bis 6, wobei die Kontrolle für eine mittelmäßige c-met-Färbungsintensität Zelllinie A549 ist; die Kontrolle für eine starke c-met-Färbungsintensität Zelllinie H441 ist; die Kontrolle für eine negative c-met-Färbungsintensität Zelllinie H1155 ist; und die Kontrolle für eine schwache c-met-Färbungsintensität Zelllinie HEK-293 ist.

8. Verfahren nach einem der Ansprüche 1, 2 und 6, wobei der Patient, verglichen mit einem Patienten, der keinen erhöhten c-met-Biomarkerwert aufweist, eine längere PFS und/oder OS aufweist.

9. Verfahren nach einem der Ansprüche 1 bis 8, wobei die Probe (i) vor der Behandlung mit dem c-met-Antagonisten, (ii) vor der Behandlung mit einem Krebsmedikament erhalten wird oder (iii) erhalten wird, nachdem der Krebs metastasiert ist.

10. Verfahren nach einem der Ansprüche 1-9, wobei die Probe mit Formalin fixiert und in Paraffin eingebettet ist.

11. Verfahren nach einem der Ansprüche 1-10, wobei die c-met-IHC unter Verwendung des Anti-c-met-Antikörpers SP44 durchgeführt wird.

12. Verfahren nach einem der Ansprüche 1-11, wobei der Krebs nichtkleinzelliger Lungenkrebs, Nierenzellenkrebs, Pankreaskrebs, Magenkarzinom, Blasenkrebs, Speiseröhrenkrebs, Mesotheliom, Melanom, Brustkrebs, Schilddrüsenkrebs, Kolorektalkrebs, Kopf- und Nackenkrebs, Osteosarkom, Prostatakrebs oder Glioblastom ist.

13. Verfahren nach Anspruch 12, wobei der Krebs nichtkleinzelliger Lungenkrebs (NSCLC) ist.

14. Verfahren nach Anspruch 13, wobei der NSCLC Folgendes ist: lokal fortgeschrittener oder metastasierter nichtkleinzelliger Zweitlinien- oder Drittlinien-Lungenkrebs; Adenokarzinom oder Plattenepithelkarzinom.

15. Verfahren nach einem der Ansprüche 1-3, 5-14, wobei der c-met-Antagonist ein Antagonisten-Anti-c-met-Antikörper ist.

16. Verfahren nach Anspruch 15, wobei der Anti-c-met-Antikörper:

(i) (a) eine HVR1, umfassend die Sequenz GYTFTSYWLH (Seq.-ID Nr. 1); (b) eine HVR2, umfassend die Sequenz GMIDPSNSDTRFNPNFKD (Seq.-ID Nr. 2); (c) eine HVR3-HC, umfassend die Sequenz ATYRSYVTPLDY (Seq.-ID Nr. 3); (d) eine HVR1-LC, umfassend die Sequenz KSSQSLLYTSSQKNYLA (Seq.-ID Nr. 4); (e) eine HVR2-LC, umfassend die Sequenz WASTRES (Seq.-ID Nr. 5); und (f) eine HVR3-LC, umfassend die Sequenz QQYYAYPWT (Seq.-ID Nr. 6), umfasst; oder

(ii) einwertig ist und (a) ein erstes Polypeptid, umfassend eine Schwerkette, wobei das Polypeptid folgende Sequenz umfasst:

EVQLVESGGGLVQPGGSLRLSCAASGYTFTSYWLHWVRQAPGKGLEWVGMIDPS
NSDTRFNPNFKDRFTISADTSKNTAYLQMNSLRAEDTAVYYCATYRSYVTPLDYWG
QGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSC
DKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSREEMTKNQVSLSCAVKGFYPSDIAVEWESNGQP
ENNYKTTPPVLDSDGSFFLVSKLTVDKSRWQQGNVFSCSVMHEALHNHYTQKSLS
LSPGK (Seq.-ID Nr. 11);

(b) ein zweites Polypeptid, umfassend eine Leichtkette, wobei das Polypeptid die folgende Sequenz umfasst:

DIQMTQSPSSLSASVGDRVTITCKSSQSLLYTSSQKNYLAWYQQKPGKAPKLLIYW
ASTRESGVPSRFSGSGSGTDFTLTISSLQPEDFATYYCQQYYAYPWTFGQGTKVEI
KRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC (Seq.-
ID Nr. 12);

und ein drittes Polypeptid, umfassend eine Fc-Sequenz, wobei das Polypeptid die folgende Sequenz umfasst:

DKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSREEMTKNQVSLWCLVKGFYPSDIAVEWESNGQP

ENNYKTTPPVLDSDGSFFLYSKLTVDKSRWQQGNVFSCSVMHEALHNHYTQKSLS
LSPGK (Seq.-ID Nr. 13),

umfasst,

wobei die variable Schwerkettendomäne und die variable Leichtkettendomäne als ein Komplex vorhanden sind und einen einzelnen Antigenbindungsarm bilden, wobei das erste und das zweite Fc-Polypeptid in einem Komplex vorhanden sind und eine Fc-Region bilden, die die Stabilität des Antikörperfragments verglichen mit einem Fab-Molekül, umfassend den Antigenbindungsarm, erhöht.

17. Verfahren nach einem der Ansprüche 1-3, 5-15, wobei der c-met-Antagonist einer oder mehrere aus Crizotinib, Tivantinib, Carbozantinib, MGCD-265, Ficlatuzumab, humanisiertem TAK-701, Rilotumumab, Foretinib, h224G11, DN-30, MK-2461, E7050, MK-8033, PF-4217903, AMG208, JNJ-38877605, EMD1204831, INC-280, LY-2801653, SGX-126, RP1040, LY2875358, BAY-853474 und/oder LA480 ist.

18. Verfahren nach einem der Ansprüche 1-3, 5-17, wobei die Behandlung in Kombination mit einer Behandlung mit einem EGFR-Antagonisten erfolgt.

19. Verfahren nach Anspruch 18, wobei der EGFR-Antagonist Erlotinib ist.

20. Verfahren nach einem der Ansprüche 1-3, 5-17, wobei der c-met-Antagonist Onartuzumab ist und die Behandlung weiters die Behandlung mit Erlotinib umfasst oder der c-met-Antagonist Crizotinib, Tivantinib, Carbozantinib, MGCD-265, Ficlatuzumab, humanisierter TAK-701 oder Foretinib ist und die Behandlung weiters die Behandlung mit Erlotinib umfasst.

21. c-met-Antagonist zur Verwendung in einem Verfahren zur Behandlung eines Patienten mit nichtkleinzelligem Lungenkrebs (NSCLC), wobei ermittelt wurde, dass die Krebserkrankung des Patienten einen hohen Wert eines c-met-Biomarkers aufweist, wobei die c-met-Biomarker-Proteinexpression in einer Krebsprobe, die dem Patienten entnommen wurde, unter Verwendung von Immunhistochemie (IHC) bestimmt wird und eine starke c-met-Biomarkerexpression (i) 50 % oder mehr der Tumorzellen mit mittelmäßiger c-met-Färbungsintensität oder kombinierter mittelmäßiger/starker c-met-Färbungsintensität, aber weniger als 50 % der Tumorzellen mit starker Färbungsintensität (IHC-Bewertung 2) oder (ii) 50 % oder mehr der Tumorzellen mit starker c-met-Färbungsintensität (IHC-Bewertung 3) ist,
wobei die c-met-Expressionsfärbungsintensität im Vergleich zu der c-met-Färbungsintensität von Kontrollzellpellets bestimmt wird und
wobei eine Kontrolle für eine mittelmäßige c-met-Färbungsintensität die c-met-Färbungsintensität von Zelllinie A549 aufweist; eine Kontrolle für eine starke c-met-Färbungsintensität die c-met-Färbungsintensität von Zelllinie H441 aufweist; eine Kontrolle für eine negative c-met-Färbungsintensität die c-met-Färbungsintensität von Zelllinie H1155 aufweist; und eine Kontrolle für eine schwache c-met-Färbungsintensität die c-met-Färbungsintensität von Zelllinie HEK-293 aufweist.

22. c-met-Antagonist zur Verwendung nach Anspruch 21, wobei der c-met-Antagonist (i) ein Anti-c-met-Antikörper oder (ii) Onartuzumab ist.

23. c-met-Antagonist zur Verwendung nach Anspruch 21 oder 22, wobei der Patient verglichen mit einem Patienten, der keinen erhöhten c-met-Biomarkerwert aufweist, eine längere PFS und/oder OS aufweist.

24. c-met-Antagonist zur Verwendung nach einem der Ansprüche 21 bis 23, wobei die Kontrolle für eine mittelmäßige c-met-Färbungsintensität Zelllinie A549 ist; die Kontrolle für eine starke c-met-Färbungsintensität Zelllinie H441 ist; die Kontrolle für eine negative c-met-Färbungsintensität Zelllinie H1155 ist; und die Kontrolle für eine schwache c-met-Färbungsintensität Zelllinie HEK-293 ist.

25. c-met-Antagonist zur Verwendung nach einem der Ansprüche 21 bis 24, wobei der NSCLC lokal fortgeschrittener oder metastasierter nichtkleinzelliger Zweitlinien- oder Drittlinien-Lungenkrebs, ein Adenokarzinom oder ein Plattenepithelkarzinom ist.

26. c-met-Antagonist zur Verwendung nach Anspruch 21 oder 22, wobei der Patient mit einer Kombination eines Anti-c-met-Antikörpers und eines EGFR-Antagonisten behandelt wird.

27. c-met-Antagonist zur Verwendung nach Anspruch 26, wobei der EGFR-Antagonist Erlotinib ist.

28. c-met-Antagonist zur Verwendung nach Anspruch 27, wobei der Patient mit (a) Onartuzumab in einer Dosis von etwa 15 mg/kg alle drei Wochen; und (b) Erlotinib (N-(3-Ethinylphenyl)-6,7-bis(2-methoxyethyoxy)-4-chinazolinamin) in einer Dosis von 150 mg an jedem Tag des dreiwöchigen Zyklus behandelt wird.

29. Verwendung eines Diagnosesets, umfassend ein oder mehrere Reagenzien zum Bestimmen der Expression eines c-met-Biomarkers in einer Probe aus einem NSCLC-Patienten und Anweisungen für die Verwendung des Sets, **dadurch gekennzeichnet, dass** die Verwendung des Sets zum Auswählen (a) eines c-met-Antagonisten zum Behandeln des NSCLC-Patienten, falls eine große Menge des c-met-Biomarkers bestimmt wird, oder (b) eines Krebsmedikaments dient, das nicht ein c-met-Antagonist ist, um den NSCLC-Patienten zu behandeln, falls eine geringe oder im Wesentlichen nicht detektierbare Menge eines c-met-Biomarkers bestimmt wurde, wobei die c-met-Biomarker-Proteinexpression in einer Krebsprobe aus dem Patienten unter Verwendung von Immunhistochemie (IHC) bestimmt wird und (a) eine starke c-met-Biomarkerexpression (i) 50 % oder mehr der Tumorzellen mit mittelmäßiger c-met-Färbungsintensität oder kombinierter mittelmäßiger/starker c-met-Färbungsintensität, aber weniger als 50 % der Tumorzellen mit starker Färbungsintensität (IHC-Bewertung 2) oder (ii) 50 % oder mehr der Tumorzellen mit starker c-met-Färbungsintensität (IHC-Bewertung 3) ist und (b) die schwache c-met-Biomarker-Expression (i) eine negative c-met-Färbung, weniger als 50 % der Tumorzellen mit schwacher oder kombinierter schwacher und mittelmäßiger c-met-Färbungsintensität (IHC-Bewertung 0) oder (ii) 50 % oder mehr Tumorzellen mit mittelmäßiger oder kombinierter mittelmäßiger und starker c-met-Färbungsintensität (IHC-Bewertung 1) ist,

wobei die c-met-Expressionsfärbungsintensität im Vergleich zu der c-met-Färbungsintensität von Kontrollzellpellets bestimmt wird und

wobei eine Kontrolle für eine mittelmäßige c-met-Färbungsintensität die c-met-Färbungsintensität von Zelllinie A549 aufweist; eine Kontrolle für eine starke c-met-Färbungsintensität die c-met-Färbungsintensität von Zelllinie H441 aufweist; eine Kontrolle für eine negative c-met-Färbungsintensität die c-met-Färbungsintensität von Zelllinie H1155 aufweist; und eine Kontrolle für eine schwache c-met-Färbungsintensität die c-met-Färbungsintensität von Zelllinie HEK-293 aufweist.

## Revendications

1. Procédé d'identification d'un patient cancéreux qui est (a) susceptible de, ou (b) moins susceptible de, répondre au traitement avec un antagoniste de c-met comprenant l'étape de détermination si le cancer du patient présente (a) une quantité élevée ou (b) une quantité faible du biomarqueur c-met, dans lequel l'expression du biomarqueur c-met indique que le patient est (a) susceptible de répondre au traitement avec l'antagoniste de c-met, ou (b) moins susceptible de répondre au traitement avec l'antagoniste de c-met,

   dans lequel l'expression de la protéine du biomarqueur c-met est déterminée dans un échantillon du cancer obtenu du patient en utilisant l'immunohistochimie (IHC) et (a) une expression élevée du biomarqueur c-met est (i) 50 % ou plus des cellules tumorales avec une intensité de coloration modérée de c-met ou une intensité de coloration modérée/forte combinée de c-met mais moins de 50 % des cellules tumorales avec une intensité de coloration forte (score d'IHC 2), ou (ii) 50 % ou plus des cellules tumorales avec une intensité de coloration forte de c-met (score d'IHC 3), et (b) une expression faible du biomarqueur c-met est (i) une coloration négative de c-met, moins de 50 % des cellules tumorales avec une intensité de coloration faible ou faible et modérée combinée de c-met (score d'IHC 0), ou (ii) 50 % ou plus des cellules tumorales avec une intensité de coloration faible ou faible et modérée combinée de c-met mais moins de 50 % des cellules tumorales avec une intensité de coloration modérée ou modérée et forte combinée de c-met (score d'IHC 1),

   dans lequel l'intensité de la coloration de l'expression de c-met est déterminée par rapport à l'intensité de la coloration de c-met de culots de cellules témoins, et

   dans lequel un témoin pour l'intensité de coloration modérée de c-met a l'intensité de la coloration de c-met de la lignée cellulaire A549 ; un témoin pour l'intensité de coloration forte de c-met a l'intensité de la coloration de c-met de la lignée cellulaire H441 ; un témoin pour l'intensité de coloration négative de c-met a l'intensité de la coloration de c-met de la lignée cellulaire H1155 ; et un témoin pour l'intensité de coloration faible de c-met a l'intensité de la coloration de c-met de la lignée cellulaire HEK-293.

2. Procédé de détermination du pronostic d'un patient cancéreux, comprenant l'étape de détermination si le cancer du patient présente une quantité élevée du biomarqueur c-met, dans lequel l'expression du biomarqueur c-met indique que le patient est susceptible de présenter une augmentation de la survie globale (SG) et/ou de la survie sans progression (SSP) lorsque le patient est traité avec un antagoniste de c-met, dans lequel l'expression de la protéine du biomarqueur c-met est déterminée dans un échantillon du cancer obtenu du patient en utilisant l'immunohistochimie (IHC) et une expression élevée du biomarqueur c-met est (i) 50 % ou plus des cellules tumorales avec une intensité de coloration modérée de c-met ou une intensité de coloration modérée/forte combinée de c-met mais moins de 50 % des cellules tumorales avec une intensité de coloration forte (score d'IHC 2), ou (ii) 50 % ou plus des cellules tumorales avec une intensité de coloration forte de c-met (score d'IHC 3),

   dans lequel l'intensité de la coloration de l'expression de c-met est déterminée par rapport à l'intensité de la coloration de c-met de culots de cellules témoins, et

   dans lequel un témoin pour l'intensité de coloration modérée de c-met a l'intensité de la coloration de c-met de la lignée cellulaire A549 ; un témoin pour l'intensité de coloration forte de c-met a l'intensité de la coloration de c-met de la lignée cellulaire H441 ; un témoin pour l'intensité de coloration négative de c-met a l'intensité de la coloration de c-met de la lignée cellulaire H1155 ; et un témoin pour l'intensité de coloration faible de c-met a l'intensité de la coloration de c-met de la lignée cellulaire HEK-293.

3. Procédé de sélection d'un traitement pour un patient comprenant la détermination de l'expression d'un biomarqueur c-met dans un échantillon du cancer obtenu du patient, et la sélection d'un médicament pour le cancer en se basant sur le taux d'expression du biomarqueur, dans lequel le patient est sélectionné pour le traitement avec (i) un antagoniste de c-met si l'échantillon du cancer exprime le biomarqueur à un taux élevé, ou (ii) un médicament pour le cancer autre qu'un antagoniste de c-met si l'échantillon du cancer exprime le biomarqueur à un taux faible ou sensiblement indétectable,

dans lequel l'expression de la protéine du biomarqueur c-met est déterminée dans un échantillon du cancer provenant du patient en utilisant l'immunohistochimie (IHC) et (a) une expression élevée du biomarqueur c-met est (i) 50 % ou plus des cellules tumorales avec une intensité de coloration modérée de c-met ou une intensité de coloration modérée/forte combinée de c-met mais moins de 50 % des cellules tumorales avec une intensité de coloration forte (score d'IHC 2), ou (ii) 50 % ou plus des cellules tumorales avec une intensité de coloration forte de c-met (score d'IHC 3), et (b) une expression faible du biomarqueur c-met est (i) une coloration négative de c-met, moins de 50 % des cellules tumorales avec une intensité de coloration faible ou faible et modérée combinée de c-met (score d'IHC 0), ou (ii) 50 % ou plus des cellules tumorales avec une intensité de coloration faible ou faible et modérée combinée de c-met mais moins de 50 % des cellules tumorales avec une intensité de coloration modérée ou modérée et forte combinée de c-met (score d'IHC 1),

dans lequel l'intensité de la coloration de l'expression de c-met est déterminée par rapport à l'intensité de la coloration de c-met de culots de cellules témoins, et

dans lequel un témoin pour l'intensité de coloration modérée de c-met a l'intensité de la coloration de c-met de la lignée cellulaire A549 ; un témoin pour l'intensité de coloration forte de c-met a l'intensité de la coloration de c-met de la lignée cellulaire H441 ; un témoin pour l'intensité de coloration négative de c-met a l'intensité de la coloration de c-met de la lignée cellulaire H1155 ; et un témoin pour l'intensité de coloration faible de c-met a l'intensité de la coloration de c-met de la lignée cellulaire HEK-293.

4. Procédé de détermination de l'expression du biomarqueur c-met, comprenant l'étape de détermination de l'expression de la protéine du biomarqueur c-met dans un échantillon du cancer obtenu d'un patient en utilisant l'immunohistochimie (IHC) avec un anticorps anti-c-met, comprenant la détermination (a) du pourcentage des cellules tumorales dans l'échantillon avec une intensité de coloration négative de c-met, (b) du pourcentage de cellules tumorales dans l'échantillon avec une intensité de coloration faible de c-met, (c) du pourcentage de cellules tumorales dans l'échantillon avec une intensité de coloration modérée de c-met, et (d) du pourcentage de cellules tumorales dans l'échantillon avec une intensité de coloration forte de c-met, dans lequel l'expression du biomarqueur c-met a un score d'IHC de 0 où l'échantillon présente une coloration négative de c-met, ou moins de 50 % des cellules tumorales avec une intensité de coloration faible ou faible et modérée combinée de c-met ; un score d'IHC de 1 où l'échantillon présente 50 % ou plus des cellules tumorales avec une intensité de coloration faible ou faible et modérée combinée de c-met mais moins de 50 % des cellules tumorales avec une intensité de coloration modérée ou modérée et forte combinée de c-met ; un score d'IHC de 2 où l'échantillon présente 50 % ou plus des cellules tumorales avec une intensité de coloration modérée ou modérée et forte combinée de c-met mais moins de 50 % des cellules tumorales avec une intensité de coloration forte ; et un score d'IHC de 3 où l'échantillon présente 50 % ou plus des cellules tumorales avec une intensité de coloration forte de c-met,

dans lequel l'intensité de la coloration de l'expression de c-met est déterminée par rapport à l'intensité de la coloration de c-met de culots de cellules témoins, et

dans lequel un témoin pour l'intensité de coloration modérée de c-met a l'intensité de la coloration de c-met de la lignée cellulaire A549 ; un témoin pour l'intensité de coloration forte de c-met a l'intensité de la coloration de c-met de la lignée cellulaire H441 ; un témoin pour l'intensité de coloration négative de c-met a l'intensité de la coloration de c-met de la lignée cellulaire H1155 ; et un témoin pour l'intensité de coloration faible de c-met a l'intensité de la coloration de c-met de la lignée cellulaire HEK-293.

5. Procédé selon la revendication 4, dans lequel l'expression du biomarqueur c-met avec un score d'IHC de 2 ou 3 indique que le patient est susceptible de répondre au traitement avec un antagoniste de c-met.

6. Procédé selon la revendication 4 ou 5, comprenant l'étape de détermination si un cancer du patient présente un taux élevé du biomarqueur c-met, dans lequel une expression élevée du biomarqueur c-met est (i) 50 % ou plus des cellules tumorales avec une intensité de coloration modérée de c-met ou une intensité de coloration modérée et forte combinée de c-met mais moins de 50 % des cellules tumorales avec une intensité de coloration forte (score d'IHC 2), ou (ii) 50 % ou plus des cellules tumorales avec une intensité de coloration forte de c-met (score d'IHC 3), dans lequel l'expression de c-met est détectée en utilisant un anticorps anti-c-met, dans lequel l'expression du biomarqueur c-met indique que le patient est susceptible de présenter une augmentation de la SG et/ou de la SSP lorsque le patient est traité avec un antagoniste de c-met.

**7.** Procédé selon l'une quelconque des revendications 1 à 6, dans lequel le témoin pour l'intensité de coloration modérée de c-met est la lignée cellulaire A549 ; le témoin pour l'intensité de coloration forte de c-met est la lignée cellulaire H441 ; le témoin pour l'intensité de coloration négative de c-met est la lignée cellulaire H1155 ; et le témoin pour l'intensité de coloration faible de c-met est la lignée cellulaire HEK-293.

**8.** Procédé selon l'une quelconque des revendications 1, 2, et 6, dans lequel le patient présente une SSP et/ou une SG supérieure par rapport à un patient qui ne présente pas un biomarqueur c-met élevé.

**9.** Procédé selon l'une quelconque des revendications 1 à 8, dans lequel l'échantillon est obtenu (i) avant le traitement avec un antagoniste de c-met, (ii) avant le traitement avec un médicament pour le cancer, ou (iii) après que le cancer a métastasé.

**10.** Procédé selon l'une quelconque des revendications 1 à 9, dans lequel l'échantillon est fixé à la formaline et inclus dans la paraffine.

**11.** Procédé selon l'une quelconque des revendications 1 à 10, dans lequel l'IHC de c-met est effectuée en utilisant l'anticorps anti-c-met SP44.

**12.** Procédé selon l'une quelconque des revendications 1 à 11, dans lequel le cancer est un cancer du poumon non à petites cellules, un cancer des cellules rénales, un cancer pancréatique, un carcinome gastrique, un cancer de la vessie, un cancer oesophagien, un mésothéliome, un mélanome, un cancer du sein, un cancer de la thyroïde, un cancer colorectal, un cancer de la tête et du cou, un ostéosarcome, un cancer de la prostate, ou un glioblastome.

**13.** Procédé selon la revendication 12, dans lequel le cancer est un cancer du poumon non à petites cellules (NSCLC).

**14.** Procédé selon la revendication 13, dans lequel le NSCLC est : un cancer du poumon non à petites cellules de seconde ligne ou de troisième ligne localement avancé ou métastasé ; un adénocarcinome ; ou un carcinome à cellules squameuses.

**15.** Procédé selon l'une quelconque des revendications 1 à 3, 5 à 14, dans lequel l'antagoniste de c-met est un anticorps antagoniste anti-c-met.

**16.** Procédé selon la revendication 15, dans lequel l'anticorps anti-c-met :

(i) comprend (a) une HVR1 comprenant la séquence GYTFTSYWLH (SEQ ID NO : 1) ; (b) une HVR2 comprenant la séquence GMIDPSNSDTRFNPNFKD (SEQ ID NO : 2) ; (c) une HVR3-HC comprenant la séquence ATYRSYVTPLDY (SEQ ID NO : 3) ; (d) une HVR1-LC comprenant la séquence KSSQSLLYTSSQKNYLA (SEQ ID NO : 4) ; (e) une HVR2-LC comprenant la séquence WASTRES (SEQ ID NO : 5) ; et (f) une HVR3-LC comprenant la séquence QQYYAYPWT (SEQ ID NO : 6) ; ou

(ii) est monovalent et comprend (a) un premier polypeptide comprenant une chaîne lourde, ledit polypeptide comprenant la séquence :

```
EVQLVESGGGLVQPGGSLRLSCAASGYTFTSYWLHWVRQAPGKGLEWVGMIDPS
NSDTRFNPNFKDRFTISADTSKNTAYLQMNSLRAEDTAVYYCATYRSYVTPLDYWGQGT
LVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSREEMTKNQVSLSCAVKGFYPSDIAVEWESNGQPENNYKTTPPVLDSDGSFFL
VSKLTVDKSRWQQGNVFSCSVMHEALHNHYTQKSLSLSPGK
```

(SEQ ID NO : 11) ; (b) un deuxième polypeptide comprenant une chaîne légère, le polypeptide comprenant la séquence

```
DIQMTQSPSSLSASVGDRVTITCKSSQSLLYTSSQKNYLAWYQQKPGKAPKLLIYWAST
RESGVPSRFSGSGSGTDFTLTISSLQPEDFATYYCQQYYAYPWTFGQGTKVEIKRTVAA
PSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
```

(SEQ ID NO : 12) ; et un troisième polypeptide comprenant une séquence Fc, le polypeptide comprenant la séquence

```
DKTHTCPPCPAPELLGGPSVFLFPPKPKDTLMISRTPEVTCVVVDVSHEDPEVKFNWYV
```

```
DGVEVHNAKTKPREEQYNSTYRVVSVLTVLHQDWLNGKEYKCKVSNKALPAPIEKTISK
AKGQPREPQVYTLPPSREEMTKNQVSLWCLVKGFYPSDIAVEWESNGQPENNYKTTPPV
LDSDGSFFLYSKLTVDKSRWQQGNVFSCSVMHEALHNHYTQKSLSLSPGK
```

(SEQ ID NO : 13), dans lequel le domaine variable de la chaîne lourde et le domaine variable de la chaîne légère sont présents sous la forme d'un complexe et forment un seul bras de liaison de l'antigène, dans lequel les premier et second polypeptides Fc sont présents dans un complexe et forment une région Fc qui augmente la stabilité dudit fragment d'anticorps comparativement à une molécule de Fab comprenant ledit bras de liaison de l'antigène.

17. Procédé selon l'une quelconque des revendications 1 à 3, 5 à 15, dans lequel l'antagoniste de c-met est un ou plusieurs des crizotinib, tivantinib, carbozantinib, MGCD-265, ficlatuzumab, TAK-701 humanisé, rilotumumab, forétinib, h224G11, DN-30, MK-2461, E7050, MK-8033, PF-4217903, AMG208, JNJ-38877605, EMD1204831, INC-280, LY-2801653, SGX-126, RP1040, LY2875358, BAY-853474, et/ou LA480.

18. Procédé selon l'une quelconque des revendications 1 à 3, 5 à 17, dans lequel le traitement est en combinaison avec un traitement avec un antagoniste de l'EGFR.

19. Procédé selon la revendication 18, dans lequel l'antagoniste de l'EGFR est l'erlotinib.

20. Procédé selon l'une quelconque des revendications 1 à 3, 5 à 17, dans lequel l'antagoniste de c-met est l'onartuzumab et le traitement comprend en outre un traitement avec l'erlotinib, ou l'antagoniste de c-met est le crizotinib, le tivantinib, le carbozantinib, MGCD-265, le ficlatuzumab, TAK-701 humanisé, ou le forétinib, et le traitement comprend en outre un traitement avec l'erlotinib.

21. Antagoniste de c-met pour une utilisation dans un procédé de traitement d'un patient souffrant d'un cancer du poumon non à petites cellules (NSCLC) où le cancer du patient a été trouvé comme présentant une quantité élevée d'un biomarqueur c-met, dans lequel l'expression de la protéine du biomarqueur c-met est déterminée dans un échantillon du cancer obtenu du patient en utilisant l'immunohistochimie (IHC) et une expression élevée du biomarqueur c-met est (i) 50 % ou plus des cellules tumorales avec une intensité de coloration modérée de c-met ou une intensité de coloration modérée/forte combinée de c-met (score d'IHC 2), ou (ii) 50 % ou plus des cellules tumorales avec une intensité de coloration forte de c-met (score d'IHC 3),
dans lequel l'intensité de la coloration de l'expression de c-met est déterminée par rapport à l'intensité de la coloration de c-met de culots de cellules témoins, et
dans lequel un témoin pour l'intensité de coloration modérée de c-met a l'intensité de la coloration de c-met de la lignée cellulaire A549 ; un témoin pour l'intensité de coloration forte de c-met a l'intensité de la coloration de c-met de la lignée cellulaire H441 ; un témoin pour l'intensité de coloration négative de c-met a l'intensité de la coloration de c-met de la lignée cellulaire H1155 ; et un témoin pour l'intensité de coloration faible de c-met a l'intensité de la coloration de c-met de la lignée cellulaire HEK-293.

22. Antagoniste de c-met pour une utilisation selon la revendication 21, dans lequel l'antagoniste de c-met est (i) un anticorps anti-c-met, ou (ii) est l'onartuzumab.

**23.** Antagoniste de c-met destiné à être utilisé selon la revendication 21 ou 22, dans lequel le patient présente une SSP et/ou une SG supérieure par rapport à un patient qui ne présente pas un biomarqueur c-met élevé.

**24.** Antagoniste de c-met destiné à être utilisé selon l'une quelconque des revendications 21 à 23, dans lequel le témoin pour l'intensité de coloration modérée de c-met est la lignée cellulaire A549 ; le témoin pour l'intensité de coloration forte de c-met est la lignée cellulaire H441 ; le témoin pour l'intensité de coloration négative de c-met est la lignée cellulaire H1155 ; et le témoin pour l'intensité de coloration faible de c-met est la lignée cellulaire HEK-293.

**25.** Antagoniste de c-met destiné à être utilisé selon l'une quelconque des revendications 21 à 24, dans lequel le NSCLC est un cancer du poumon non à petites cellules de seconde ligne ou de troisième ligne localement avancé ou métastasé, un adénocarcinome, ou un carcinome à cellules squameuses.

**26.** Antagoniste de c-met destiné à être utilisé selon la revendication 21 ou 22, dans lequel le patient est traité avec une combinaison d'un anticorps anti-c-met et d'un antagoniste de l'EGFR.

**27.** Antagoniste de c-met destiné à être utilisé selon la revendication 26, dans lequel l'antagoniste de l'EGFR est l'erlotinib.

**28.** Antagoniste de c-met destiné à être utilisé selon la revendication 27, dans lequel le patient est traité avec (a) l'onartuzumab à une dose d'environ 15 mg/kg toutes les trois semaines ; et (b) l'erlotinib (N-(3-éthynylphényl)-6,7-bis(2-méthoxyéthoxy)-4-quinazolinamine) à une dose d'environ 150 mg, tous les jours d'un cycle de trois semaines.

**29.** Utilisation d'un kit de diagnostic comprenant un ou plusieurs réactifs pour la détermination de l'expression d'un biomarqueur c-met dans un échantillon provenant d'un patient souffrant d'un NSCLC, et des instructions pour utiliser le kit **caractérisée en ce que** l'utilisation du kit est destinée à la sélection (a) d'un antagoniste de c-met pour traiter le patient souffrant de NSCLC si une quantité élevée du biomarqueur c-met est déterminée ou (b) un médicament pour le cancer autre qu'un antagoniste de c-met pour traiter le patient souffrant de NSCLC si une quantité faible ou sensiblement indétectable du biomarqueur c-met est déterminée, dans laquelle l'expression de la protéine du biomarqueur c-met est déterminée dans un échantillon du cancer provenant du patient en utilisant l'immunohistochimie (IHC) et (a) une expression élevée du biomarqueur c-met est (a) 50 % ou plus des cellules tumorales avec une intensité de coloration modérée de c-met ou une intensité de coloration modérée/forte combinée de c-met mais moins de 50 % des cellules tumorales avec une intensité de coloration forte (score d'IHC 2), ou (ii) 50 % ou plus des cellules tumorales avec une intensité de coloration forte de c-met (score d'IHC 3), et (b) une expression faible du biomarqueur c-met est

(i) une coloration négative de c-met, moins de 50 % des cellules tumorales avec une intensité de coloration faible ou faible et modérée combinée de c-met (score d'IHC 0), ou
(ii) 50 % ou plus des cellules tumorales avec une intensité de coloration faible ou faible et modérée combinée de c-met mais moins de 50 % des cellules tumorales avec une intensité de coloration modérée ou modérée et forte combinée de c-met (score d'IHC 1),
dans lequel l'intensité de la coloration de l'expression de c-met est déterminée par rapport à l'intensité de la coloration de c-met de culots de cellules témoins, et
dans lequel un témoin pour l'intensité de coloration modérée de c-met a l'intensité de la coloration de c-met de la lignée cellulaire A549 ; un témoin pour l'intensité de coloration forte de c-met a l'intensité de la coloration de c-met de la lignée cellulaire H441 ; un témoin pour l'intensité de coloration négative de c-met a l'intensité de la coloration de c-met de la lignée cellulaire H1155 ; et un témoin pour l'intensité de coloration faible de c-met a l'intensité de la coloration de c-met de la lignée cellulaire HEK-293.

H1155
negative

293
weak

A549
moderate

H441
strong

*FIG. 1*

FIG. 2

## Analysis of Met High* Patients

### PFS, HR=0.56

|  | Erlotinib +Placebo (n=30) | Erlotinib +MetMab (n=35) |
|---|---|---|
| Median PFS (wk) | 6.4 | 12.4 |
| Hazard ratio | | 0.56 |
| 95% CI | | 0.31-1.02 |
| Log-rank p-value | | 0.0547 |
| # Events | 25 | 19 |

Number at Risk:

| | | | | | | | | | |
|---|---|---|---|---|---|---|---|---|---|
| Erlotinib+Placebo | 30 | 18 | 5 | 3 | 3 | 2 | 2 | 0 | 0 |
| Erlotinib+MetMab | 35 | 22 | 9 | 5 | 3 | 1 | 1 | 1 | 1 |

### Met High OS, HR=0.55

|  | Erlontinib +Placebo (n=30) | Erlotinib +MetMab (n=35) |
|---|---|---|
| Median OS (mo) | 7.4 | 7.7 |
| Hazard ratio | | 0.55 |
| 95% CI | | 0.26-1.16 |
| Log-rank p-value | | 0.1113 |
| # Events | 20 | 13 |

Number at Risk:

| | | | | | | |
|---|---|---|---|---|---|---|
| Erlotinib+Placebo | 30 | 17 | 9 | 3 | 0 | 0 |
| Erlotinib+MetMab | 35 | 26 | 10 | 3 | 1 | 0 |

12/23 patients from the erlotinib+placebo arm who crossed over to MetMAb were Met High.

*Met High = 50% or more tumor cells with a staining intensity of $2^+$ or $3^+$

## FIG. 3

## Analysis of Met Low Patients

### PFS, HR=2.01

|  | Erlontinib +Placebo (n=29) | Erlotinib +MetMab (n=27) |
|---|---|---|
| Median PFS (wk) | 11.4 | 6.0 |
| Hazard ratio | | 2.01 |
| 95% CI | | 1.04-3.91 |
| Log-rank p-value | | 0.0354 |
| # Events | 20 | 23 |

Number at Risk:

| | 0 | 3 | 6 | 9 | 12 | 15 | 18 | 21 | 24 |
|---|---|---|---|---|---|---|---|---|---|
| Erlotinib+Placebo | 29 | 22 | 9 | 6 | 2 | 2 | 0 | 0 | 0 |
| Erlotinib+MetMab | 27 | 15 | 5 | 1 | 0 | 0 | 0 | 0 | 0 |

### OS, HR=3.02

|  | Erlontinib +Placebo (n=29) | Erlotinib +MetMab (n=27) |
|---|---|---|
| Median OS (mo) | 9.2 | 5.5 |
| Hazard ratio | | 3.02 |
| 95% CI | | 1.13-8.11 |
| Log-rank p-value | | 0.0212 |
| # Events | 9 | 14 |

Number at Risk:

| | 0 | 3 | 6 | 9 | 12 | 15 |
|---|---|---|---|---|---|---|
| Erlotinib+Placebo | 29 | 23 | 9 | 3 | 0 | 0 |
| Erlotinib+MetMab | 27 | 12 | 3 | 0 | 0 | 0 |

10/23 patients from the erlotinib+placebo arm who crossed over to MetMAb were Met Low.

## FIG. 4

## Analysis of All Patients

### PFS, HR=1.09

|  | Erlontinib +Placebo (n=64) | Erlotinib +MetMab (n=64) |
|---|---|---|
| Median PFS (wk) | 11.1 | 9.6 |
| Hazard ratio | | 1.09 |
| 95% CI | | 0.71-1.67 |
| Log-rank p-value | | 0.6988 |
| # Events | 45 | 40 |

Number at Risk:

| | | | | | | | | | |
|---|---|---|---|---|---|---|---|---|---|
| Erlotinib+Placebo | 64 | 44 | 17 | 11 | 6 | 5 | 2 | 0 | 0 |
| Erlotinib+MetMab | 64 | 39 | 14 | 6 | 3 | 1 | 1 | 1 | 1 |

### OS, HR=1.09

|  | Erlontinib +Placebo (n=64) | Erlotinib +MetMab (n=64) |
|---|---|---|
| Median OS (mo) | 8.2 | 7.1 |
| Hazard ratio | | 1.09 |
| 95% CI | | 0.62-1.91 |
| Log-rank p-value | | 0.7642 |
| # Events | 25 | 24 |

Number at Risk:

| | | | | | | |
|---|---|---|---|---|---|---|
| Erlotinib+Placebo | 64 | 44 | 20 | 6 | 0 | 0 |
| Erlotinib+MetMab | 64 | 38 | 13 | 3 | 1 | 0 |

23 patients from the erlotinib+placebo arm crossed over to MetMAb.

## FIG. 5

## FIG. 6

### PFS by subgroups (overall population)

| Baseline Risk Factor | Erlotinib +Placebo Median (wk) | Erlotinib +MetMAb Median (wk) | Hazard Ratio | (95% CI) | p-Value |
|---|---|---|---|---|---|
| All Subjects (n=128) | 11.1 | 9.6 | 1.09 | (0.71–1.67) | 0.699 |
| Met IHC Scheme II Score | | | | | |
| 0 (n=18) | 8.8 | 5.9 | 2.94 | (0.92–9.40) | 0.058 |
| 1 (n=38) | 11.4 | 6.1 | 1.82 | (0.79–4.18) | 0.151 |
| 2 (n=51) | 6.4 | 12.9 | 0.57 | (0.28–1.14) | 0.105 |
| 3 (n=14) | 6.3 | 11.6 | 0.36 | (0.09–1.33) | 0.108 |
| Histology Category | | | | | |
| Non-Squamous Cell (n=97) | 9.4 | 9.6 | 1.05 | (0.64–1.74) | 0.836 |
| Squamous Cell (n=31) | 11.4 | 6.3 | 1.27 | (0.55–2.95) | 0.579 |
| Tobacco History | | | | | |
| <100 Cigarettes (n=18) | 11.7 | 5.9 | 2.30 | (0.44–11.87) | 0.308 |
| ≥100 Cigarettes (n=110) | 10.9 | 9.6 | 0.99 | (0.63–1.55) | 0.967 |
| ECOG | | | | | |
| 0/1 (n=122) | 11.3 | 9.7 | 1.08 | (0.70–1.68) | 0.727 |
| 2+ (n=6) | 6.0 | 5.9 | 0.85 | (0.14–5.24) | 0.863 |
| EGFR Mutation | | | | | |
| Wildtype (n=99) | 10.9 | 6.1 | 1.28 | (0.80–2.05) | 0.308 |
| Mutant (n=13) | na | na | 1.63 | (0.10–26.5) | 0.728 |
| KRAS Mutation | | | | | |
| Wildtype (n=86) | 11.3 | 6.3 | 1.19 | (0.71–2.00) | 0.517 |
| Mutant (n=26) | 11.4 | 6.0 | 1.04 | (0.38–2.79) | 0.945 |

Erlotinib +MetMAb Better — Erlotinib +Placebo Better

0.5  1  2

## FIG. 7

### OS by subgroups

| Baseline Risk Factor | Erlotinib +Placebo Median (mo) | Erlotinib +MetMAb Median (mo) | Hazard Ratio | (95% CI) | p-Value |
|---|---|---|---|---|---|
| All Subjects (n=128) | 8.2 | 7.1 | 1.09 | (0.62–1.91) | 0.764 |
| Met IHC Scheme II Score | | | | | |
| 0 (n=18) | na | 2.3 | 3.98 | (0.92–17.23) | 0.049 |
| 1 (n=38) | 9.2 | 6.5 | 2.91 | (0.74–11.43) | 0.110 |
| 2 (n=51) | 7.4 | na | 0.57 | (0.24–1.39) | 0.211 |
| 3 (n=14) | 3.8 | 7.1 | 0.10 | (0.01–1.00) | 0.018 |
| Histology Category | | | | | |
| Non-Squamous Cell (n=97) | na | 7.1 | 0.98 | (0.50–1.90) | 0.941 |
| Squamous Cell (n=31) | 7.7 | 7.7 | 1.27 | (0.44–3.65) | 0.655 |
| Tobacco History | | | | | |
| Current (n=25) | 4.1 | 5.6 | 0.71 | (0.20–2.53) | 0.597 |
| Never (n=18) | na | na | 1.24 | (0.28–5.57) | 0.781 |
| Previous (n=85) | 8.2 | 7.7 | 1.07 | (0.53–2.16) | 0.861 |
| ECOG | | | | | |
| 0/1 (n=122) | 8.2 | 7.7 | 1.02 | (0.57–1.84) | 0.945 |
| 2+ (n=6) | na | 3.4 | 0.98 | (0.09–10.98) | 0.984 |
| EGFR Mutation | | | | | |
| Wildtype (n=99) | 7.7 | 6.5 | 1.28 | (0.70–2.35) | 0.429 |
| Mutant (n=13) | na | na | 8.04E7 | (0.00– ) | 0.317 |
| KRAS Mutation | | | | | |
| Wildtype (n=86) | 8.2 | 7.1 | 1.33 | (0.66–2.66) | 0.421 |
| Mutant (n=26) | na | 7.7 | 1.00 | (0.31–3.31) | 0.995 |

Erlotinib +MetMAb Better — Erlotinib +Placebo Better

0.5   1   2

EP 2 612 151 B1

# Analysis of Placebo-Treated Patients

## PFS, HR=1.73

|  | Met Low (n=29) | Met High (n=30) |
|---|---|---|
| Median PFS (wk) | 11.4 | 6.4 |
| Hazard ratio | | 1.73 |
| 95% CI | | 0.93-3.23 |
| Log-rank p-value | | 0.0817 |
| # Events | 17 | 25 |

## OS, HR=2.52

|  | Met Low | Met High |
|---|---|---|
| Median PFS (mo) | 9.2 | 7.4 |
| Hazard ratio | | 2.52 |
| 95% CI | | 1.04-6.13 |
| Log-rank p-value | | 0.0350 |
| # Events | 17 | 25 |

----- Met Low (n=29)
——— Met High (n=30)

**Met High Definition:** ≥50% Tumor Cells with a Staining Intensity of $2^+$ or $3^+$

## FIG. 8

## FIG. 9

### Subgroup analysis of PFS in Met High patients

| Baseline Risk Factor | Total n | Placebo+ Tarceva | | MetMAb+ Tarceva | | Hazard Ratio | (95% CI) | p-Value | MetMAb+ Tarceva better | Placebo+ Tarceva better |
|---|---|---|---|---|---|---|---|---|---|---|
| | | n | Median (wk) | n | Median (wk) | | | | | |
| All Subjects | 65 | 30 | 6.4 | 35 | 12.6 | 0.509 | (0.278 - 0.935) | 0.027 | | |
| Met IHC | | | | | | | | | | |
| 2 | 51 | 25 | 6.4 | 26 | 17.9 | 0.511 | (0.251 - 1.039) | 0.059 | | |
| 3 | 14 | 5 | 6.3 | 9 | 11.6 | 0.351 | (0.092 - 1.331) | 0.108 | | |
| Histology Category | | | | | | | | | | |
| Non-Squamous Cell | 56 | 26 | 6.4 | 30 | 12.4 | 0.559 | (0.290 - 1.079) | 0.079 | | |
| Squamous Cell | 9 | 4 | 8.6 | 5 | 23.9 | 0.166 | (0.018 - 1.542) | 0.076 | | |
| Tobacco History | | | | | | | | | | |
| Current | 14 | 8 | 6.3 | 6 | 10.9 | 0.706 | (0.176 - 2.840) | 0.623 | | |
| Never | 13 | 6 | 11.7 | 7 | | 0.980 | (0.197 - 4.884) | 0.981 | | |
| Previous | 38 | 16 | 6.4 | 22 | 12.6 | 0.418 | (0.193 - 0.906) | 0.023 | | |
| EGFR Mutation | | | | | | | | | | |
| Wildtype | 48 | 24 | 6.4 | 24 | 10.9 | 0.707 | (0.360 - 1.390) | 0.313 | | |
| Mutant | 9 | 2 | | 7 | | 3.24E7 | (0.000 - ) | 0.655 | | |
| KRAS Mutation | | | | | | | | | | |
| Wildtype | 44 | 20 | 9.4 | 24 | 12.6 | 0.544 | (0.259 - 1.143) | 0.103 | | |
| Mutant | 13 | 6 | 6.3 | 7 | 11.9 | 0.494 | (0.108 - 2.250) | 0.353 | | |

EP 2 612 151 B1

## Subgroup analysis of OS in Met High patients

### FIG. 10

| Baseline Risk Factor | Total n | Placebo+ Tarceva | | MetMAb+ Tarceva | | Hazard Ratio | (95% CI) | p-Value | | |
|---|---|---|---|---|---|---|---|---|---|---|
| | | n | Median (mo) | n | Median (mo) | | | | MetMAb+ Tarceva better | Placebo+ Tarceva better |
| All Subjects | 65 | 30 | 3.8 | 35 | 12.6 | 0.448 | (0.222 - 0.902) | 0.021 | | |
| Met IHC Scheme II | | | | | | | | | | |
| 2 | 51 | 25 | 7.4 | 26 | | 0.484 | (0.204 - 1.144) | 0.091 | | |
| 3 | 14 | 5 | 3.6 | 9 | 12.6 | 0.050 | (0.006 - 0.943) | 0.000 | | |
| Histology Category | | | | | | | | | | |
| Non-Squamous Cell | 56 | 26 | 3.7 | 30 | 12.6 | 0.478 | (0.229 - 0.996) | 0.044 | | |
| Squamous Cell | 9 | 4 | 7.4 | 5 | | 0.000 | (0.000 - ) | 0.048 | | |
| Tobacco History | | | | | | | | | | |
| Current | 14 | 8 | 2.6 | 6 | 7.1 | 0.621 | (0.145 - 2.660) | 0.517 | | |
| Never | 13 | 6 | | 7 | | 0.874 | (0.176 - 4.337) | 0.869 | | |
| Previous | 38 | 16 | 5.4 | 22 | 12.6 | 0.375 | (0.142 - 0.987) | 0.039 | | |
| EGFR Mutation | | | | | | | | | | |
| Wildtype | 48 | 24 | 3.8 | 24 | 7.7 | 0.554 | (0.251 - 1.222) | 0.138 | | |
| Mutant | 9 | 2 | | 7 | 12.6 | 1.33E7 | (0.000 - ) | 0.593 | | |
| KRAS Mutation | | | | | | | | | | |
| Wildtype | 44 | 20 | 8.1 | 24 | 12.6 | 0.478 | (0.195 - 1.173) | 0.100 | | |
| Mutant | 13 | 6 | 2.8 | 7 | 7.7 | 0.594 | (0.131 - 2.685) | 0.494 | | |

EP 2 612 151 B1

## FIG. 11

| Baseline Risk Factor | Total n | Placebo+ Tarceva | | MetMAb+ Tarceva | | Hazard Ratio | (95% CI) | p-Value | MetMAb+ Tarceva better | Placebo+ Tarceva better |
|---|---|---|---|---|---|---|---|---|---|---|
| | | n | Median (wk) | n | Median (wk) | | | | | |
| All Subjects | 56 | 29 | 11.4 | 27 | 6.0 | 2.187 | (1.170 - 4.088) | 0.012 | | |
| Met IHC | | | | | | | | | | |
| 0 | 18 | 11 | 8.8 | 7 | 5.9 | 2.937 | (0.918 - 9.403) | 0.058 | | |
| 1 | 38 | 18 | 11.4 | 20 | 6.1 | 2.039 | (0.940 - 4.425) | 0.066 | | |
| Histology Category | | | | | | | | | | |
| Non-Squamous Cell | 36 | 19 | 11.4 | 17 | 6.0 | 2.605 | (1.131 - 6.002) | 0.020 | | |
| Squamous Cell | 20 | 10 | 11.4 | 10 | 6.1 | 1.802 | (0.645 - 5.035) | 0.255 | | |
| Tobacco History | | | | | | | | | | |
| Current | 8 | 2 | 13.4 | 6 | 6.0 | 3.684 | (0.434 - 31.246 | 0.202 | | |
| Never | 3 | 1 | 29.3 | 2 | 5.9 | 4.73E8 | (0.000 - ) | 0.225 | | |
| Previous | 45 | 26 | 11.4 | 19 | 6.1 | 1.906 | (0.929 - 3.911) | 0.073 | | |
| EGFR Mutation | | | | | | | | | | |
| Wildtype | 49 | 24 | 11.1 | 25 | 6.0 | 1.813 | (0.941 - 3.494) | 0.071 | | |
| Mutant | 4 | 4 | 30.3 | | | | ( - ) | 0.000 | | |
| KRAS Mutation | | | | | | | | | | |
| Wildtype | 40 | 21 | 11.6 | 19 | 6.1 | 2.375 | (1.113 - 5.066) | 0.021 | | |
| Mutant | 13 | 7 | 16.0 | 6 | 5.9 | 2.552 | (0.602 - 10.814 | 0.188 | | |

EP 2 612 151 B1

## Subgroup analysis of OS in Met Low patients

**FIG. 12**

| Baseline Risk Factor | Total n | Placebo+ Tarceva n | Placebo+ Tarceva Median (mo) | MetMAb+ Tarceva n | MetMAb+ Tarceva Median (mo) | Hazard Ratio | (95% CI) | p-Value |
|---|---|---|---|---|---|---|---|---|
| All Subjects | 56 | 29 | | 27 | 6.5 | 2.481 | (1.059 - 5.814) | 0.031 |
| Met IHC Scheme II | | | | | | | | |
| 0 | 18 | 11 | | 7 | 2.3 | 3.913 | (0.926 - 16.534 | 0.046 |
| 1 | 38 | 18 | | 20 | 8.1 | 2.235 | (0.772 - 6.471) | 0.129 |
| Histology Category | | | | | | | | |
| Non-Squamous Cell | 36 | 19 | | 17 | 8.1 | 2.315 | (0.754 - 7.104) | 0.131 |
| Squamous Cell | 20 | 10 | 9.2 | 10 | 2.3 | 2.630 | (0.650 - 10.642 | 0.159 |
| Tobacco History | | | | | | | | |
| Current | 8 | 2 | | 6 | 8.1 | 4.03E7 | (0.000 - ) | 0.392 |
| Never | 3 | 1 | 7.4 | 2 | | 1.28E8 | (0.000 - ) | 0.480 |
| Previous | 45 | 26 | | 19 | 6.5 | 2.577 | (1.003 - 6.620) | 0.042 |
| EGFR Mutation | | | | | | | | |
| Wildtype | 49 | 24 | | 25 | 6.5 | 2.154 | (0.879 - 5.281) | 0.086 |
| Mutant | 4 | 4 | | | | | ( - ) | 0.000 |
| KRAS Mutation | | | | | | | | |
| Wildtype | 40 | 21 | 9.2 | 19 | 6.5 | 2.440 | (0.935 - 6.369) | 0.060 |
| Mutant | 13 | 7 | | 6 | | 1.439 | (0.202 - 10.269 | 0.715 |

MetMAb+ Tarceva better — Placebo+ Tarceva better

FIG. 13

FIG. 14

**PFS: HR=1.09**

|  | Placebo + Erlotinib | MetMAb + Erlotinib |
|---|---|---|
| Median (mo) | 2.6 | 2.2 |
| HR | | 1.09 |
| (95% CI) | | (0.73–1.62) |
| Log-rank p-value | | 0.69 |
| No. of events | 56 | 48 |

Probability of Progression Free

—— Placebo + Erlotinib
----- MetMAb + Erlotinib

Time to Progression (Months)

**OS: HR=0.8**

|  | Placebo + Erlotinib | MetMAb + Erlotinib |
|---|---|---|
| Median (mo) | 7.4 | 8.9 |
| HR | | 0.80 |
| (95% CI) | | (0.50–1.30) |
| Log-rank p-value | | 0.34 |
| No. of events | 41 | 34 |

Probability of Survival

—— Placebo + Erlotinib
----- MetMAb + Erlotinib

Overall Survival (Months)

*FIG. 15*

## FIG. 16

| Baseline risk factor | Placebo+ erlotinib | | MetMAb+ erlotinib | | Hazard Ratio |
|---|---|---|---|---|---|
| | n | Median (mo) | n | Median (mo) | |
| All subjects | 68 | 7.4 | 69 | 8.9 | 0.80 |
| Met Dx status | | | | | |
| Negative | 31 | 15.3 | 31 | 8.1 | 2.11 |
| Positive | 31 | 3.8 | 35 | 12.6 | 0.38 |
| Histology category | | | | | |
| Non-squamous cell | 48 | 7.6 | 49 | 10.4 | 0.80 |
| Squamous cell | 20 | 7.1 | 20 | 8.6 | 0.81 |
| Tobacco history | | | | | |
| <100 cigarettes | 8 | 8.3 | 10 | NE | 0.76 |
| ≥100 cigarettes | 60 | 7.4 | 59 | 8.9 | 0.80 |
| ECOG performance status  0/1 | 66 | 7.6 | 65 | 8.9 | 0.79 |
| ≥2 | 2 | 3.9 | 4 | 3.4 | 0.85 |
| EGFR mutation | | | | | |
| Wildtype | 50 | 7.4 | 49 | 8.5 | 0.92 |
| Mutant | 6 | NE | 7 | NE | 2.19 |
| KRAS mutation | | | | | |
| Wildtype | 43 | 7.7 | 43 | 10.4 | 0.78 |
| Mutant | 13 | NE | 13 | 8.5 | 1.63 |

MetMAb+ erlotinib better / Placebo+ erlotinib better

0.5    1    2

Median overall survival was estimated from Kaplan–Meier curves
Hazard ratio relative to placebo + erlotinib was estimated by Cox regression. Unstratified hazard ratio is displayed
NE: Not evaluable

## FIG. 17

| Baseline risk factor | Placebo+ erlotinib | | MetMAb+ erlotinib | | Hazard Ratio |
|---|---|---|---|---|---|
| | n | Median (mo) | n | Median (mo) | |
| Met Dx IHC status | | | | | |
| 0 | 12 | NE | 7 | 5.5 | 2.32 |
| 1 | 19 | 15.3 | 24 | 8.6 | 2.31 |
| Histology category | | | | | |
| Non-squamous cell | 19 | 15.3 | 17 | 10.4 | 1.90 |
| Squamous cell | 12 | 9.2 | 14 | 5.5 | 2.51 |
| Tobacco history | | | | | |
| ≥100 cigarettes | 30 | 15.3 | 29 | 8.1 | 2.28 |
| ECOG performance status | | | | | |
| 0/1 | 31 | 15.3 | 28 | 8.6 | 1.82 |
| EGFR mutation | | | | | |
| Wildtype | 24 | 15.3 | 25 | 8.1 | 2.00 |
| KRAS mutation | | | | | |
| Wildtype | 21 | 9.2 | 19 | 8.1 | 2.26 |
| Mutant | 7 | NE | 6 | NE | 1.44 |

MetMAb+ erlotinib better   Placebo+ erlotinib better

0.5    1    2

Median overall survival was estimated from Kaplan–Meier curves
Hazard ratio relative to placebo + erlotinib was estimated by Cox regression. Unstratified hazard ratio is displayed
NE: Not evaluable

EP 2 612 151 B1

FIG. 18

EP 2 612 151 B1

FIG. 19

*FIG. 20*

*FIG. 21*

EP 2 612 151 B1

# EP 2 612 151 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005012359 A
- WO 200487207 A
- US 4943533 A, Mendelsohn
- WO 9640210 A
- US 5212290 A
- US 5891996 A
- WO 9850433 A
- EP 659439 A2
- US 4683195 A
- WO 2006015371 A
- WO 05016382 A
- WO 2007126799 A
- WO 2009007427 A
- WO 20110129481 A
- US 20110104176 A
- WO 2009134776 A
- WO 2010059654 A
- WO 2011020925 A
- WO 2005063816 A
- WO 2007143090 A
- US 7718174 B2
- WO 2009111691 A
- US 6235883 B
- US 20100254989 A
- US 5616582 A
- US 5457105 A
- US 5475001 A
- US 5654307 A
- US 5679683 A
- US 6084095 A
- US 6265410 B
- US 6455534 B
- US 6521620 B
- US 6596726 B
- US 6713484 B
- US 5770599 A
- US 6140332 A
- US 5866572 A
- US 6399602 B
- US 6344459 B
- US 6602863 B
- US 6391874 B
- WO 9814451 A
- WO 9850038 A
- WO 9909016 A
- WO 9924037 A
- WO 9935146 A
- WO 0132651 A
- US 6344455 B

- US 5760041 A
- US 6002008 A
- US 5747498 A
- WO 2003013541 A
- WO 9702266 A
- WO 9316185 A
- US 5571894 A
- US 5587458 A
- US 5869046 A
- EP 404097 A
- WO 199301161 A
- US 6248516 B1
- US 5731168 A
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- WO 2009089004 A1
- US 4676980 A
- US 20060025576 A1
- US 20080069820 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A

- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 6737056 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 5208020 A

- US 5416064 A
- EP 0425235 B1
- US 5635483 A
- US 5780588 A
- US 7498298 B
- US 5712374 A
- US 5714586 A
- US 5739116 A
- US 5767285 A
- US 5770701 A
- US 5770710 A
- US 5773001 A
- US 5877296 A
- US 6630579 B
- WO 9411026 A
- US 4275149 A
- US 4737456 A
- US 4318980 A
- US 61378911 B
- WO 2010045345 A
- WO 61503489 A
- WO 61492338 A
- WO 61487527 A
- WO 61420703 A
- WO 61390995 A
- WO 61389922 A
- WO 61378911 A

**Non-patent literature cited in the description**

- **GOETSCH et al.** *Biomarkers in Medicine,* 2010, vol. 4, 149-170
- **SMOLEN et al.** *PNAS,* 2006, vol. 103, 2316-2321
- **OKUDA et al.** *Cancer Science,* 2008, vol. 99, 2280-2285
- **KAMIYA.** *Human c-Met ELISA for the quantitative determination of c-Met in human serum, EDTA-plasma or cell culture media,* 2008, 1-6
- **ULLRICH et al.** *Nature,* 1984, vol. 309, 418425
- **LYNCH et al.** *New England Journal of Medicine,* 2004, vol. 350, 2129
- **PAEZ et al.** *Science,* 2004, vol. 304, 1497
- **PAO et al.** *PNAS,* 2004, vol. 101, 13306
- **STRAGLIOTTO et al.** *Eur. J. Cancer,* 1996, vol. 32A, 636-640
- **JOHNS et al.** *J. Biol. Chem.,* 2004, vol. 279 (29), 30375-30384
- **MULLIS et al.** Cold Spring Harbor Symp. Quant. Biol. PCR Technology, 1987, vol. 51, 263
- **CRONIN et al.** *Am. J. Pathol.,* 2004, vol. 164 (1), 35-42
- **MA et al.** *Cancer Cell,* 2004, vol. 5, 607-616
- **NICOLAOU et al.** *Angew. Chem Intl. Ed. Engl.,* 1994, vol. 33, 183-186

- **KABAT et al.** Sequences of Proteins of Immunological Interest,. Public Health Service, National Institutes of Health, 1991
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **ALMAGRO ; FRANSSON.** *Front. Biosci.,* 2008, vol. 13, 1619-1633
- **JIN et al.** *Cancer Res,* 2008, vol. 68, 4360
- *CHEMICAL ABSTRACTS,* 877399-52-5
- *CHEMICAL ABSTRACTS,* 943540-75-8
- *CHEMICAL ABSTRACTS,* 658084-23-2
- *CHEMICAL ABSTRACTS,* 849217-64-7
- *CHEMICAL ABSTRACTS,* 875337-44-3
- **MUNCHI et al.** *Mol Cancer Ther,* June 2010, vol. 9, 1544
- *CHEMICAL ABSTRACTS,* 905854-02-6
- *CHEMICAL ABSTRACTS,* 849217-68-1
- *CHEMICAL ABSTRACTS,* 1196681-49-8
- *CHEMICAL ABSTRACTS,* 917879-39-1
- **HUDSON et al.** *Nat. Med.,* 2003, vol. 9, 129-134

- **PLUCKTHÜN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MARTENS et al.** *Clin Cancer Res,* 2006, vol. 12, 6144
- **MORRISON et al.** *Proc. Natl. Acad. Sci. USA,* 1984, vol. 81, 6851-6855
- **ALMAGRO ; FRANSSON.** *Frost. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **HOOGENBOOM et al.** *Methods in Molecular Biology,* vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** *Methods in Molecular Biology,* vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **GRUBER et al.** *J. Immunol,* 1994, vol. 152, 5368
- **TUTT et al.** *J. Immunol.,* 1991, vol. 147, 60
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **KAM et al.** *Proc. Natl. Acad. Sci. USA,* 2005, vol. 102, 11600-11605
- **HINMAN et al.** *Cancer Res.,* 1993, vol. 53, 3336-3342
- **LODE et al.** *Cancer Res.,* 1998, vol. 58, 2925-2928
- **KRATZ et al.** *Current Med. Chem.,* 2006, vol. 13, 477-523

- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834
- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **VITETTA et al.** *Science,* 1987, vol. 238, 1098
- **CHARI et al.** *Cancer Res.,* 1992, vol. 52, 127-131
- **PARKER ; BARNES.** *Methods in Molecular Biology,* 1999, vol. 106, 247-283
- **HOD.** *Biotechniques,* 1992, vol. 13, 852-854
- **WEIS et al.** *Trends in Genetics,* 1992, vol. 8, 263-264
- **AUSUBEL et al.** Current Protocols of Molecular Biology. John Wiley and Sons, 1997
- **RUPP ; LOCKER.** *Lab Invest.,* 1987, vol. 56, A67
- **DE ANDRÉS et al.** *BioTechniques,* 1995, vol. 18, 42044
- **HELD et al.** *Genome Research,* 1996, vol. 6, 986-994
- **GODFREY et al.** *J. Molec. Diagnostics,* 2000, vol. 2, 84-91
- **SPECHT et al.** *Am. J. Pathol.,* 2001, vol. 158, 419-29
- **KENT, W.** *Genome Res.,* 2002, vol. 12 (4), 656-64
- Primer3 on the WWW for general users and for biologist programmers. **ROZEN ; SKALETSKY.** Bioinformatics Methods and Protocols: Methods in Molecular Biology. Humana Press, 2000, 365-386
- General Concepts for PCR Primer Design. **DIEFFENBACH et al.** PCR Primer, A Laboratory Manual. Cold Spring Harbor Laboratory Press, 1995, 133-155
- Optimization of PCRs. **INNIS ; GELFAND.** PCR Protocols, A Guide to Methods and Applications. CRC Press, 1994, 5-11
- **PLASTERER.** T. N. Primerselect: Primer and probe design. *Methods Mol. Biol.,* 1997, vol. 70, 520-527
- **WANG et al.** RNA-Seq: a revolutionary tool for transcriptomics. *Nature Reviews Genetics,* January 2009, vol. 10 (1), 57-63
- **RYAN et al.** *BioTechniques,* 2008, vol. 45 (1), 81-94
- **MAHER et al.** Transcriptome sequencing to detect gene fusions in cancer. *Nature,* January 2009, vol. 458 (7234), 97-101
- **SCHENA et al.** *Proc. Natl. Acad. Sci. USA,* 1996, vol. 93 (2), 106-149
- **VELCULESCU et al.** *Science,* 1995, vol. 270, 484-487
- **VELCULESCU et al.** *Cell,* 1997, vol. 88, 243-51
- Current Protocols in Immunology. Wiley-Interscience, 1991, vol. 1 and 2
- Methods for the Preparation of Enzyme-Antibody Conjugates for use in Enzyme Immunoassay. **O'SULLIVAN et al.** Methods in Enzym. Academic press, 1981, vol. 73, 147-166
- **LEONG et al.** *Appl. Immunohistochem.,* 1996, vol. 4 (3), 201
- **KALLIONIEMI et al.** *Science,* 1992, vol. 258, 818-821
- **PARKIN DM.** *CA Cancer J Clin,* 2005, vol. 55, 74-108
- **SCAGLIOTTI et al.** *J Clin Oncol.,* 2008, vol. 26 (21), 3543-51
- **SANDLER et al.** *N Engl J Med.,* 2006, vol. 355 (24), 2542-50
- American Joint Committee on Cancer's AJCC. Cancer Staging Manual
- **CAPPUZZO et al.** *J Clin Oncol,* 2009, vol. 27, 1667-9
- **VARELLA-GARCIA et al.** *J Clin Pathol,* 2009, vol. 62, 970-7
- **CAPUZZO et al.** *JNCI,* 2005, vol. 97, 643-55